# EUROPÄISCHE PATENTSCHRIFT

(11) **EP 2 274 299 B1**
(45) Veröffentlichungstag und Bekanntmachung des Hinweises auf die Patenterteilung: **15.06.2016**
(21) Anmeldenummer: 09720733.6
(22) Anmeldetag: 26.02.2009
(51) Int. Cl.: C07D 401/14, C07D 409/04, C07D 413/14, C07D 417/14, A61K 31/4427, A61P 9/00, A61P 3/00

(54) **HETEROARYL-SUBSTITUIERTE DICYANOPYRIDINE UND IHRE VERWENDUNG ZUR BEHANDLUNG KARDIOVASKULÄRER ERKRANKUNGEN**
HETEROARYL-SUBSTITUTED DICYANOPYRIDINES AND USE THEREOF FOR TREATMENT OF CARDIOVASCULAR DISEASES
DICYANOPYRIDINES SUBSTITUÉES PAR HÉTÉROARYLE ET LEUR UTILISATION DANS LE TRAITEMENT DES MALADIES CARDIOVASCULAIRES

(30) Priorität: 11.03.2008 DE 102008013587
(43) Veröffentlichungstag der Anmeldung: 19.01.2011
(73) Patentinhaber: Bayer Intellectual Property GmbH, 40789 Monheim (DE)
(72) Erfinder: MEIBOM, Daniel, 51373 Leverkusen (DE); VAKALOPOULOS, Alexandros, 40721 Hilden (DE); ALBRECHT-KÜPPER, Barbara, 42289 Wülfrath (DE); ZIMMERMANN, Katja, 40470 Düsseldorf (DE); NELL, Peter, 42115 Wuppertal (DE); SÜSSMEIER, Frank, 42277 Wuppertal (DE)
(74) Vertreter: BIP Patents
(86) Internationale Anmeldenummer: PCT/EP2009/001352
(87) Internationale Veröffentlichungsnummer: WO 2009/112155

(56) Entgegenhaltungen:
- WO-A-01/62233
- WO-A-02/070485
- WO-A-2008/064788
- WO-A-2008/064789

## Beschreibung

Die vorliegende Anmeldung betrifft neue heteroaryl-substituierte Dicyanopyridine, Verfahren zu ihrer Herstellung, diese Verbindungen zur Behandlung und/oder Prävention von Krankheiten sowie ihre Verwendung zur Herstellung von Arzneimitteln zur Behandlung und/oder Prävention von Krankheiten, vorzugsweise zur Behandlung und/oder Prävention von kardiovaskulären Erkrankungen.

Adenosin, ein Purin-Nukleosid, ist in allen Zellen vorhanden und wird unter einer Vielzahl von physiologischen und pathophysiologischen Stimuli freigesetzt. Adenosin entsteht intrazellulär beim Abbau von Adenosin-5'-monophosphat (AMP) und S-Adenosylhomocystein als Zwischenprodukt, kann jedoch aus der Zelle freigesetzt werden und übt dann durch Bindung an spezifische Rezeptoren Funktionen als hormonähnliche Substanz oder Neurotransmitter aus.

Unter normoxischen Bedingungen ist die Konzentration des freien Adenosin im Extrazellulärraum sehr niedrig. Die extrazelluläre Konzentration von Adenosin erhöht sich in den betroffenen Organen jedoch dramatisch unter ischämischen bzw. hypoxischen Bedingungen. So ist beispielsweise bekannt, dass Adenosin die Thrombozyten-Aggregation hemmt und die Durchblutung der Herzkranzgefäße steigert. Weiterhin wirkt es auf den Blutdruck, die Herzfrequenz, auf die Ausschüttung von Neurotransmittern und auf die Lymphozyten-Differenzierung. In Adipozyten ist Adenosin in der Lage, die Lipolyse zu hemmen und somit die Konzentration an freien Fettsäuren und Triglyzeriden im Blut zu senken.

Diese Wirkungen von Adenosin zielen darauf ab, das Sauerstoffangebot der betroffenen Organe zu erhöhen bzw. den Stoffwechsel dieser Organe zu drosseln, um damit unter ischämischen oder hypoxischen Bedingungen eine Anpassung des Organstoffwechsels an die Organdurchblutung zu erreichen.

Die Wirkung von Adenosin wird über spezifische Rezeptoren vermittelt. Bekannt sind bisher die Subtypen A1, A2a, A2b und A3. Als "Adenosinrezeptor-selektive Liganden" werden erfindungsgemäß solche Substanzen bezeichnet, die selektiv an einen oder mehrere Subtypen der Adenosinrezeptoren binden und dabei entweder die Wirkung des Adenosin nachahmen (Adenosin-Agonisten) oder dessen Wirkung blockieren (Adenosin-Antagonisten) können.

Die Wirkungen dieser Adenosin-Rezeptoren werden intrazellulär durch den Botenstoff cAMP vermittelt. Im Falle der Bindung von Adenosin an die A2a- oder A2b-Rezeptoren kommt es über eine Aktivierung der membranständigen Adenylatzyklase zu einem Anstieg des intrazellulären cAMP, während die Bindung des Adenosin an die A1- oder A3-Rezeptoren über eine Hemmung der Adenylatzyklase eine Abnahme des intrazellulären cAMP-Gehalts bewirkt.

Im Herz-Kreislaufsystem sind die Hauptwirkungen der Aktivierung von Adenosin-Rezeptoren: Bradykardie, negative Inotropie und Protektion des Herzens vor Ischämie ("preconditioning") über A1-Rezeptoren, Dilation der Gefäße über A2a- und A2b-Rezeptoren sowie Inhibition der Fibroblasten und Glattmuskelzellproliferation über A2b-Rezeptoren.

Im Falle von A1-Agonisten (Kopplung bevorzugt über Gᵢ-Proteine) wird dabei eine Abnahme des intrazellulären cAMP-Gehaltes beobachtet (bevorzugt nach direkter Vorstimulation der Adenylatzyklase durch Forskolin). Entsprechend führen A2a- und A2b-Agonisten (Kopplung bevorzugt über Gₛ-Proteine) zu einer Zunahme und A2a- und A2b-Antagonisten zu einer Abnahme im cAMP-Gehalt der Zellen. Im Falle der A2-Rezeptoren ist eine direkte Vorstimulation der Adenylatzyklase durch Forskolin nicht hilfreich.

Die Aktivierung von A1-Rezeptoren durch spezifische A1-Agonisten führt beim Menschen zu einer frequenzabhängigen Senkung der Herzfrequenz, ohne einen Einfluss auf den Blutdruck zu haben. Selektive A1-Agonisten könnten somit unter anderem für die Behandlung von Angina pectoris und Vorhofflimmern geeignet sein.

Die kardioprotektive Wirkung der A1-Rezeptoren im Herzen kann unter anderem durch die Aktivierung dieser A1-Rezeptoren durch spezifische A1-Agonisten für die Behandlung und Organprotektion bei akutem Myokardinfarkt, akutem Koronarsyndrom, Herzinsuffizienz, Bypass Operationen, Herzkatheteruntersuchungen und Organtransplantationen genutzt werden.

Die Aktivierung von A2b-Rezeptoren durch Adenosin oder spezifische A2b-Agonisten führt über die Erweiterung von Gefäßen zu einer Blutdrucksenkung. Die Blutdrucksenkung ist von einem reflektorischen Herzfrequenzanstieg begleitet. Der Herzfrequenzanstieg kann durch die Aktivierung von A1-Rezeptoren durch spezifische A1-Agonisten reduziert werden.

Die Hemmung von A1-Rezeptoren durch spezifische A1-Antagonisten wirkt beim Menschen urikosurisch, natriuretisch sowie Kalium sparend diuretisch ohne Beeinflussung der glomerulären Filtrationsrate und somit nierenprotektiv. Selektive A1-Antagonisten können somit unter anderem zur Behandlung von akut dekompensierter Herzinsuffizienz und chronischer Herzinsuffizienz geeignet sein. Desweiteren können sie zur Nierenprotektion bei Nephropathie und anderen Nierenerkrankungen eingesetzt werden.

In Adipozyten bewirkt die Aktivierung von A1-Rezeptoren eine Inhibition der Lipolyse. Die Wirkung von A1-Agonisten auf den Lipidstoffwechsel führt somit zu einer Senkung von freien Fettsäuren und Triglyzeriden. Eine Senkung der Lipide wiederum führt bei Patienten mit Metabolischem Syndrom und bei Diabetikern zur Verringerung der Insulinresistenz und zur Verbesserung der Symptomatik.

Die zuvor genannte Rezeptor-Selektivität lässt sich bestimmen durch die Wirkung der Substanzen an Zelllinien, die nach stabiler Transfektion mit der entsprechenden cDNA die jeweiligen Rezeptorsubtypen exprimieren (siehe hierzu die Druckschrift M. E. Olah, H. Ren, J. Ostrowski, K. A. Jacobson, G. L. Stiles, "Cloning, expression, and characterization of the unique bovine A1 adenosine receptor. Studies on the ligand binding site by site-directed mutagenesis", J. Biol. Chem. 267 (1992), Seiten 10764-10770, deren Offenbarung hiermit im vollen Umfang durch Bezugnahme eingeschlossen ist).

Die Wirkung der Substanzen an solchen Zelllinien lässt sich erfassen durch biochemische Messung des intrazellulären Botenstoffes cAMP (siehe hierzu die Druckschrift K. N. Klotz, J. Hessling, J. Hegler, C. Owman, B. Kull, B. B. Fredholm, M. J. Lohse, "Comparative pharmacology of human adenosine receptor subtypes - characterization of stably transfected receptors in CHO cells", Naunyn Schmiedebergs Arch. Pharmacol. 357 (1998), Seiten 1-9, deren Offenbarung hiermit im vollen Umfang durch Bezugnahme eingeschlossen ist).

Bei den aus dem Stand der Technik bekannten, als "Adenosinrezeptor-spezifisch" geltenden Liganden handelt es sich überwiegend um Derivate auf Basis des natürlichen Adenosins [S.-A. Poulsen und R. J. Quinn, "Adenosine receptors: New opportunities for future drugs", Bioorganic and Medicinal Chemistry 6 (1998), Seiten 619-641]. Diese aus dem Stand der Technik bekannten Adenosin-Liganden haben jedoch meistens den Nachteil, dass sie nicht wirklich rezeptorspezifisch wirken, schwächer wirksam sind als das natürliche Adenosin oder nach oraler Applikation nur sehr schwach wirksam sind. Deshalb werden sie überwiegend nur für experimentelle Zwecke verwendet. In klinischer Entwicklung befindliche Verbindungen dieser Art eignen sich bislang nur zur intravenösen Applikation.

In WO 01/25210, WO 02/070484, WO 02/070485, WO 2008028590, WO 2008064788 und WO 2008/064789 werden substituierte 2-Thio- bzw. 2-Oxy-3,5-dicyano-4-phenyl-6-aminopyridine als Adenosinrezeptor-Liganden für die Behandlung von kardiovaskulären Erkrankungen offenbart. In WO 03/053441 werden spezifisch substituierte 2-Thio-3,5-dicyano-4-phenyl-6-aminopyridine als selektive Liganden des Adenosin A1-Rezeptors zur Behandlung kardiovaskulärer Erkrankungen beschrieben. Allerdings zeigte es sich, dass diese Verbindungen bezüglich ihrer physikochemischen Eigenschaften, wie beispielsweise ihrer Löslichkeit und/oder Formulierbarkeit, und/oder hinsichtlich ihrer *in vivo*-Eigenschaften, wie beispielsweise ihrem pharmakokinetischen Verhalten, ihrer Dosis-Wirkungsbeziehung und/oder ihrem Metabolisierungsweg, Nachteile aufweisen.

Diverse 4-Thienyl-3,5-dicyanopyridine sind in der Datenbank Chemical Abstracts ohne nähere Angaben zur Herstellung und Verwendung aufgeführt.

Substituierte 2-Amino-3,5-dicyanopyridine werden in J. Med. Chem. 2007, 50, 65-73 zur Behandlung von Prionen Infektionen beschrieben. JP 09-132529 beschreibt Aminopyridine als NO-Synthase Inhibitoren. JP 10-324687 beansprucht substituierte Pyrrole als Fungizide. Weiterhin werden in WO 01/62233 verschiedene Pyridin- und Pyrimidin-Derivate sowie deren Verwendung als Adenosinrezeptor-Modulatoren offenbart. Substituierte 3,5-Dicyanopyridine als Calciumabhängige Kaliumkanalöffner zur Behandlung urologischer Erkrankungen werden in EP 1 302 463-A1 und JP 2003-183254 beansprucht. In WO 03/091246 werden Pyrrol-substituierte Pyridine und Pyrimidine als Kinase Inhibitoren zur Behandlung von beispielsweise Krebs beschrieben.

Aufgabe der vorliegenden Erfindung ist die Bereitstellung neuer Verbindungen, die als potente und selektive Liganden des Adenosin A1-Rezeptors wirken und als solche zur Behandlung und/oder Prävention von Krankheiten, insbesondere zur Behandlung und/oder Prävention von kardiovaskulären Erkrankungen geeignet sind und ein gleiches oder verbessertes physikochemisches, pharmakokinetisches und/ oder therapeutisches Profil gegenüber den aus dem Stand der Technik bekannten Verbindungen aufweisen.

Gegenstand der vorliegenden Erfindung sind Verbindungen der Formel (I) 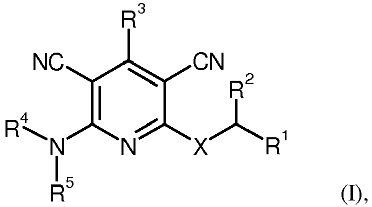 in welcher
- X: für O oder S steht,
- R¹: für 5- oder 6-gliedriges Heteroaryl steht,
wobei 5- oder 6-gliedriges Heteroaryl mit 1 oder 2 Substituenten unabhängig voneinander ausgewählt aus der Gruppe Halogen, Nitro, Cyano, (C₁-C₆)-Alkyl, Trifluormethyl, Hydroxy, (C₁-C₆)-Alkoxy, Amino, Mono-(C₁-C₆)-alkylamino, Di-(C₁-C₆)-alkylamino, Hydroxycarbonyl, (C₁-C₆)-Alkoxycarbonyl, Aminocarbonyl, Mono-(C₁-C₆)-alkylaminocarbonyl, Di-(C₁-C₆)-alkylaminocarbonyl, (C₃-C₇)-Cycloalkylaminocarbonyl, Aminosulfonyl, Mono-(C₁-C₆)-alkylaminosulfonyl, Di-(C₁-C₆)-alkylaminosulfonyl, (C₁-C₆)-Alkylsulfonylamino, Pyrrolidino, Piperidino, Morpholino, Piperazino, *N*'-(C₁-C₄)-Alkylpiperazino, Pyrrolidinocarbonyl, Piperidinocarbonyl, Morpholinocarbonyl, Piperazinocarbonyl, *N*'-(C₁-C₄)-Alkylpiperazinocarbonyl, Phenyl und 5- oder 6-gliedriges Heteroaryl substituiert sind,
worin Phenyl und 5- oder 6-gliedriges Heteroaryl mit 1 bis 3 Substituenten unabhängig voneinander ausgewählt aus der Gruppe Halogen, Nitro, Cyano, (C₁-C₆)-Alkyl, Difluormethyl, Trifluormethyl, Hydroxy, (C₁-C₆)-Alkoxy, Difluormethoxy, Trifluormethoxy, Amino, Mono-(C₁-C₆)-alkylamino, Di-(C₁-C₆)-alkylamino, Hydroxycarbonyl und (C₁-C₆)-Alkoxycarbonyl substituiert sein können,

R² für Wasserstoff oder (C₁-C₄)-Alkyl steht,
R³ für Thienyl, Pyrrolyl, Oxazolyl, Thiazolyl, Isoxazolyl, Isothiazolyl, Pyrazolyl, Imidazolyl, Triazolyl, Oxadiazolyl, Thiadiazolyl oder Tetrazolyl steht,
wobei Thienyl, Pyrrolyl, Oxazolyl, Thiazolyl, Isoxazolyl, Isothiazolyl, Pyrazolyl, Imidazolyl, Triazolyl, Oxadiazolyl, Thiadiazolyl und Tetrazolyl mit 1 oder 2 Substituenten unabhängig voneinander ausgewählt aus der Gruppe Halogen, Cyano, Hydroxy, (C₁-C₆)-Alkyl, (C₁-C₆)-Alkoxy, (C₃-C₇)-Cycloalkoxy und -NR^{A}R^{B} substituiert sein können,
worin (C₁-C₆)-Alkyl und (C₁-C₆)-Alkoxy mit 1 bis 3 Substituenten unabhängig voneinander ausgewählt aus der Gruppe Fluor, Trifluormethyl, (C₃-C₇)-Cycloalkyl, Oxo, Hydroxy, (C₁-C₄)-Alkoxy, Hydroxycarbonyl, Amino, Mono-(C₁-C₄)-alkylamino und Di-(C₁-C₄)-alkylamino substituiert sein können,
und
worin (C₃-C₇)-Cycloalkoxy mit 1 oder 2 Substituenten unabhängig voneinander ausgewählt aus der Gruppe (C₁-C₄)-Alkyl, Hydroxy, Oxo und (C₁-C₄)-Alkoxy substituiert sein kann,
und
worin
   R^{A} für Wasserstoff oder (C₁-C₆)-Alkyl steht,
   worin (C₁-C₆)-Alkyl seinerseits mit 1 bis 3 Substituenten Fluor substituiert sein kann,
   und
   worin (C₁-C₆)-Alkyl seinerseits mit einem Substituenten ausgewählt aus der Gruppe Hydroxy und (C₁-C₄)-Alkoxy substituiert sein kann,
   R^{B} für Wasserstoff, (C₁-C₆)-Alkyl, (C₃-C₇)-Cycloalkyl, (C₁-C₆)-Alkylcarbonyl, (C₁-C₄)-Alkylsulfonyl oder (C₃-C₇)-Cycloalkylsulfonyl steht, worin (C₁-C₆)-Alkyl seinerseits mit 1 bis 3 Substituenten Fluor substituiert sein kann, und worin (C₁-C₆)-Alkyl seinerseits mit 1 oder 2 Substituenten unabhängig voneinander ausgewählt aus der Gruppe (C₃-C₇)-Cycloalkyl, Oxo, Hydroxy, (C₁-C₄)-Alkoxy, Hydroxycarbonyl, Amino, Mono-(C₁-C₄)-alkylamino, Di-(C₁-C₄)-alkylamino und (C₃-C₇)-Cycloalkylamino substituiert sein kann, und worin (C₃-C₇)-Cycloalkyl seinerseits mit 1 oder 2 Substituenten unabhängig voneinander ausgewählt aus der Gruppe (C₁-C₄)-Alkyl, Hydroxy, Oxo und (C₁-C₄)-Alkoxy substituiert sein kann,
oder
   R^{A} und R^{B} zusammen mit dem Stickstoffatom, an das sie gebunden sind, einen 4-bis 7-gliedrigen Heterocyclus bilden, der ein weiteres Ring-Heteroatom aus der Reihe N, O oder S enthalten und mit 1 oder 2 Substituenten unabhängig voneinander ausgewählt aus der Gruppe (C₁-C₄)-Alkyl, Hydroxy, Oxo und (C₁-C₄)-Alkoxy substituiert sein kann,
- R⁴: für Wasserstoff oder (C₁-C₆)-Alkyl steht,
wobei (C₁-C₆)-Alkyl mit 1 oder 2 Substituenten unabhängig voneinander ausgewählt aus der Gruppe Hydroxy, (C₁-C₄)-Alkoxy, Hydroxycarbonyl, (C₁-C₄)-Alkoxycarbonyl, Amino, Mono-(C₁-C₄)-alkylamino und Di-(C₁-C₄)-alkylamino substituiert sein kann,
- R⁵: für Wasserstoff oder (C₁-C₆)-Alkyl steht,
wobei (C₁-C₆)-Alkyl mit 1 oder 2 Substituenten unabhängig voneinander ausgewählt aus der Gruppe Hydroxy, (C₁-C₄)-Alkoxy, Hydroxycarbonyl, (C₁-C₄)-Alkoxycarbonyl, Amino, Mono-(C₁-C₄)-alkylamino und Di-(C₁-C₄)-alkylamino substituiert sein kann,
oder
R⁴ und R⁵ zusammen mit dem Stickstoffatom, an das sie gebunden sind, einen 4- bis 7-gliedrigen Heterocyclus bilden, der ein weiteres Ring-Heteroatom aus der Reihe N, O oder S enthalten und mit 1 oder 2 Substituenten unabhängig voneinander ausgewählt aus der Gruppe (C₁-C₄)-Alkyl, Hydroxy, Oxo und (C₁-C₄)-Alkoxy substituiert sein kann,
sowie ihre N-Oxide, Salze, Solvate, Salze der N-Oxide und Solvate der N-Oxide und Salze.

Erfindungsgemäße Verbindungen sind die Verbindungen der Formel (I) und deren N-Oxide, Salze, Solvate, Salze der N-Oxide und Solvate der Salze und N-Oxide, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Im Rahmen der vorliegenden Erfindung haben die Substituenten, soweit nicht anders spezifiziert, die folgende Bedeutung:
Alkyl steht im Rahmen der Erfindung für einen linearen oder verzweigten Alkylrest mit 1 bis 6 bzw. 1 bis 4 Kohlenstoffatomen. Bevorzugt ist ein linearer oder verzweigter Alkylrest mit 1 bis 4 Kohlenstoffatomen. Beispielhaft und vorzugsweise seien genannt: Methyl, Ethyl, n-Propyl, Isopropyl, n-Butyl, iso-Butyl, sec.-Butyl, tert.-Butyl, 1-Ethylpropyl, n-Pentyl und n-Hexyl.

Cycloalkyl steht im Rahmen der Erfindung für einen monocyclischen, gesättigten Carbocyclus mit 3 bis 7 bzw. 5 bis 6 Ring-Kohlenstoffatomen. Beispielhaft und vorzugsweise seien genannt: Cyclopropyl, Cyclobutyl, Cyclopentyl, Cyclohexyl und Cycloheptyl.

Alkylcarbonyl steht im Rahmen der Erfindung für einen linearen oder verzweigten Alkylrest mit 1 bis 6 bzw. 1 bis 4 Kohlenstoffatomen und einer in 1-Position angebundenen Carbonylgruppe. Beispielhaft und vorzugsweise seien genannt: Methylcarbonyl, Ethylcarbonyl, n-Propylcarbonyl, iso-Propylcarbonyl, n-Butylcarbonyl, iso-Butylcarbonyl und tert.-Butylcarbonyl.

Alkoxy steht im Rahmen der Erfindung für einen linearen oder verzweigten Alkoxyrest mit 1 bis 6 bzw. 1 bis 4 bzw. 2 bis 4 Kohlenstoffatomen. Bevorzugt ist ein linearer oder verzweigter Alkoxyrest mit 1 bis 4 bzw. 2 bis 4 Kohlenstoffatomen. Beispielhaft und vorzugsweise seien genannt: Methoxy, Ethoxy, n-Propoxy, Isopropoxy, n-Butoxy, tert.-Butoxy, n-Pentoxy und n-Hexoxy.

Cycloalkoxy steht im Rahmen der Erfindung für einen monocyclischen, gesättigten Carbocyclus mit 3 bis 7 Kohlenstoffatomen, der über ein Sauerstoffatom gebunden ist. Beispielhaft und vorzugsweise seien genannt: Cyclopropyloxy, Cyclobutyloxy, Cyclopentyloxy, Cyclohexyloxy und Cycloheptyloxy.

Alkoxycarbonyl stehen im Rahmen der Erfindung für einen linearen oder verzweigten Alkoxyrest mit 1 bis 6 bzw. 1 bis 4 Kohlenstoffatomen und einer am Sauerstoff angebundenen Carbonylgruppe. Bevorzugt ist ein linearer oder verzweigter Alkoxycarbonylrest mit 1 bis 4 Kohlenstoffatomen in der Alkoxy-Gruppe. Beispielhaft und vorzugsweise seien genannt: Methoxycarbonyl, Ethoxycarbonyl, n-Propoxycarbonyl, Isopropoxycarbonyl und tert.-Butoxycarbonyl.

Mono-alkylamino steht im Rahmen der Erfindung für eine Amino-Gruppe mit einem linearen oder verzweigten Alkylsubstituenten, der 1 bis 6 bzw. 1 bis 4 Kohlenstoffatome aufweist. Bevorzugt ist ein linearer oder verzweigter Monoalkylamino-Rest mit 1 bis 4 Kohlenstoffatomen. Beispielhaft und vorzugsweise seien genannt: Methylamino, Ethylamino, n-Propylamino, Isopropylamino, n-Butylamino, tert.-Butylamino, n-Pentylamino und n-Hexylamino.

Di-alkylamino steht im Rahmen der Erfindung für eine Amino-Gruppe mit zwei gleichen oder verschiedenen linearen oder verzweigten Alkylsubstituenten, die jeweils 1 bis 6 bzw. 1 bis 4 Kohlenstoffatome aufweisen. Bevorzugt sind lineare oder verzweigte Dialkylamino-Reste mit jeweils 1 bis 4 Kohlenstoffatomen. Beispielhaft und vorzugsweise seien genannt: *N,N-*Dimethylamino, *N,N*-Diethylamino, *N*-Ethyl-*N*-methylamino, *N*-Methyl-*N*-n-propylamino, *N*-Isopropyl-*N*-n-propylamino, *N,N*-Diisopropylamino, *N*-n-Butyl-*N*-methylamino, *N*-tert.-Butyl-*N-*methylamino, *N*-Ethyl-*N*-n-pentylamino und *N*-n-Hexyl-*N*-methylamino.

Cycloalkylamino steht im Rahmen der Erfindung für eine Amino-Gruppe mit einem monocyclischen, gesättigten Carbocyclus mit 3 bis 7 Kohlenstoffatomen. Beispielhaft und vorzugsweise seien genannt: Cyclopropylamino, Cyclobutylamino, Cyclopentylamino, Cyclohexylamino und Cycloheptylamino.

Mono-alkylaminocarbonyl steht im Rahmen der Erfindung für eine Amino-Gruppe, die über eine Carbonylgruppe verknüpft ist und die einen linearen oder verzweigten Alkylsubstituenten mit 1 bis 6 bzw. 1 bis 4 Kohlenstoffatomen aufweist. Bevorzugt ist ein Mono-alkylaminocarbonyl-Rest mit 1 bis 4 Kohlenstoffatomen in der Alkylgruppe. Beispielhaft und vorzugsweise seien genannt: Methylaminocarbonyl, Ethylaminocarbonyl, *n*-Propylaminocarbonyl, Isopropylaminocarbonyl, *n-*Butylaminocarbonyl, *tert*.-Butylaminocarbonyl, *n*-Pentylaminocarbonyl und *n-*Hexylaminocarbonyl.

Di-alkylaminocarbonyl steht im Rahmen der Erfindung für eine Amino-Gruppe, die über eine Carbonylgruppe verknüpft ist und die zwei gleiche oder verschiedene lineare oder verzweigte Alkylsubstituenten mit jeweils 1 bis 6 bzw. 1 bis 4 Kohlenstoffatomen aufweist. Bevorzugt ist ein Dialkylaminocarbonyl-Rest mit jeweils 1 bis 4 Kohlenstoffatomen pro Alkylgruppe. Beispielhaft und vorzugsweise seien genannt: *N,N*-Dimethylaminocarbonyl, *N,N*-Diethylaminocarbonyl, *N-*Ethyl-*N*-methylaminocarbonyl, *N*-Methyl-*N*-*n*-propylaminocarbonyl, *N*-*n*-Butyl-*N*-methylaminocarbonyl, *N*-tert.-Butyl-*N*-methylaminocarbonyl, *N*-*n*-Pentyl-*N*-methylaminocarbonyl und *N-n*-Hexyl-*N*-methylaminocarbonyl.

Cycloalkylaminocarbonyl steht im Rahmen der Erfindung für eine Amino-Gruppe, die über eine Carbonylgruppe verknüpft ist und die einen monocyclischen, gesättigten Carbocyclus mit 3 bis 7 Kohlenstoffatomen aufweist. Beispielhaft und vorzugsweise seien genannt: Cyclopropylaminocarbonyl, Cyclobutylaminocarbonyl, Cyclopentylaminocarbonyl, Cyclohexylaminocarbonyl und Cycloheptylaminocarbonyl.

Mono-alkylaminosulfonyl steht im Rahmen der Erfindung für eine Amino-Gruppe, die über eine Sulfonylgruppe verknüpft ist und die einen linearen oder verzweigten Alkylsubstituenten mit 1 bis 6 Kohlenstoffatomen aufweist. Beispielhaft und vorzugsweise seien genannt: Methylaminosulfonyl, Ethylaminosulfonyl, *n*-Propylaminosulfonyl, Isopropylaminosulfonyl, *n*-Butylaminosulfonyl und *tert*.-Butylaminosulfonyl.

Di-alkylaminosulfonyl steht im Rahmen der Erfindung für eine Amino-Gruppe, die über eine Sulfonylgruppe verknüpft ist und die zwei gleiche oder verschiedene lineare oder verzweigte Alkylsubstituenten mit jeweils 1 bis 6 Kohlenstoffatomen aufweist. Beispielhaft und vorzugsweise seien genannt: *N,N*-Dimethylaminosulfonyl, *N,N*-Diethylaminosulfonyl, *N*-Ethyl-*N*-methylaminosulfonyl, *N*-Methyl-*N*-*n*-propylaminosulfonyl, *N*-*n*-Butyl-*N*-methylaminosulfonyl und *N-tert.-*Butyl-*N*-methylaminosulfonyl.

Alkylsulfonyl steht in Rahmen der Erfindung für einen linearen oder verzweigten Alkylrest mit 1 bis 4 Kohlenstoffatomen, der über eine Sulfonylgruppe gebunden ist. Beispielhaft und vorzugsweise seinen genannt: Methylsulfonyl, Ethylsulfonyl, n-Propylsulfonyl, iso-Propylsulfonyl, n-Butylsulfonyl und tert.-Butylsulfonyl.

Cycloalkylsulfonyl steht in Rahmen der Erfindung für einen monocyclischen, gesättigten Carbocyclus mit 3 bis 7 Kohlenstoffatomen, der über eine Sulfonylgruppe gebunden ist. Beispielhaft und vorzugsweise seinen genannt: Cyclopropylsulfonyl, Cyclobutylsulfonyl, Cyclopentylsulfonyl, Cyclohexylsulfonyl und Cycloheptylsulfonyl.

Alkylsulfonylamino steht im Rahmen der Erfindung für eine Amino-Gruppe mit einem linearen oder verzweigten Alkylsulfonyl-Substituenten, der 1 bis 6 Kohlenstoffatome aufweist und über die Sulfonylgruppe mit dem N-Atom verknüpft ist. Beispielhaft und vorzugsweise seien genannt: Methylsulfonylamino, Ethylsulfonylamino, *n*-Propylsulfonylamino, Isopropylsulfonylamino, *n-*Butylsulfonylamino, *tert*.-Butylsulfonylamino, *n*-Pentylsulfonylamino und *n*-Hexylsulfonylamino. Heterocyclus steht im Rahmen der Erfindung für einen gesättigten Heterocyclus mit insgesamt 4 bis 7 Ringatomen, der ein oder zwei Ring-Heteroatome aus der Reihe N, O und/oder S enthält und über ein Ring-Kohlenstoffatom oder gegebenenfalls ein Ring-Stickstoffatom verknüpft ist. Beispielhaft seien genannt: Azetidinyl, Pyrrolidinyl, Pyrazolidinyl, Tetrahydrofuranyl, Piperidinyl, Piperazinyl, Tetrahydropyranyl, Morpholinyl und Thiomorpholinyl. Bevorzugt sind Azetidinyl, Pyrrolidinyl, Tetrahydrofuranyl, Piperidinyl, Piperazinyl, Tetrahydropyranyl und Morpholinyl.

Heteroaryl steht im Rahmen der Erfindung für einen monocyclischen aromatischen Heterocyclus (Heteroaromaten) mit insgesamt 5 oder 6 Ringatomen, der bis zu drei gleiche oder verschiedene Ring-Heteroatome aus der Reihe N, O und/oder S enthält und über ein Ring-Kohlenstoffatom oder gegebenenfalls über ein Ring-Stickstoffatom verknüpft ist. Beispielhaft und vorzugsweise seien genannt: Furyl, Pyrrolyl, Thienyl, Pyrazolyl, Imidazolyl, Thiazolyl, Oxazolyl, Isoxazolyl, Isothiazolyl, Triazolyl, Oxadiazolyl, Thiadiazolyl, Pyridyl, Pyrimidinyl, Pyridazinyl, Pyrazinyl und Triazinyl.

Halogen schließt im Rahmen der Erfindung Fluor, Chlor, Brom und Iod ein. Bevorzugt sind Chlor oder Fluor.

Eine Oxo-Gruppe steht im Rahmen der Erfindung für ein Sauerstoffatom, das über eine Doppelbindung an ein Kohlenstoffatom gebunden ist.

In den Formeln der Gruppe, für die R³ stehen kann, steht der Endpunkt der Linie, an dem ein Zeichen * steht, nicht für ein Kohlenstoffatom beziehungsweise eine CH₂-Gruppe, sondern ist Bestandteil der Bindung zu dem jeweils bezeichneten Atom, an das R³ gebunden ist.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem oder zwei gleichen oder verschiedenen Substituenten.

Bevorzugt sind im Rahmen der vorliegenden Erfindung Verbindungen der Formel (I), in welcher
- X: für O oder S steht,
- R¹: für 5- oder 6-gliedriges Heteroaryl steht,
wobei 5- oder 6-gliedriges Heteroaryl mit 1 oder 2 Substituenten unabhängig voneinander ausgewählt aus der Gruppe Fluor, Chlor, Cyano, (C₁-C₄)-Alkyl, Trifluormethyl, (C₁-C₄)-Alkoxy, Mono-(C₁-C₄)-alkylamino, Di-(C₁-C₄)-alkylamino, Hydroxycarbonyl, (C₁-C₄)-Alkoxycarbonyl, Aminocarbonyl, Mono-(C₁-C₄)-alkylamino-carbonyl, Di-(C₁-C₄)-alkylaminocarbonyl, Pyrrolidino, Piperidino, Morpholino, Piperazino, *N'*-(C₁-C₄)-Alkylpiperazino, Phenyl und 5- oder 6-gliedriges Heteroaryl substituiert sind,
worin Phenyl und 5- oder 6-gliedriges Heteroaryl mit 1 oder 2 Substituenten unabhängig voneinander ausgewählt aus der Gruppe Fluor, Chlor, Cyano, (C₁-C₄)-Alkyl, Difluormethyl, Trifluormethyl, (C₁-C₄)-Alkoxy, Difluormethoxy, Trifluormethoxy, Hydroxycarbonyl und (C₁-C₄)-Alkoxycarbonyl substituiert sein können,
- R²: für Wasserstoff oder Methyl steht,
- R³: für Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isoxazol-5-yl, Pyrazol-1-yl, Pyrazol-3-yl, Pyrazol-4-yl, Pyrazol-5-yl, Imidazol-1-yl, Imidazol-2-yl, Imidazol-4-yl oder Imidazol-5-yl steht,
wobei Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isoxazol-5-yl, Pyrazol-1-yl, Pyrazol-3-yl, Pyrazol-4-yl, Pyrazol-5-yl, Imidazol-1-yl, Imidazol-2-yl, Imidazol-4-yl und Imidazol-5-yl mit 1 oder 2 Substituenten unabhängig voneinander ausgewählt aus der Gruppe Fluor, (C₁-C₆)-Alkyl, (C₁-C₄)-Alkoxy und -NR^{A}R^{B} substituiert sein können,
worin (C₁-C₆)-Alkyl und (C₁-C₄)-Alkoxy mit 1 bis 3 Substituenten Fluor substituiert sein können,
und
worin (C₁-C₆)-Alkyl und (C₂-C₄)-Alkoxy mit 1 oder 2 Substituenten unabhängig voneinander ausgewählt aus der Gruppe Trifluormethyl, Hydroxy, Methoxy, Ethoxy, Hydroxycarbonyl, Amino, Methylamino, Ethylamino, *N,N*-Dimethylamino und *N,N*-Diethylamino substituiert sein können,
und
worin
   R^{A} für Wasserstoff oder (C₁-C₄)-Alkyl steht,
   worin (C₁-C₄)-Alkyl seinerseits mit einem Substituenten ausgewählt aus der Gruppe Hydroxy und (C₁-C₄)-Alkoxy substituiert sein kann,
   R^{B} für Wasserstoff oder (C₁-C₄)-Alkyl steht,
   worin (C₁-C₄)-Alkyl seinerseits mit 1 oder 2 Substituenten unabhängig voneinander ausgewählt aus der Gruppe Hydroxy, (C₁-C₄)-Alkoxy und Hydroxycarbonyl substituiert sein kann,
- R⁴: für Wasserstoff oder Methyl steht,
- R⁵: für Wasserstoff oder (C₁-C₄)-Alkyl steht,
wobei (C₁-C₄)-Alkyl mit 1 oder 2 Substituenten unabhängig voneinander ausgewählt aus der Gruppe Hydroxy, Methoxy und Ethoxy substituiert sein kann,
oder
R⁴ und R⁵ zusammen mit dem Stickstoffatom, an das sie gebunden sind, einen 4- bis 6-gliedrigen Heterocyclus bilden, der ein weiteres Ring-Heteroatom aus der Reihe N, O oder S enthalten und mit 1 oder 2 Substituenten unabhängig voneinander ausgewählt aus der Gruppe (C₁-C₄)-Alkyl, Hydroxy, Methoxy und Ethoxy substituiert sein kann,
sowie ihre Salze, Solvate und Solvate der Salze.

Bevorzugt sind im Rahmen der vorliegenden Erfindung auch Verbindungen der Formel (I), in welcher
- X: für O oder S steht,
- R¹: für 5- oder 6-gliedriges Heteroaryl steht,
wobei 5- oder 6-gliedriges Heteroaryl mit 1 oder 2 Substituenten unabhängig voneinander ausgewählt aus der Gruppe Fluor, Chlor, Cyano, (C₁-C₄)-Alkyl, Trifluormethyl, (C₁-C₄)-Alkoxy, Mono-(C₁-C₄)-alkylamino, Di-(C₁-C₄)-alkylamino, Hydroxycarbonyl, (C₁-C₄)-Alkoxycarbonyl, Aminocarbonyl, Mono-(C₁-C₄)-alkylaminocarbonyl, Di-(C₁-C₄)-alkyl-aminocarbonyl, Pyrrolidino, Piperidino, Morpholino, Piperazino, *N'*-(C₁-C₄)-Alkylpiperazino, Phenyl und 5- oder 6-gliedriges Heteroaryl substituiert sind, worin Phenyl und 5- oder 6-gliedriges Heteroaryl mit 1 oder 2 Substituenten unabhängig voneinander ausgewählt aus der Gruppe Fluor, Chlor, Cyano, (C₁-C₄)-Alkyl, Difluormethyl, Trifluormethyl, (C₁-C₄)-Alkoxy, Difluormethoxy, Trifluormethoxy, Hydroxycarbonyl und (C₁-C₄)-Alkoxycarbonyl substituiert sein können,
- R²: für Wasserstoff oder Methyl steht,
- R³: für Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isoxazol-5-yl, Pyrazol-1-yl, Pyrazol-3-yl, Pyrazol-4-yl, Pyrazol-5-yl, Imidazol-1-yl, Imidazol-2-yl, Imidazol-4-yl oder Imidazol-5-yl steht,
wobei Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isoxazol-5-yl, Pyrazol-1-yl, Pyrazol-3-yl, Pyrazol-4-yl, Pyrazol-5-yl, Imidazol-1-yl, Imidazol-2-yl, Imidazol-4-yl und Imidazol-5-yl mit 1 oder 2 Substituenten unabhängig voneinander ausgewählt aus der Gruppe Fluor, Trifluormethyl (C₁-C₆)-Alkyl, (C₁-C₄)-Alkoxy und NR^{A}R^{B} substituiert sein können,
worin (C₁-C₆)-Alkyl und (C₂-C₄)-Alkoxy mit 1 oder 2 Substituenten unabhängig voneinander ausgewählt aus der Gruppe Fluor, Trifluormethyl, Hydroxy, Methoxy, Ethoxy, Hydroxycarbonyl, Amino, Methylamino, Ethylamino, *N,N*-Dimethylamino und *N,N*-Diethylamino substituiert sein können,
und
worin
   R^{A} für Wasserstoff oder (C₁-C₄)-Alkyl steht, worin (C₁-C₄)-Alkyl seinerseits mit einem Substituenten ausgewählt aus der Gruppe Hydroxy und (C₁-C₄)-Alkoxy substituiert sein kann,
   R^{B} für Wasserstoff oder (C₁-C₄)-Alkyl steht, worin (C₁-C₄)-Alkyl seinerseits mit 1 oder 2 Substituenten unabhängig voneinander ausgewählt aus der Gruppe Hydroxy, (C₁-C₄)-Alkoxy und Hydroxycarbonyl substituiert sein kann,
- R⁴: für Wasserstoff oder Methyl steht,
- R⁵: für Wasserstoff oder (C₁-C₄)-Alkyl steht,
wobei (C₁-C₄)-Alkyl mit 1 oder 2 Substituenten unabhängig voneinander ausgewählt aus der Gruppe Hydroxy, Methoxy und Ethoxy substituiert sein kann,
oder
R⁴ und R⁵ zusammen mit dem Stickstoffatom, an das sie gebunden sind, einen 4- bis 6-gliedrigen Heterocyclus bilden, der ein weiteres Ring-Heteroatom aus der Reihe N, O oder S enthalten und mit 1 oder 2 Substituenten unabhängig voneinander ausgewählt aus der Gruppe (C₁-C₄)-Alkyl, Hydroxy, Methoxy und Ethoxy substituiert sein kann,
sowie ihre Salze, Solvate und Solvate der Salze.

Besonders bevorzugt sind im Rahmen der vorliegenden Erfindung Verbindungen der Formel (I), in welcher
- X: für S steht,
- R¹: für Oxazolyl, Thiazolyl oder Pyridyl steht,
wobei Pyridyl mit einem Substituenten ausgewählt aus der Gruppe Cyano, Methoxy, Ethoxy, Hydroxycarbonyl, Methoxycarbonyl, Ethoxycarbonyl, Aminocarbonyl, Methylaminocarbonyl und Dimethylaminocarbonyl substituiert ist,
und
wobei Pyridyl mit einem Substituenten ausgewählt aus der Gruppe Fluor, Chlor, Methyl, Ethyl und Trifluormethyl substituiert sein kann,
und
wobei Oxazolyl und Thiazolyl mit einem Substituenten Phenyl substituiert sind,
worin Phenyl mit einem Substituenten ausgewählt aus der Gruppe Fluor, Chlor, Cyano, Methyl, Methoxy, Hydroxycarbonyl und Methoxycarbonyl substituiert sein kann,
und
wobei Oxazolyl und Thiazolyl mit einem Substituenten ausgewählt aus der Gruppe Fluor, Methyl, Ethyl, Methoxy, Hydroxycarbonyl und Methoxycarbonyl substituiert sein können,
- R²: für Wasserstoff steht,
- R³: für eine Gruppe der Formel 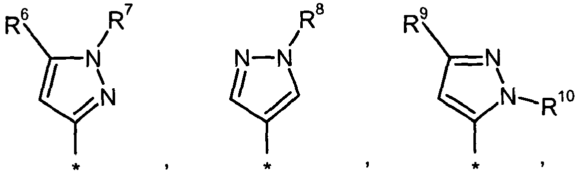 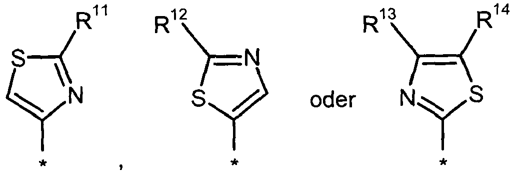 steht,
wobei
* die Anknüpfstelle an das Pyridin bedeutet,
und
wobei
R⁶ für Wasserstoff, Methyl oder Ethyl steht,
R⁷ für Wasserstoff oder (C₁-C₆)-Alkyl steht, worin (C₁-C₆)-Alkyl mit 1 oder 2 Substituenten unabhängig voneinander ausgewählt aus der Gruppe Hydroxy und Methoxy substituiert sein kann,
R⁸ für Wasserstoff oder (C₁-C₆)-Alkyl steht,
worin (C₁-C₆)-Alkyl mit 1 oder 2 Substituenten unabhängig voneinander ausgewählt aus der Gruppe Hydroxy und Methoxy substituiert sein kann,
R⁹ für Wasserstoff, Methyl oder Ethyl steht,
R¹⁰ für Wasserstoff steht,
R¹¹ für Wasserstoff oder (C₁-C₆)-Alkyl steht,
worin (C₁-C₆)-Alkyl mit 1 oder 2 Substituenten unabhängig voneinander ausgewählt aus der Gruppe Hydroxy und Methoxy substituiert sein kann,
R¹² für Wasserstoff oder (C₁-C₆)-Alkyl steht,
worin (C₁-C₆)-Alkyl mit 1 oder 2 Substituenten unabhängig voneinander ausgewählt aus der Gruppe Hydroxy und Methoxy substituiert sein kann,
R¹³ für Wasserstoff, Methyl oder Ethyl steht, und
R¹⁴ für Wasserstoff oder (C₁-C₆)-Alkyl steht,
worin (C₁-C₆)-Alkyl mit 1 oder 2 Substituenten unabhängig voneinander ausgewählt aus der Gruppe Hydroxy und Methoxy substituiert sein kann,
- R⁴: für Wasserstoff steht,
- R⁵: für Wasserstoff, Ethyl, n-Propyl oder sec.-Butyl steht,
wobei Ethyl, n-Propyl und sec.-Butyl mit 1 oder 2 Substituenten Hydroxy substituiert sein können,
oder
R⁴ und R⁵ zusammen mit dem Stickstoffatom, an das sie gebunden sind, einen Azetidinyl- oder Pyrrolidinyl-Ring bilden, der mit einem Substituenten Hydroxy substituiert sein kann,
sowie ihre Salze, Solvate und Solvate der Salze.

Bevorzugt sind im Rahmen der vorliegenden Erfindung auch Verbindungen der Formel (I), in welcher
- X: für O oder S steht,
- R¹: für Oxazolyl, Thiazolyl oder Pyridyl steht, wobei Oxazolyl, Thiazolyl und Pyridyl mit 1 oder 2 Substituenten unabhängig voneinander ausgewählt aus der Gruppe Fluor, Chlor, Cyano, (C₁-C₄)-Alkyl, Trifluormethyl, (C₁-C₄)-Alkoxy, Hydroxycarbonyl, (C₁-C₄)-Alkoxycarbonyl, Aminocarbonyl, Mono-(C₁-C₄)-alkylaminocarbonyl, Di-(C₁-C₄)-alkylaminocarbonyl, Pyrrolidino, Piperidino, Morpholino, Piperazino, *N'*-(C₁-C₄)-Alkylpiperazino, Phenyl und 5- oder 6-gliedriges Heteroaryl, substituiert sind,
worin Phenyl und 5- oder 6-gliedriges Heteroaryl mit 1 oder 2 Substituenten unabhängig voneinander ausgewählt aus der Gruppe Fluor, Chlor, Cyano, (C₁-C₄)-Alkyl, Difluormethyl, Trifluormethyl, (C₁-C₄)-Alkoxy, Difluormethoxy, Trifluormethoxy, Hydroxycarbonyl und (C₁-C₄)-Alkoxycarbonyl substituiert sein können,
- R²: für Wasserstoff oder (C₁-C₄)-Alkyl steht,
- R³: für Thien-2-yl oder Thien-3-yl steht,
wobei Thien-2-yl und Thien-3-yl mit 1 oder 2 Substituenten unabhängig voneinander ausgewählt aus der Gruppe Fluor, (C₁-C₆)-Alkyl, (C₁-C₄)-Alkoxy und -NR^{A}R^{B} substituiert sein können,
worin (C₁-C₆)-Alkyl und (C₁-C₄)-Alkoxy mit 1 bis 3 Substituenten Fluor substituiert sein können,
und
worin (C₁-C₆)-Alkyl und (C₂-C₄)-Alkoxy mit 1 oder 2 Substituenten unabhängig voneinander ausgewählt aus der Gruppe Trifluormethyl, Hydroxy, Methoxy, Ethoxy, Hydroxycarbonyl, Amino, Mono-(C₁-C₄)-alkylamino und Di-(C₁-C₄)-alkylamino substituiert sein können,
und
worin
   R^{A} für Wasserstoff oder (C₁-C₄)-Alkyl steht,
   worin (C₁-C₄)-Alkyl seinerseits mit einem Substituenten ausgewählt aus der Gruppe Hydroxy und (C₁-C₄)-Alkoxy substituiert sein kann,
   R^{B} für Wasserstoff oder (C₁-C₄)-Alkyl steht,
   worin (C₁-C₄)-Alkyl seinerseits mit 1 oder 2 Substituenten unabhängig voneinander ausgewählt aus der Gruppe Hydroxy, (C₁-C₄)-Alkoxy und Hydroxycarbonyl substituiert sein kann,
- R⁴: für Wasserstoff oder Methyl steht,
- R⁵: für Wasserstoff oder (C₁-C₄)-Alkyl steht,
wobei (C₁-C₄)-Alkyl mit 1 oder 2 Substituenten unabhängig voneinander ausgewählt aus der Gruppe Hydroxy, Methoxy und Ethoxy substituiert sein kann,
oder
R⁴ und R⁵ zusammen mit dem Stickstoffatom, an das sie gebunden sind, einen 4- bis 6-gliedrigen Heterocyclus bilden, der ein weiteres Ring-Heteroatom aus der Reihe N, O oder S enthalten und mit 1 oder 2 Substituenten unabhängig voneinander ausgewählt aus der Gruppe (C₁-C₄)-Alkyl, Hydroxy, Methoxy und Ethoxy substituiert sein kann,
sowie ihre Salze, Solvate und Solvate der Salze.

Bevorzugt sind im Rahmen der vorliegenden Erfindung auch Verbindungen der Formel (I), in welcher
- X: für O oder S steht,
- R¹: für Oxazolyl, Thiazolyl oder Pyridyl steht, wobei Oxazolyl, Thiazolyl und Pyridyl mit 1 oder 2 Substituenten unabhängig voneinander ausgewählt aus der Gruppe Fluor, Chlor, Cyano, (C₁-C₄)-Alkyl, Trifluormethyl, (C₁-C₄)-Alkoxy, Hydroxycarbonyl, (C₁-C₄)-Alkoxycarbonyl, Aminocarbonyl, Mono-(C₁-C₄)-alkylaminocarbonyl, Di-(C₁-C₄)-alkylaminocarbonyl, Pyrrolidino, Piperidino, Morpholino, Piperazino, *N'*-(C₁-C₄)-Alkylpiperazino, Phenyl und 5- oder 6-gliedriges Heteroaryl, substituiert sind,
worin Phenyl und 5- oder 6-gliedriges Heteroaryl mit 1 oder 2 Substituenten unabhängig voneinander ausgewählt aus der Gruppe Fluor, Chlor, Cyano, (C₁-C₄)-Alkyl, Difluormethyl, Trifluormethyl, (C₁-C₄)-Alkoxy, Difluormethoxy, Trifluormethoxy, Hydroxycarbonyl und (C₁-C₄)-Alkoxycarbonyl substituiert sein können,
- R²: für Wasserstoff oder (C₁-C₄)-Alkyl steht,
- R³: für Thien-2-yl oder Thien-3-yl steht,
wobei Thien-2-yl und Thien-3-yl mit 1 oder 2 Substituenten unabhängig voneinander ausgewählt aus der Gruppe Fluor, (C₁-C₆)-Alkyl, (C₁-C₄)-Alkoxy und -NR^{A}R^{B} substituiert sein können,
worin (C₁-C₆)-Alkyl und (C₂-C₄)-Alkoxy mit 1 oder 2 Substituenten unabhängig voneinander ausgewählt aus der Gruppe Fluor, Trifluormethyl, Hydroxy, Methoxy, Ethoxy, Hydroxycarbonyl, Amino, Methylamino, Ethylamino, *N,N*-Dimethylamino und *N,N*-Diethylamino substituiert sein können,
und
worin
R^{A} für Wasserstoff oder (C₁-C₄)-Alkyl steht,
worin (C₁-C₄)-Alkyl seinerseits mit einem Substituenten ausgewählt aus der Gruppe Hydroxy und (C₁-C₄)-Alkoxy substituiert sein kann,
R^{B} für Wasserstoff oder (C₁-C₄)-Alkyl steht,
worin (C₁-C₄)-Alkyl seinerseits mit 1 oder 2 Substituenten unabhängig voneinander ausgewählt aus der Gruppe Hydroxy, (C₁-C₄)-Alkoxy und Hydroxycarbonyl substituiert sein kann,
- R⁴: für Wasserstoff oder Methyl steht,
- R⁵: für Wasserstoff oder (C₁-C₄)-Alkyl steht,
wobei (C₁-C₄)-Alkyl mit 1 oder 2 Substituenten unabhängig voneinander ausgewählt aus der Gruppe Hydroxy, Methoxy und Ethoxy substituiert sein kann,
oder
R⁴ und R⁵ zusammen mit dem Stickstoffatom, an das sie gebunden sind, einen 4- bis 6-gliedrigen Heterocyclus bilden, der ein weiteres Ring-Heteroatom aus der Reihe N, O oder S enthalten und mit 1 oder 2 Substituenten unabhängig voneinander ausgewählt aus der Gruppe (C₁-C₄)-Alkyl, Hydroxy, Methoxy und Ethoxy substituiert sein kann,
sowie ihre Salze, Solvate und Solvate der Salze.

Besonders bevorzugt sind im Rahmen der vorliegenden Erfindung auch Verbindungen der Formel (I), in welcher
- X: für S steht,
- R¹: für Oxazolyl, Thiazolyl oder Pyridyl steht,
wobei Pyridyl mit einem Substituenten ausgewählt aus der Gruppe Cyano, Methoxy, Ethoxy, Hydroxycarbonyl, Methoxycarbonyl, Ethoxycarbonyl, Aminocarbonyl, Methylaminocarbonyl und Dimethylaminocarbonyl substituiert ist,
und
wobei Pyridyl mit einem Substituenten ausgewählt aus der Gruppe Fluor, Chlor, Methyl, Ethyl und Trifluormethyl substituiert sein kann,
und
wobei Oxazolyl und Thiazolyl mit einem Substituenten Phenyl substituiert sind,
worin Phenyl mit einem Substituenten ausgewählt aus der Gruppe Fluor, Chlor, Cyano, Methyl, Methoxy, Hydroxycarbonyl und Methoxycarbonyl substituiert sein kann,
und
wobei Oxazolyl und Thiazolyl mit einem Substituenten ausgewählt aus der Gruppe Fluor, Methyl, Ethyl, Methoxy, Hydroxycarbonyl und Methoxycarbonyl substituiert sein können,
- R²: für Wasserstoff steht,
- R³: für eine Gruppe der Formel 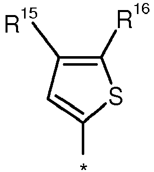 steht,
wobei
* die Anknüpfstelle an das Pyridin bedeutet,
und wobei
R¹⁵ für Wasserstoff, Methyl oder Ethyl steht,
R¹⁶ für Wasserstoff oder (C₁-C₄)-Alkyl steht,
worin (C₁-C₄)-Alkyl mit 1 oder 2 Substituenten unabhängig voneinander ausgewählt aus der Gruppe Hydroxy und Methoxy substituiert sein kann,
- R⁴: für Wasserstoff steht,
- R⁵: für Wasserstoff, Ethyl, n-Propyl oder sec.-Butyl steht,
wobei Ethyl, n-Propyl und sec.-Butyl mit 1 oder 2 Substituenten Hydroxy substituiert sein können,
oder
- R⁴ und R⁵: zusammen mit dem Stickstoffatom, an das sie gebunden sind, einen Azetidinyl- oder Pyrrolidinyl-Ring bilden, der mit einem Substituenten Hydroxy substituiert sein kann,
sowie ihre Salze, Solvate und Solvate der Salze.

Bevorzugt sind im Rahmen der vorliegenden Erfindung auch Verbindungen der Formel (I), in welcher
- R¹: für Oxazolyl, Thiazolyl oder Pyridyl steht,
wobei Pyridyl mit einem Substituenten ausgewählt aus der Gruppe Cyano, Methoxy, Ethoxy, Hydroxycarbonyl, Methoxycarbonyl, Ethoxycarbonyl, Aminocarbonyl, Methylaminocarbonyl oder Dimethylaminocarbonyl substituiert ist,
und
wobei Pyridyl mit einem Substituenten ausgewählt aus der Gruppe Fluor, Chlor, Methyl, Ethyl und Trifluormethyl substituiert sein kann,
und
wobei Oxazolyl und Thiazolyl mit einem Substituenten Phenyl substituiert sind,
worin Phenyl mit einem Substituenten ausgewählt aus der Gruppe Fluor, Chlor, Cyano, Methyl, Methoxy, Hydroxycarbonyl und Methoxycarbonyl substituiert sein können,
und
wobei Oxazolyl und Thiazolyl mit einem Substituenten ausgewählt aus der Gruppe Fluor, Methyl, Ethyl, Methoxy, Hydroxycarbonyl und Methoxycarbonyl substituiert sein können,
sowie ihre Salze, Solvate und Solvate der Salze.

Bevorzugt sind im Rahmen der vorliegenden Erfindung auch Verbindungen der Formel (I), in welcher
- R³: für eine Gruppe der Formel 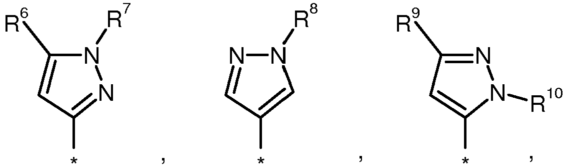 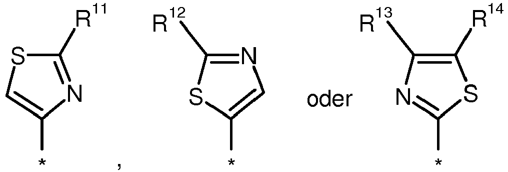 steht,
wobei
* die Anknüpfstelle an das Pyridin bedeutet,
und
wobei
R⁶ für Wasserstoff, Methyl oder Ethyl steht,
R⁷ für Wasserstoff oder (C₁-C₆)-Alkyl steht,
worin (C₁-C₆)-Alkyl mit 1 oder 2 Substituenten unabhängig voneinander ausgewählt aus der Gruppe Hydroxy und Methoxy substituiert sein kann,
R⁸ für Wasserstoff oder (C₁-C₆)-Alkyl steht,
worin (C₁-C₆)-Alkyl mit 1 oder 2 Substituenten unabhängig voneinander ausgewählt aus der Gruppe Hydroxy und Methoxy substituiert sein kann,
R⁹ für Wasserstoff, Methyl oder Ethyl steht,
R¹⁰ für Wasserstoff steht,
R¹¹ für Wasserstoff oder (C₁-C₆)-Alkyl steht,
worin (C₁-C₆)-Alkyl mit 1 oder 2 Substituenten unabhängig voneinander ausgewählt aus der Gruppe Hydroxy und Methoxy substituiert sein kann,
R¹² für Wasserstoff oder (C₁-C₆)-Alkyl steht,
worin (C₁-C₆)-Alkyl mit 1 oder 2 Substituenten unabhängig voneinander ausgewählt aus der Gruppe Hydroxy und Methoxy substituiert sein kann,
R¹³ für Wasserstoff, Methyl oder Ethyl steht, und
R¹⁴ für Wasserstoff oder (C₁-C₆)-Alkyl steht,
worin (C₁-C₆)-Alkyl mit 1 oder 2 Substituenten unabhängig voneinander ausgewählt aus der Gruppe Hydroxy und Methoxy substituiert sein kann,
sowie ihre Salze, Solvate und Solvate der Salze.

Weiterer Gegenstand der vorliegenden Erfindung ist ein Verfahren zur Herstellung der erfindungsgemäßen Verbindungen der Formel (I), dadurch gekennzeichnet, dass man
[A] eine Verbindung der Formel (II) 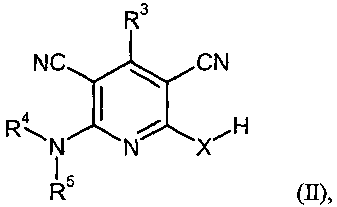
   in welcher X, R³, R⁴ und R⁵ jeweils die oben angegebenen Bedeutungen haben,
   in einem inerten Lösungsmittel in Gegenwart einer Base mit einer Verbindung der Formel (III) 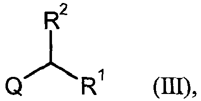
   in welcher R¹ und R² jeweils die oben angegebenen Bedeutungen haben und
      - Q: für eine geeignete Abgangsgruppe, vorzugsweise für Halogen, insbesondere Chlor, Brom oder Iod, oder für Mesylat, Tosylat oder Triflat steht,
      umsetzt
      oder
[B] im Fall, dass X für O steht, eine Verbindung der Formel (IV) 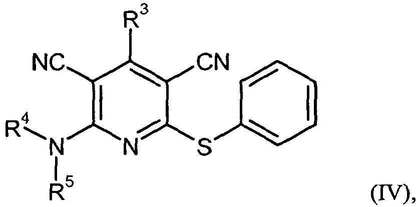
   in welcher R³, R⁴ und R⁵ jeweils die oben angegebenen Bedeutungen haben,
   in einem inerten Lösungsmittel in Gegenwart einer Base mit einer Verbindung der Formel (V) 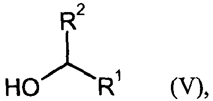 in welcher R¹ und R² die oben angegebenen Bedeutungen haben,
      umsetzt
      oder
[C] eine Verbindung der Formel (I-A) 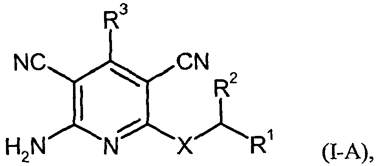
   in welcher R¹, R² und R³ jeweils die oben angegebenen Bedeutungen haben,
   zunächst mit Kupfer(II)chlorid und Isoamylnitrit in einem geeigneten Lösungsmittel in eine Verbindung der Formel (XV) 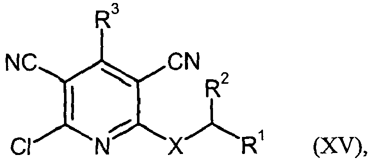
   in welcher R¹, R² und R³ jeweils die oben angegebenen Bedeutungen haben,
   überführt und diese anschließend mit einer Verbindung der Formel (VIII) 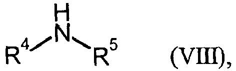
   in welcher R⁴ und R⁵ jeweils die oben angegebenen Bedeutungen haben,
   und
   wobei mindestens einer der beiden Reste R⁴ und R⁵ von Wasserstoff verschieden ist,
   zu einer Verbindung der Formel (I-B) 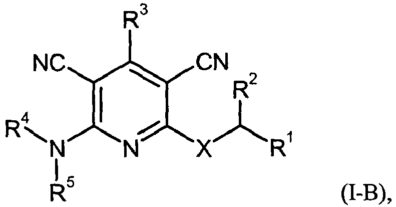
   in welcher R¹, R², R³, R⁴ und R⁵ jeweils die oben angegebenen Bedeutungen haben,
   und
   wobei mindestens einer der beiden Reste R⁴ und R⁵ von Wasserstoff verschieden ist,
   umsetzt,
anschließend gegebenenfalls vorhandene Schutzgruppen abspaltet und die resultierenden Verbindungen der Formel (I) gegebenenfalls mit den entsprechenden (*i*) Lösungsmitteln und/oder (*ii*) Basen oder Säuren in ihre Solvate, Salze und/oder Solvate der Salze überführt.

Die Verbindungen der Formel (I-A) und die Verbindungen der Formel (I-B) bilden zusammen die Verbindungen der Formel (I).

In den Verbindungen der Formeln (II) und (IV) bzw. in den Resten R³, R⁴ und/oder R⁵ gegebenenfalls vorhandene funktionelle Gruppen - wie insbesondere Amino-, Hydroxy- und Carboxylgruppen - können bei diesem Verfahren, falls zweckmäßig oder erforderlich, auch in temporär geschützter Form vorliegen. Die Einführung und Entfernung solcher Schutzgruppen erfolgt hierbei nach üblichen, dem Fachmann bekannten Methoden [siehe z.B. T.W. Greene und P.G.M. Wuts, Protective Groups in Organic Synthesis, Wiley, New York, 1999; M. Bodanszky und A. Bodanszky, The Practice of Peptide Synthesis, Springer-Verlag, Berlin, 1984]. Bei Vorhandensein mehrerer Schutzgruppen kann die Entfernung gegebenenfalls simultan in einer Eintopf-Reaktion oder in separaten Reaktionsschritten vorgenommen werden.

Als Amino-Schutzgruppe wird bevorzugt *tert*.-Butoxycarbonyl (Boc) oder Benzyloxycarbonyl (Z) verwendet. Zum Schutz von Carboxylgruppen eignen sich insbesondere die entsprechenden Methyl-, Ethyl- oder *tert*.-Butylester. Für eine Hydroxy-Funktion wird als Schutzgruppe vorzugsweise Benzyl oder eine Silylgruppe wie Trimethylsilyl, *tert*.-Butyldimethylsilyl oder Dimethylphenylsilyl eingesetzt. Bei Vorliegen einer 1,2- oder 1,3-Diol-Gruppierung wird bevorzugt ein von symmetrischen Ketonen wie Aceton oder Cyclohexanon abgeleitetes Ketal (1,3-Dioxolan bzw. 1,3-Dioxan) als gemeinsame Schutzgruppe verwendet.

Das zuvor beschriebene Verfahren kann durch die folgenden Reaktionsschemata 1 und 2 beispielhaft erläutert werden:

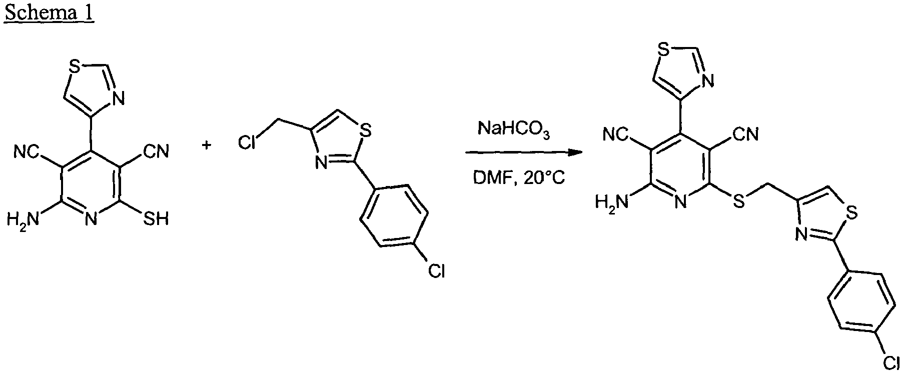

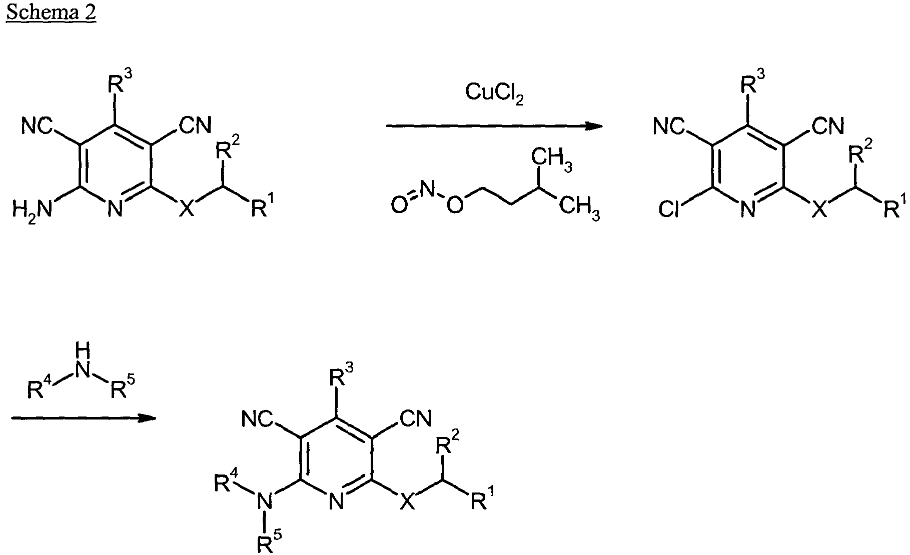

Als Lösungsmittel für die Reaktion (II) + (III) eignen sich alle organischen Lösungsmittel, die unter den Reaktionsbedingungen inert sind. Hierzu gehören Ketone wie Aceton und Methylethylketon, acyclische und cyclische Ether wie Diethylether, Methyl-tert.-butylether, 1,2-Dimethoxyethan, Tetrahydrofuran und Dioxan, Ester wie Essigsäureethylester oder Essigsäurebutylester, Kohlenwasserstoffe wie Benzol, Toluol, Xylol, Hexan und Cyclohexan, chlorierte Kohlenwasserstoffe wie Dichlormethan, Trichlormethan und Chlorbenzol, oder andere Lösungsmittel wie Dimethylformamid (DMF), Dimethylsulfoxid (DMSO), *N*-Methylpyrrolidinon (NMP), Acetonitril oder Pyridin. Ebenso ist es möglich, Gemische der zuvor genannten Lösungsmittel einzusetzen. Bevorzugt wird Dimethylformamid verwendet.

Als Basen für diese Umsetzung eignen sich die üblichen anorganischen oder organischen Basen. Hierzu gehören bevorzugt Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, Amide wie Natriumamid, Lithium-, Natrium- oder Kalium-bis-(trimethylsilyl)amid oder Lithiumdiisopropylamid, metallorganische Verbindungen wie Butyllithium oder Phenyllithium, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt sind Alkalicarbonate und -hydrogencarbonate wie Kaliumcarbonat und Natriumhydrogencarbonat.

Die Base kann hierbei in einer Menge von 1 bis 10 Mol, bevorzugt von 1 bis 5 Mol, insbesondere von 1 bis 3 Mol, bezogen auf 1 Mol der Verbindung der Formel (II), eingesetzt werden.

Die Reaktion (II) + (III) erfolgt im Allgemeinen in einem Temperaturbereich von -78°C bis +140°C, bevorzugt im Bereich von -20°C bis +100°C, insbesondere bei 0°C bis +60°C (für X = S) bzw. +20°C bis +100°C (für X = O), gegebenenfalls in einer Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Als inerte Lösungsmittel für die Reaktion (IV) + (V) eignen sich insbesondere acyclische und cyclische Ether wie Diethylether, Methyl-tert.-butylether, 1,2-Dimethoxyethan, Tetrahydrofuran und Dioxan, Kohlenwasserstoffe wie Benzol, Toluol, Xylol, Hexan und Cyclohexan, oder dipolare Lösungsmittel wie Dimethylformamid (DMF), Dimethylsulfoxid (DMSO), *N*-Methylpyrrolidinon (NMP) und Pyridin. Ebenso ist es möglich, Gemische dieser Lösungsmittel einzusetzen. Bevorzugt wird 1,2-Dimethoxyethan verwendet.

Als Basen für diese Umsetzung sind insbesondere Alkalialkoholate wie Natrium- oder Kaliummethanolat, Natrium- oder Kaliumethanolat oder Natrium- oder Kalium-tert.-butylat, Amide wie Natriumamid, Lithium-, Natrium- oder Kalium-bis-(trimethylsilyl)amid oder Lithiumdiisopropylamid, oder metallorganische Verbindungen wie Butyllithium oder Phenyllithium geeignet. Bevorzugt wird Kalium-tert.-butylat verwendet.

Die Base wird hierbei in der Regel in einer Menge von 1 bis 1.25 Mol, bevorzugt in äquimolarer Menge, bezogen auf 1 Mol der Verbindung der Formel (V), eingesetzt.

Die Reaktion (IV) + (V) erfolgt im Allgemeinen in einem Temperaturbereich von -20°C bis +120°C, bevorzugt bei +20°C bis +100°C, gegebenenfalls in einer Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Der Verfahrensschritt (I-A) → (XV) erfolgt im Allgemeinen mit einem Molverhältnis von 2 bis 12 Mol Kupfer(II)chlorid und 2 bis 12 Mol Isoamylnitrit bezogen auf 1 Mol der Verbindung der Formel (I-A).

Als Lösungsmittel für diesen Verfahrensschritt eignen sich alle organischen Lösungsmittel, die unter den Reaktionsbedingungen inert sind. Hierzu gehören acyclische und cyclische Ether wie Diethylether und Tetrahydrofuran, Ester wie Essigsäureethylester oder Essigsäurebutylester, Kohlenwasserstoffe wie Benzol, Toluol, Xylol, Hexan und Cyclohexan, chlorierte Kohlenwasserstoffe wie Dichlormethan, 1,2-Dichlorethan und Chlorbenzol, oder andere Lösungsmittel wie Dimethylformamid, Acetonitril oder Pyridin. Ebenso ist es möglich, Gemische dieser Lösungsmittel einzusetzen. Bevorzugte Lösungsmittel sind Acetonitril und Dimethylformamid.

Die Reaktion erfolgt im Allgemeinen in einem Temperaturbereich von -78°C bis +180°C, bevorzugt im Bereich von +20°C bis +100°C, insbesondere bei +20°C bis +60°C, gegebenenfalls in einer Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Der Verfahrensschritt (XV) + (VIII) erfolgt im Allgemeinen mit einem Molverhältnis von 1 bis 8 Mol der Verbindung der Formel (VIII) bezogen auf 1 Mol der Verbindung der Formel (XV).

Als Lösungsmittel für diesen Verfahrensschritt eignen sich alle organischen Lösungsmittel, die unter den Reaktionsbedingungen inert sind. Hierzu gehören Alkohole wie Methanol, Ethanol, n-Propanol, Isopropanol, n-Butanol und tert.-Butanol, Ketone wie Aceton und Methylethylketon, acyclische und cyclische Ether wie Diethylether, 1,2-Dimethoxyethan, Tetrahydrofuran und Dioxan, Ester wie Essigsäureethylester oder Essigsäurebutylester, Kohlenwasserstoffe wie Benzol, Toluol, Xylol, Hexan und Cyclohexan, chlorierte Kohlenwasserstoffe wie Dichlormethan, 1,2-Dichlorethan und Chlorbenzol, oder andere Lösungsmittel wie Dimethylformamid, Acetonitril, Pyridin oder Dimethylsulfoxid. Wasser ist als Lösungsmittel ebenfalls geeignet. Ebenso ist es möglich, Gemische dieser Lösungsmittel einzusetzen. Bevorzugtes Lösungsmittel ist Dimethylformamid.

Die Reaktion erfolgt im Allgemeinen in einem Temperaturbereich von 0°C bis +180°C, bevorzugt im Bereich von +20°C bis +120°C, insbesondere bei +20°C bis +100°C, gegebenenfalls in einer Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Die Verbindungen der Formel (VIII) sind entweder kommerziell erhältlich, dem Fachmann bekannt oder nach üblichen Methoden herstellbar.

Die Verbindungen der Formel (III) sind kommerziell erhältlich, literaturbekannt oder nach literaturbekannten Methoden herstellbar. So können beispielsweise durch Reaktion von Amiden bzw. Thioamiden mit einem 1,3-Dihalogenaceton substituierte Oxazol- und Thiazol-Derivate der Formel (III-A) und (III-B) erhalten werden (siehe Schema 3):

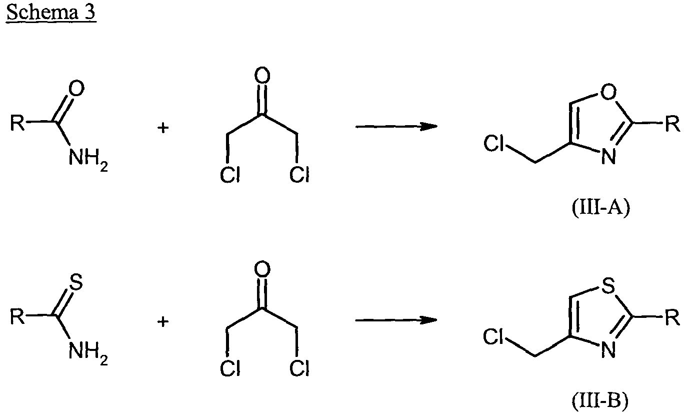

Die Verbindungen der Formel (V) sind gleichfalls kommerziell erhältlich oder literaturbekannt oder sie können in Analogie zu literaturbeschriebenen Verfahren, beispielsweise ähnlich wie die Verbindungen der Formel (III), hergestellt werden.

Verbindungen der Formel (II), worin X für S und R⁴ und R⁵ für Wasserstoff stehen, können in Analogie zu literaturbekannten Methoden beispielsweise dadurch hergestellt werden, dass man Aldehyde der Formel (VI) 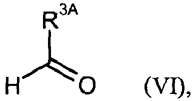 in welcher
- R^{3A}: für Thienyl, C-gebundenes Pyrrolyl, Oxazolyl, Thiazolyl, Isoxazolyl, Isothiazolyl, C-gebundenes Pyrazolyl, C-gebundenes Imidazolyl, C-gebundenes Triazolyl, Oxadiazolyl, Thiadiazolyl oder C-gebundenes Tetrazolyl steht,
welche in dem für R³ angegebenen Bedeutungsumfang substituiert sein können,

in Gegenwart einer Base mit zwei Äquivalenten Cyanothioacetamid umsetzt [siehe Schema 4; vgl. z.B. Dyachenko et al., Russ. J. Chem. 33 (7), 1014-1017 (1997), 34 (4), 557-563 (1998); Dyachenko et al., Chemistry of Heterocyclic Compounds 34 (2), 188-194 (1998); Qintela et al., Eur. J. Med. Chem. 33, 887-897 (1998); Kandeel et al., Z. Naturforsch. 42b, 107-111 (1987); Reddy et al., J. Med. Chem. 49, 607-615 (2006); Evdokimov et al., Org. Lett. 8, 899-902 (2006)].

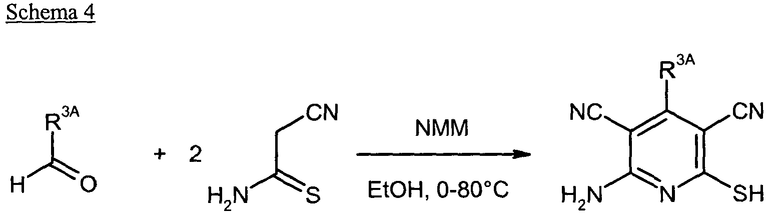

Die Verbindungen der Formel (IV) können in Analogie zu literaturbeschriebenen Verfahren hergestellt werden [vgl. z.B. Kambe et al., Synthesis, 531-533 (1981); Elnagdi et al., Z. Naturforsch. 47b, 572-578 (1991); Reddy et al., J. Med. Chem. 49, 607-615 (2006); Evdokimov et al., Org. Lett. 8, 899-902 (2006)] oder durch Umsetzung von Verbindungen der Formel (II), in welcher X für S steht, in Analogie zu literaturbeschriebenen Verfahren [vgl. z. B. Fujiwara, H. et al., Heterocycles 1993, 36 (5), 1105-1113, Su et al., J. Med Chem. 1988, 31, 1209-1215].

Die Verbindungen der Formel (VI) sind kommerziell erhältlich, literaturbekannt oder nach literaturbekannten Methoden herstellbar.

Verbindungen der Formel (II), worin X für S steht, können auch ausgehend von Verbindungen der Formel (IV) durch Reaktion mit einem Alkalisulfid erhalten werden. Diese Herstellungsmethode wird durch das folgende Formelschema 5 erläutert:

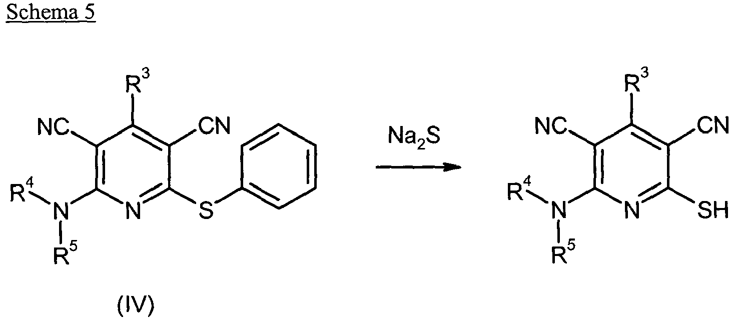

Als Alkalisulfid wird vorzugsweise Natriumsulfid in einer Menge von 1 bis 10 Mol, bevorzugt von 1 bis 8 Mol, insbesondere von 1 bis 5 Mol, bezogen auf 1 Mol der Verbindung der Formel (IV), eingesetzt.

Als Lösungsmittel für diesen Verfahrensschritt eignen sich alle organischen Lösungsmittel, die unter den Reaktionsbedingungen inert sind. Hierzu gehören Alkohole wie Methanol, Ethanol, n-Propanol, Isopropanol, n-Butanol und tert.-Butanol, Ketone wie Aceton und Methylethylketon, acyclische und cyclische Ether wie Diethylether, 1,2-Dimethoxyethan, Tetrahydrofuran und Dioxan, Ester wie Essigsäureethylester oder Essigsäurebutylester, Kohlenwasserstoffe wie Benzol, Toluol, Xylol, Hexan und Cyclohexan, chlorierte Kohlenwasserstoffe wie Dichlormethan, 1,2-Dichlorethan und Chlorbenzol, oder dipolare Lösungsmittel wie Acetonitril, Pyridin, Dimethylformamid, Dimethylsulfoxid oder *N*-Methylpyrrolidinon. Wasser ist als Lösungsmittel ebenfalls geeignet. Ebenso ist es möglich, Gemische der zuvor genannten Lösungsmittel einzusetzen. Bevorzugtes Lösungsmittel ist Dimethylformamid.

Die Reaktion erfolgt im Allgemeinen in einem Temperaturbereich von 0°C bis +180°C, bevorzugt im Bereich von +20°C bis +120°C, insbesondere bei +40°C bis +100°C, gegebenenfalls in einer Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Verbindungen der Formel (IV), worin mindestens einer der beiden Reste R⁴ und R⁵ nicht für Wasserstoff steht, können hergestellt werden, indem man Verbindungen der Formel (IVa) 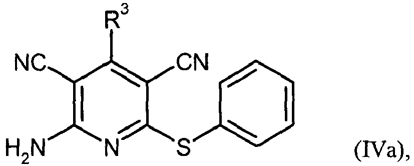
in welcher R³ die oben angegebene Bedeutung hat,
zunächst mit Kupfer(II)chlorid und Isoamylnitrit in einem geeigneten Lösungsmittel in Verbindungen der Formel (VII) 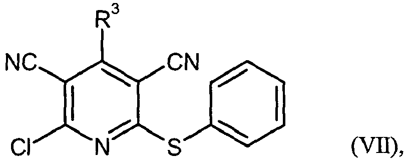
in welcher R³ die oben angegebene Bedeutung hat,
überführt und anschließend mit einer Verbindung der Formel (VIII) 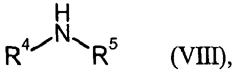
in welcher R⁴ und R⁵ jeweils die oben angegebenen Bedeutungen haben,
und
wobei mindestens einer der beiden Reste R⁴ und R⁵ von Wasserstoff verschieden ist,
zu Verbindungen der Formel (IVb) 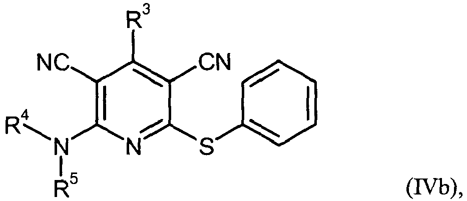
in welcher R³, R⁴ und R⁵ jeweils die oben angegebenen Bedeutungen haben,
umsetzt; gegebenenfalls können diese dann mit Hilfe eines Alkalisulfids, wie oben beschrieben, in entsprechende Verbindungen der Formel (II), worin X für S steht und mindestens einer der beiden Reste R⁴ und R⁵ nicht für Wasserstoff steht, überführt werden. Dieses Verfahren kann durch das folgende Reaktionsschema veranschaulicht werden: 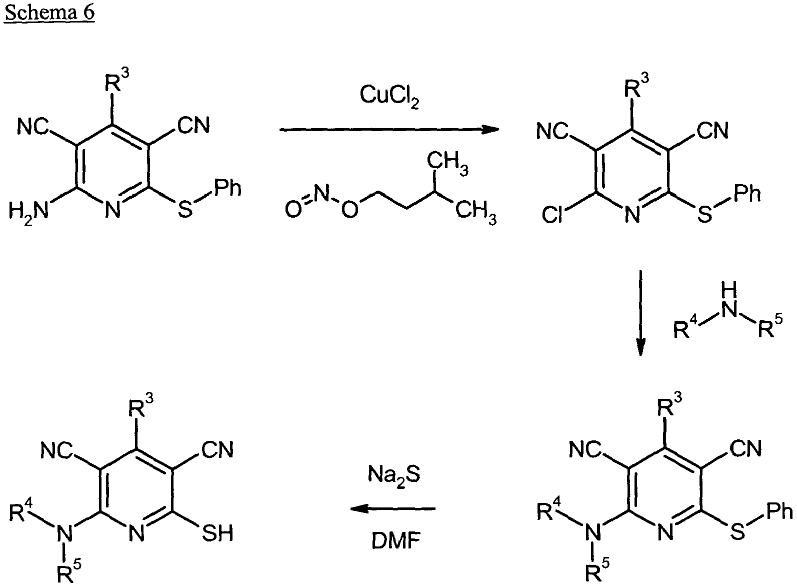
[Ph = Phenyl].

Für diesen Verfahrensweg finden die zuvor für die Sequenz (I-A) → (XV) → (I-B) beschriebenen Reaktionsparameter wie Lösungsmittel, Reaktionstemperaturen und Molverhältnisse in analoger Weise Anwendung.

Verbindungen der Formel (II), worin X für O steht, können ausgehend von Verbindungen der Formel (IV) durch Erhitzen mit einem Alkalihydroxid erhalten werden. Diese Herstellungsmethode wird durch das folgende Reaktionsschema erläutert:

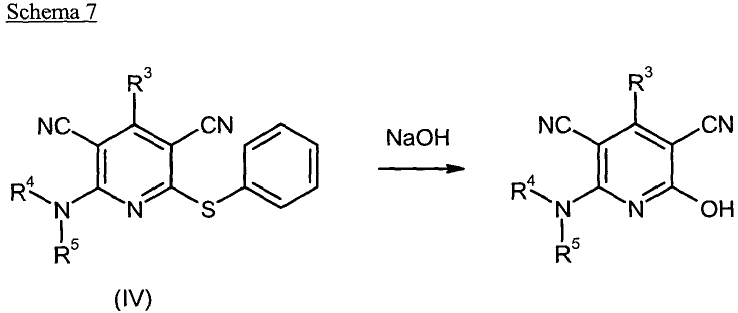

Als Alkalihydroxid wird vorzugsweise überschüssiges Natrium- oder Kaliumhydroxid eingesetzt. Als Lösungsmittel sind insbesondere Alkohole wie Methanol, Ethanol, n-Propanol, Isopropanol, n-Butanol und tert.-Butanol sowie deren Mischungen mit Wasser geeignet. Die Umsetzung erfolgt im Allgemeinen in einem Temperaturbereich von +20°C bis +120°C, bevorzugt bei +50°C bis +100°C.

Weitere Verbindungen der Formel (II), in welcher X für S und R⁴ und R⁵ für Wasserstoff stehen, können hergestellt werden, indem man die Verbindung der Formel (IX) 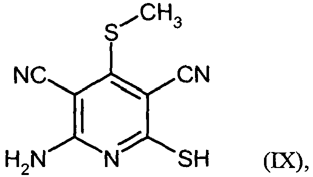 in einem inerten Lösungsmittel in Gegenwart einer Base mit einer Verbindung der Formel (III) in eine Verbindung der Formel (X) 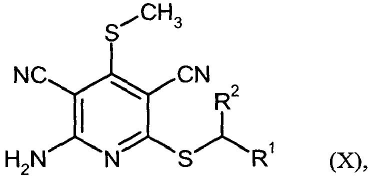
in welcher R¹ und R² die oben angegebenen Bedeutungen haben,
überführt und diese dann in einem inerten Lösungsmittel oder ohne Lösungsmittel mit einer Verbindung der Formel (XI)

   R^{3B}-H (XI),

   in welcher
   - R^{3B}: für N-gebundenes Pyrrolyl, Pyrazolyl, Imidazolyl, Triazolyl oder Tetrazolyl steht, welche jeweils wie im für R³ angegebenen Bedeutungsumfang substituiert sein können,
zu Verbindungen der Formel (II-B) 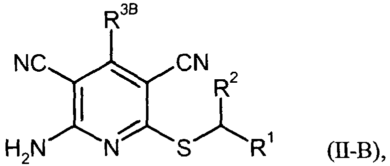
in welcher R¹, R² und R^{3B} jeweils die oben angegebenen Bedeutungen haben,
umsetzt.

Als Lösungsmittel für den Verfahrensschritt (IX) + (III) → (X) eignen sich alle organischen Lösungsmittel, die unter den Reaktionsbedingungen inert sind. Hierzu gehören Alkohole wie Methanol, Ethanol, n-Propanol, Isopropanol, n-Butanol und tert.-Butanol, Ketone wie Aceton und Methylethylketon, acyclische und cyclische Ether wie Diethylether, Methyl-tert.-butylether, 1,2-Dimethoxyethan, Tetrahydrofuran und Dioxan, Ester wie Essigsäureethylester oder Essigsäurebutylester, Kohlenwasserstoffe wie Benzol, Toluol, Xylol, Hexan und Cyclohexan, chlorierte Kohlenwasserstoffe wie Dichlormethan, Trichlormethan und Chlorbenzol, oder andere Lösungsmittel wie Dimethylformamid (DMF), Dimethylsulfoxid (DMSO), *N*-Methylpyrrolidinon (NMP), Acetonitril oder Pyridin. Wasser ist als Lösungsmittel ebenfalls geeignet. Ebenso ist es möglich, Gemische der zuvor genannten Lösungsmittel einzusetzen. Bevorzugt wird Dimethylformamid als Lösungsmittel verwendet.

Als Base für den Verfahrensschritt (IX) + (III) → (X) eignen sich die üblichen anorganischen oder organischen Basen. Hierzu gehören bevorzugt Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, Amide wie Natriumamid, Lithium-, Natrium- oder Kalium-bis-(trimethylsilyl)amid oder Lithiumdiisopropylamid, metallorganische Verbindungen wie Butyllithium oder Phenyllithium, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt sind Alkalicarbonate und -hydrogencarbonate.

Die Base kann hierbei in einer Menge von 1 bis 10 Mol, bevorzugt von 1 bis 5 Mol, insbesondere von 1 bis 4 Mol, bezogen auf 1 Mol der Verbindung der Formel (IX), eingesetzt werden.

Die Reaktion erfolgt im Allgemeinen in einem Temperaturbereich von -78°C bis +140°C, bevorzugt im Bereich von -20°C bis +80°C, insbesondere bei 0°C bis +50°C, gegebenenfalls in der Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Als Lösungsmittel für den Verfahrensschritt (X) + (XI) → (II-B) eignen sich alle organischen Lösungsmittel, die unter den Reaktionsbedingungen inert sind. Hierzu gehören Alkohole wie Methanol, Ethanol, n-Propanol, Isopropanol, n-Butanol und tert.-Butanol, Ketone wie Aceton und Methylethylketon, acyclische und cyclische Ether wie Diethylether, Methyl-tert.-butylether, 1,2-Dimethoxyethan, Tetrahydrofuran und Dioxan, Ester wie Essigsäureethylester oder Essigsäurebutylester, Kohlenwasserstoffe wie Benzol, Toluol, Xylol, Hexan und Cyclohexan, chlorierte Kohlenwasserstoffe wie Dichlormethan oder Chlorbenzol, oder andere Lösungsmittel wie Dimethylformamid (DMF), Dimethylsulfoxid (DMSO), *N*-Methylpyrrolidinon (NMP), Acetonitril oder Pyridin. Wasser ist als Lösungsmittel ebenfalls geeignet. Ebenso ist es möglich, Gemische der zuvor genannten Lösungsmittel einzusetzen. Gegebenenfalls kann die Reaktion auch vorteilhaft in Gegenwart eines Überschusses der Verbindung (XI) ohne Zusatz eines weiteren Lösungsmittels durchgeführt werden. Bevorzugt wird die Umsetzung in Aceton oder *N*-Methylpyrrolidinon als Lösungsmittel durchgeführt.

Der Verfahrensschritt (X) + (XI) → (II-B) erfolgt im Allgemeinen in einem Temperaturbereich von 0°C bis +180°C, bevorzugt im Bereich von +20°C bis +100°C, insbesondere bei +60°C bis +100°C, gegebenenfalls in der Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Die Verbindungen der Formel (XI) sind kommerziell erhältlich, literaturbekannt oder können in Analogie zu literaturbekannten Verfahren hergestellt werden.

Die Verbindung der Formel (IX) lässt sich auf einfache Weise durch Umsetzung von [Bis(methylthio)methylen]malononitril mit Cyanothioacetamid in Gegenwart einer Base wie Triethylamin erhalten.

Weitere Verbindungen der Formel (II), in welcher X für O und R⁴ und R⁵ für Wasserstoff stehen, können hergestellt werden, indem man die Verbindung der Formel (XII) 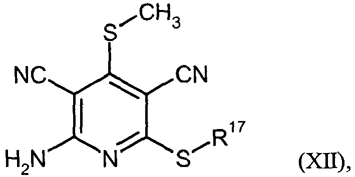 in welcher
- R¹⁷: für (C₁-C₄)-Alkyl oder Phenyl steht,
in einem inerten Lösungsmittel in Gegenwart einer Base mit einer Verbindung der Formel (V) in eine Verbindung der Formel (XIII) 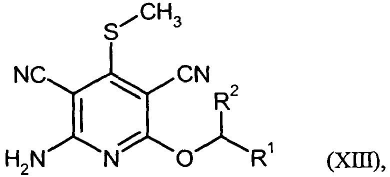
in welcher R¹ und R² die oben angegebenen Bedeutungen haben,
überführt und diese dann in einem inerten Lösungsmittel oder ohne Lösungsmittel mit einer Verbindung der Formel (XI) zu Verbindungen der Formel (II-C) 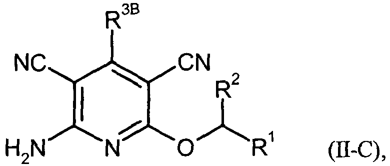
   in welcher R¹, R² und R^{3B} jeweils die oben angegebenen Bedeutungen haben,
   umsetzt, oder
   alternativ eine Verbindung der Formel (XII) zuerst in einem inerten Lösungsmittel oder ohne Lösungsmittel mit einer Verbindung der Formel (XI) zu Verbindungen der Formel (XIV) 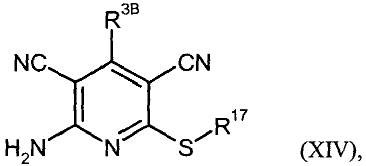
   in welcher R^{3B} und R¹⁷ jeweils die oben angegebenen Bedeutungen haben,
   umsetzt, und diese anschliessend mit einer Verbindung der Formel (V) in Verbindungen der Formel (II-C) überführt.

Die Verbindungen der Formel (XII), in welcher R¹⁷ für Phenyl steht, lassen sich aus der Verbindung der Formel (IX) in Analogie zu dem in Fujiwara, H. et al., Heterocycles 1993, 36 (5), 1105-1113 beschriebenen Verfahren herstellen.

Die Verbindungen der Formel (XII), in welcher R" für (C₁-C₄)-Alkyl steht, lassen sich aus der Verbindung der Formel (IX) in Analogie zu dem in Su et al., J. Med Chem. 1988, 31, 1209-1215 beschriebenen Verfahren herstellen.

Als inerte Lösungsmittel für die Reaktionen (XII) + (V) und (XIV) + (V) eignen sich insbesondere acyclische und cyclische Ether wie Diethylether, Methyl-tert.-butylether, 1,2-Dimethoxyethan, Tetrahydrofuran und Dioxan, Kohlenwasserstoffe wie Benzol, Toluol, Xylol, Hexan und Cyclohexan, oder andere Lösungsmittel wie Dimethylformamid (DMF), Dimethylsulfoxid (DMSO), *N*-Methylpyrrolidinon (NMP) und Pyridin. Ebenso ist es möglich, Gemische dieser Lösungsmittel einzusetzen. Bevorzugt wird 1,2-Dimethoxyethan verwendet.

Als Basen für diese Umsetzungen sind insbesondere Alkalialkoholate wie Natrium- oder Kaliummethanolat, Natrium- oder Kaliumethanolat oder Natrium- oder Kalium-tert.-butylat, Alkalihydride wie Lithium-, Natrium- oder Kaliumhydrid, Amide wie Natriumamid, Lithium-, Natrium- oder Kalium-bis-(trimethylsilyl)amid oder Lithiumdiisopropylamid, oder metallorganische Verbindungen wie Butyllithium oder Phenyllithium geeignet. Bevorzugt wird Kalium-tert.-butylat verwendet.

Die Base wird hierbei in der Regel in einer Menge von 1 bis 1.25 Mol, bevorzugt in äquimolarer Menge, bezogen auf 1 Mol der Verbindung der Formel (V), eingesetzt.

Die Reaktionen (XII) + (V) und (XIV) + (V) erfolgen im Allgemeinen in einem Temperaturbereich von -20°C bis +120°C, bevorzugt bei +20°C bis +100°C, gegebenenfalls in der Mikrowelle. Die Umsetzungen können bei normalem, erhöhtem oder erniedrigtem Druck durchgeführt werden (z.B. im Bereich von 0.5 bis 5 bar). Im Allgemeinen arbeitet man bei Normaldruck.

Als Lösungsmittel für die Verfahrensschritte (XII) bzw. (XIII) + (XI) → (II-C) eignen sich alle organischen Lösungsmittel, die unter den Reaktionsbedingungen inert sind. Hierzu gehören Alkohole wie Methanol, Ethanol, n-Propanol, Isopropanol, n-Butanol und tert.-Butanol, Ketone wie Aceton und Methylethylketon, acyclische und cyclische Ether wie Diethylether, Methyl-tert.-butylether, 1,2-Dimethoxyethan, Tetrahydrofuran und Dioxan, Ester wie Essigsäureethylester oder Essigsäurebutylester, Kohlenwasserstoffe wie Benzol, Toluol, Xylol, Hexan und Cyclohexan, chlorierte Kohlenwasserstoffe wie Dichlormethan oder Chlorbenzol, oder andere Lösungsmittel wie Dimethylformamid (DMF), Dimethylsulfoxid (DMSO), *N*-Methylpyrrolidinon (NMP), Acetonitril oder Pyridin. Wasser ist als Lösungsmittel ebenfalls geeignet. Ebenso ist es möglich, Gemische der zuvor genannten Lösungsmittel einzusetzen. Gegebenenfalls kann die Reaktion auch vorteilhaft in Gegenwart eines Überschusses der Verbindung (XI) ohne Zusatz eines weiteren Lösungsmittels durchgeführt werden. Bevorzugt wird die Umsetzung in Aceton oder *N*-Methylpyrrolidinon als Lösungsmittel durchgeführt.

Die Verfahrensschritte (XII) bzw. (XIII) + (XI) → (II-C) erfolgen im Allgemeinen in einem Temperaturbereich von 0°C bis +180°C, bevorzugt im Bereich von +20°C bis +100°C, insbesondere bei +60°C bis +100°C, gegebenenfalls in der Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

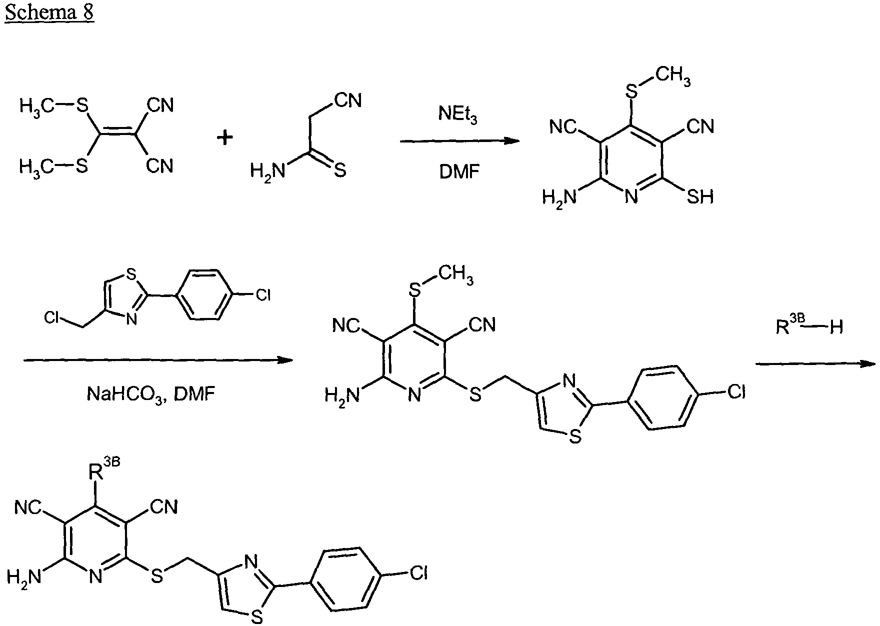

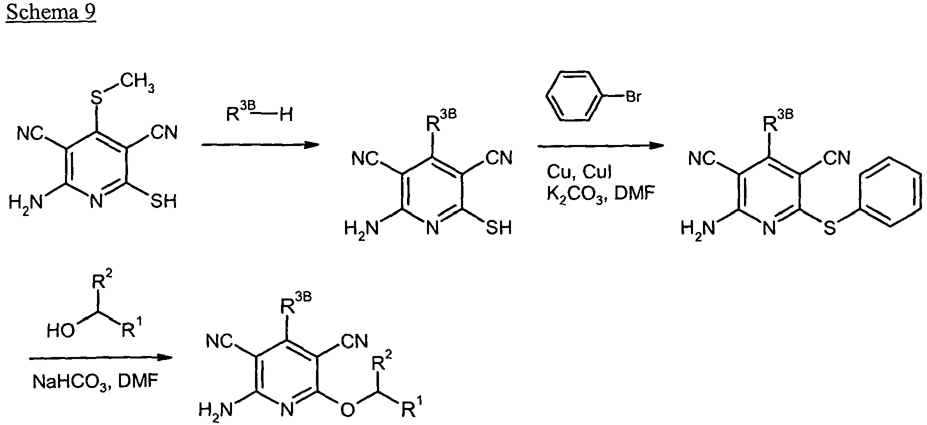

Weitere erfindungsgemäße Verbindungen können gegebenenfalls auch hergestellt werden durch Umwandlungen von funktionellen Gruppen einzelner Substituenten, insbesondere den unter R¹, R², R³, R⁴ und R⁵ aufgeführten, ausgehend von den nach obigen Verfahren erhaltenen Verbindungen der Formel (I). Diese Umwandlungen werden nach üblichen, dem Fachmann bekannten Methoden durchgeführt und umfassen beispielsweise Reaktionen wie nukleophile und elektrophile Substitutionen, Oxidationen, Reduktionen, Hydrierungen, Übergangsmetall-katalysierte Kupplungsreaktionen, Eliminierungen, Alkylierung, Aminierung, Veresterung, Esterspaltung, Veretherung, Etherspaltung, Bildung von Carbonamiden, sowie Einführung und Entfernung temporärer Schutzgruppen. Diese Verfahren werden durch die folgenden Reaktionsschemata beispielhaft veranschaulicht:

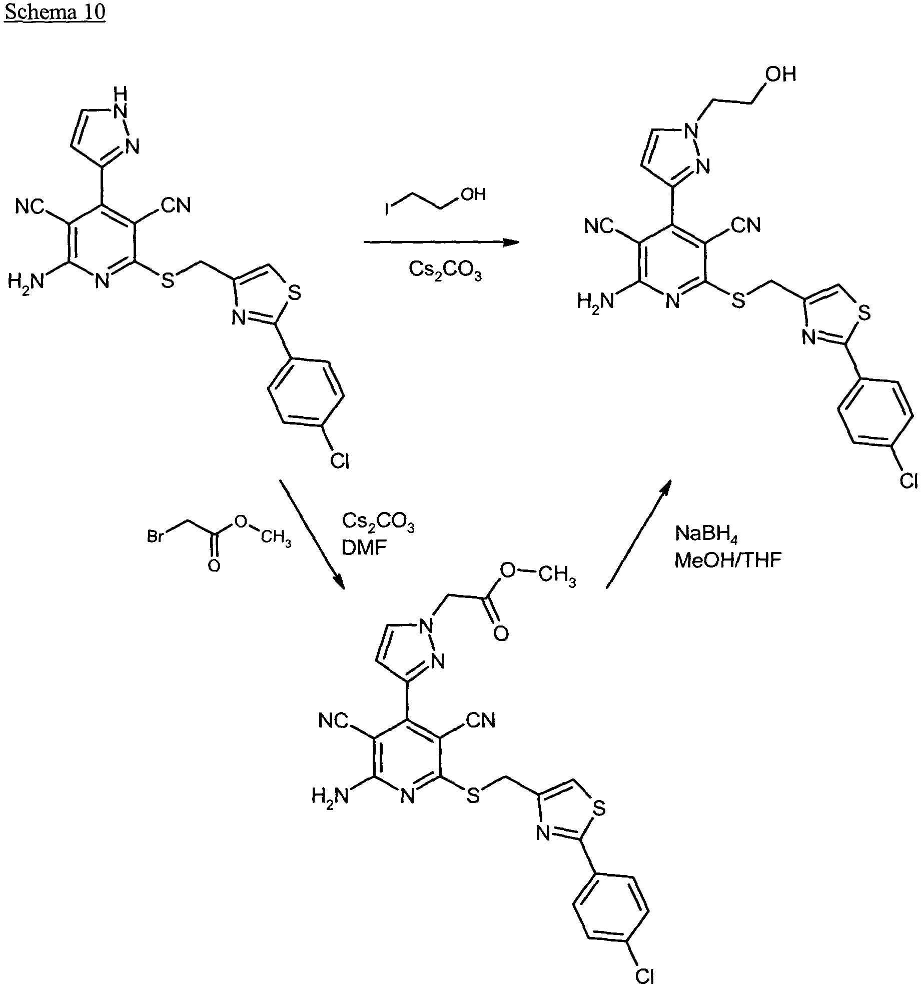

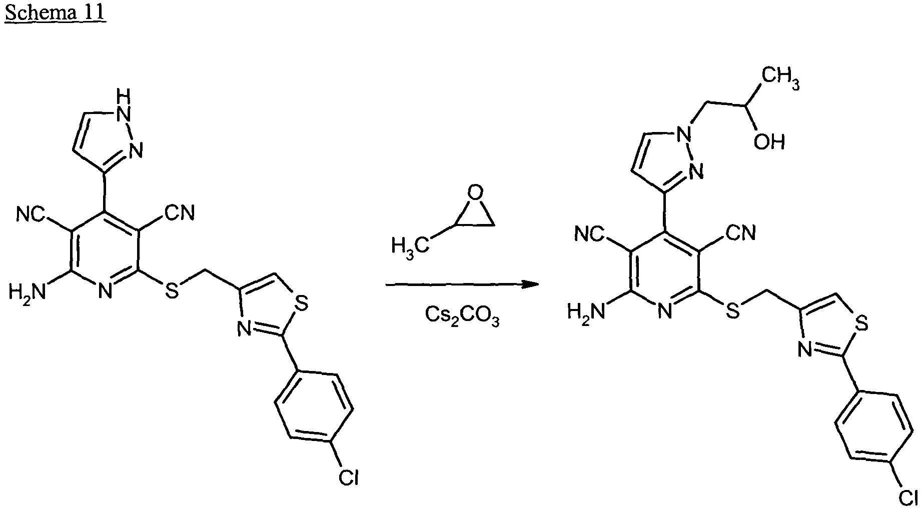

Überraschenderweise zeigen die erfindungsgemäßen Verbindungen ein nicht vorhersehbares, wertvolles pharmakologisches Wirkspektrum und sind daher insbesondere zur Prävention und/oder Behandlung von Erkrankungen geeignet.

Die pharmazeutische Wirksamkeit der erfindungsgemäßen Verbindungen lässt sich durch ihre Wirkung als potente, selektive Liganden an den Adenosin A1-Rezeptor erklären. Sie wirken hierbei als selektive A1-Agonisten oder selektive A1-Antagonisten. Die erfindungsgemäßen Verbindungen zeigen ein gleiches oder verbessertes physikochemisches, pharmakokinetisches und/ oder therapeutisches Profil. Die erfindungsgemäßen Verbindungen wirken vorwiegend als selektive Adenosin A1-Agonisten.

Als "selektive Liganden an den Adenosin A1-Rezeptor" werden im Rahmen der vorliegenden Erfindung solche Adenosin-Rezeptorliganden bezeichnet, bei denen einerseits eine deutliche Wirkung am A1-Adenosinrezeptor und andererseits keine oder eine deutliche schwächere Wirkung (Faktor 10 oder höher) an A2a-, A2b- und A3-Adenosinrezeptor-Subtypen zu beobachten ist, wobei bezüglich der Testmethoden für die Wirk-Selektivität Bezug genommen wird auf die im Abschnitt B-1. und B-5. beschriebenen Tests.

Die erfindungsgemäßen Verbindungen können in Abhängigkeit von ihrer jeweiligen Struktur als volle, als partielle Adenosinrezeptor-Agonisten oder als Adenosinrezeptor-Antagonisten fungieren. Partielle Adenosinrezeptor-Agonisten sind hierbei definiert als Rezeptorliganden, die eine funktionelle Antwort an Adenosinrezeptoren auslösen, welche geringer ist als bei vollen Agonisten (wie beispielsweise Adenosin selbst). Partielle Agonisten weisen infolgedessen eine geringere Wirksamkeit bezüglich der Rezeptoraktivierung auf als volle Agonisten.

Die Verbindungen der Formel (I) sind allein oder in Kombination mit einem oder mehreren anderen Wirkstoffen zur Prävention und/oder Behandlung verschiedener Erkrankungen geeignet, so beispielsweise Erkrankungen des Herzkreislauf-Systems (kardiovaskulären Erkrankungen), zur Kardioprotektion nach Schädigungen des Herzens sowie von Stoffwechsel- und Nierenerkrankungen.

Im Sinne der vorliegenden Erfindung sind unter Erkrankungen des Herzkreislauf-Systems bzw. kardiovaskulären Erkrankungen beispielsweise die folgenden Erkrankungen zu verstehen: periphere und kardiale Gefäßerkrankungen, koronare Herzerkrankung, koronare Restenose wie z.B. Restenose nach Ballondilatation von peripheren Blutgefäßen, Myokardinfarkt, akutes Koronarsyndrom, akutes Koronarsyndrom mit ST-Erhebung, akutes Koronarsyndrom ohne ST-Erhebung, stabile und instabile Angina pectoris, Herzmuskelschwäche, Prinzmetal Angina, persistierende ischämische Dysfunktion (hibernating Myokardium), vorübergehende postischämische Dysfunktion (stunned Myokardium), Herzinsuffizienz, Tachykardien, atriale Tachykardie, Arrhythmien, Vorhof- und Kammerflimmern, persistierendes Vorhofflimmern, permanentes Vorhofflimmern, Vorhofflimmern mit normaler linksventrikulärer Funktion, Vorhofflimmern mit eingeschränkter linksventrikulärer Funktion, Wolff-Parkinson-White Syndrom, periphere Durchblutungsstörungen, erhöhte Spiegel von Fibrinogen und von LDL geringer Dichte, sowie erhöhte Konzentrationen von Plasminogenaktivator-Inhibitor 1 (PAI-1), insbesondere koronare Herzerkrankung, akutes Koronarsyndrom, Angina pectoris, Herzinsuffizienz, Myokardinfarkt und Vorhofflimmern

Im Sinne der vorliegenden Erfindung umfasst der Begriff Herzinsuffizienz sowohl akute als auch chronische Erscheinungsformen der Herzinsuffizienz, wie auch spezifischere oder verwandte Krankheitsformen wie akute dekompensierte Herzinsuffizienz, Rechtsherzinsuffizienz, Linksherzinsuffizienz, Globalinsuffizienz, ischämische Kardiomyopathie, dilatative Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische sowie systolische Herzinsuffizienz.

Weiterhin eignen sich die erfindungsgemäßen Verbindungen auch zur Reduktion des von einem Infarkt betroffenen Myokardbereichs sowie zur Prävention von Sekundärinfarkten.

Des weiteren sind die erfindungsgemäßen Verbindungen zur Prävention und/oder Behandlung von thromboembolischen Erkrankungen, Reperfusionsschäden nach Ischämie, mikro- und makrovaskulären Schädigungen (Vaskulitis), arteriellen sowie venösen Thrombosen, Ödemen, Ischämien wie Myokardinfarkt, Hirnschlag und transitorischen ischämischen Attacken, zur Kardioprotektion bei Koronararterien Bypass Operationen (CABG), primären PTCAs, PTCAs nach Thrombolyse, Rescue-PTCA, Herztransplantationen und Operationen am offenen Herzen, sowie zur Organprotektion bei Transplantationen, Bypass Operationen, Herzkatheteruntersuchungen und anderen operativen Eingriffen geeignet.

Weiterhin eignen sich die erfindungsgemäßen Verbindungen zur Behandlung und/oder Prävention von Nierenerkrankungen, insbesondere von Niereninsuffizienz. Im Sinne der vorliegenden Erfindung umfasst der Begriff Niereninsuffizienz sowohl akute als auch chronische Erscheinungsformen der Niereninsuffizienz, wie auch zugrundeliegende oder verwandte Nierenerkrankungen wie renale Hypoperfusion, obstruktive Uropathie, Glomerulonephritis, akute Glomerulonephritis, tubulointerstitielle Erkrankungen, nephropathische Erkrankungen wie primäre und angeborene Nierenerkrankung, Nierenentzündung, durch toxische Substanzen induzierte Nephropathie, diabetische Nephropathie, Pyelonephritis, Nierenzysten und Nephrosklerose, welche diagnostisch beispielsweise durch abnorm verminderte Kreatinin- und/oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Verbindungen zur Behandlung und/oder Prävention von Folgeerscheinungen einer Niereninsuffizienz, wie beispielsweise Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Weitere Indikationsgebiete, für die die erfindungsgemäßen Verbindungen eingesetzt werden können, sind beispielsweise die Prävention und/oder Behandlung von Erkrankungen des Urogenitalbereiches, wie z.B. Reizblase, erektile Dysfunktion und weibliche sexuelle Dysfunktion, daneben aber auch die Prävention und/oder Behandlung von inflammatorischen Erkrankungen, wie z.B. entzündliche Dermatosen (Psoriasis, Akne, Ekzeme, Neurodermitis, Dermatitis, Keratitis, Narbenbildung, Warzenbildung, Frostbeulen), von Erkrankungen des Zentralen Nervensystems und neurodegenerativen Störungen (Schlaganfall, Alzheimer'sche Krankheit, Parkinson'sche Krankheit, Demenz, Epilepsie, Depressionen, Multiple Sklerose), von Schmerzzuständen, Krebserkrankungen (Hautkrebs, Liposarcome, Karzinome des Magen-Darm-Traktes, der Leber, Bauchspeicheldrüse, Lunge, Niere, Harnleiter, Prostata und des Genitaltraktes) sowie von Übelkeit und Erbrechen in Verbindung mit Krebstherapien.

Weitere Indikationsgebiete sind beispielsweise die Prävention und/oder Behandlung von Entzündungs- und Immunerkrankungen (Morbus Crohn, Colitis ulcerosa, Lupus erythematodes, rheumatoide Arthritis) und von Erkrankungen der Atemwege, wie beispielsweise chronischobstruktive Atemwegserkrankungen (chronische Bronchitis, COPD), Asthma, Lungenemphysem, Bronchiektasien, zystische Fibrose (Mukoviszidose) und pulmonale Hypertonie, insbesondere pulmonale arterielle Hypertonie.

Schließlich kommen die erfindungsgemäßen Verbindungen auch für die Prävention und/ oder Behandlung von Diabetes, insbesondere Diabetes mellitus, Gestationsdiabetes, Insulin-abhängiger Diabetes und Nicht-Insulin-abhängiger Diabetes, von diabetischen Folgeerkrankungen wie z.B. Retinopathie, Nephropathie und Neuropathie, von metabolischen Erkrankungen (Metabolisches Syndrom, Hyperglykämie, Gestationsdiabetes, Hyperinsulinämie, Insulinresistenz, Glukose-Intoleranz, Fettsucht (Adipositas)) sowie von Arteriosklerose und Dyslipidämien (Hypercholesterolämie, Hypertriglyceridämie, erhöhte Konzentrationen der postprandialen Plasma-Triglyceride, Hypoalphalipoproteinämie, kombinierte Hyperlipidämien), insbesondere von Diabetes, Metabolischem Syndrom und Dyslipidämien, in Betracht

Darüber hinaus können die erfindungsgemäßen Verbindungen auch zur Behandlung und/oder Prävention von Schilddrüsenerkrankungen (Hyperthyreose), Erkrankungen der Bauchspeicheldrüse (Pankreatitis), Leberfibrose, viralen Erkrankungen (HPV, HCMV, HIV), Kachexie, Osteoporose, Gicht, Inkontinenz sowie zur Wundheilung und Angiogenese eingesetzt werden.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen. Weiterer Gegenstand der vorliegenden Erfindung sind die erfindungsgemäßen Verbindungen zur Verwendung in einem Verfahren zur Behandlung und/oder Prophylaxe von koronarer Herzerkrankung, akutem Koronarsyndrom, Angina pectoris, Herzinsuffizienz, Myokardinfarkt und Vorhofflimmern.

Weiterer Gegenstand der vorliegenden Erfindung sind die erfindungsgemäßen Verbindungen zur Verfahren zur Behandlung und/oder Prophylaxe von Diabetes, Metabolischem Syndrom und Dyslipidämien.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen.

Als geeignete Kombinationswirkstoffe seien beispielhaft und vorzugsweise genannt: den Fettstoffwechsel verändernde Wirkstoffe, Antidiabetika, Blutdruck-Senker, durchblutungsfördernd und/ oder antithrombotisch wirkende Mittel, Antioxidantien, Chemokin-Rezeptor-Antagonisten, p38-Kinase-Inhibitoren, NPY-Agonisten, Orexin-Agonisten, Anorektika, PAF-AH-Inhibitoren, Antiphlogistika (COX-Inhibitoren, LTB₄-Rezeptor-Antagonisten), Analgetika wie beispielsweise Aspirin, Anti-Depressiva und andere Pyschopharmaka.

Gegenstand der vorliegenden Erfindung sind insbesondere Kombinationen mindestens einer der erfindungsgemäßen Verbindungen mit mindestens einem den Fettstoffwechsel verändernden Wirkstoff, einem Antidiabetikum, einem blutdrucksenkenden Wirkstoff und/oder einem antithrombotisch wirkenden Mittel.

Die erfindungsgemäßen Verbindungen können vorzugsweise mit einem oder mehreren
- den Fettstoffwechsel verändernden Wirkstoffen, beispielhaft und vorzugsweise aus der Gruppe der HMG-CoA-Reduktase-Inhibitoren, Inhibitoren der HMG-CoA-Reduktase-Expression, Squalensynthese-Inhibitoren, ACAT-Inhibitoren, LDL-Rezeptor-Induktoren, Cholesterin-Absorptionshemmer, polymeren Gallensäureadsorber, Gallensäure-Reabsorptionshemmer, MTP-Inhibitoren, Lipase-Inhibitoren, LpL-Aktivatoren, Fibrate, Niacin, CETP-Inhibitoren, PPAR-α-, PPAR-γ- und/oder PPAR-δ-Agonisten, RXR-Modulatoren, FXR-Modulatoren, LXR-Modulatoren, Thyroidhormone und/oder Thyroidmimetika, ATP-Citrat-Lyase-Inhibitoren, Lp(a)-Antagonisten, Cannabinoid-Rezeptor 1-Antagonisten, Leptin-Rezeptor-Agonisten, Bombesin-Rezeptor-Agonisten, Histamin-Rezeptor-Agonisten sowie der Antioxidantien/Radikalfänger;
- Antidiabetika, die in der Roten Liste 2004/II, Kapitel 12 genannt sind, sowie beispielhaft und vorzugsweise jenen aus der Gruppe der Sulphonylharnstoffe, Biguanide, Meglitinid-Derivate, Glukosidase-Inhibitoren, Inhibitoren der Dipeptidyl-Peptidase IV (DPP-IV-Inhibitoren), Oxadiazolidinone, Thiazolidindione, GLP 1-Rezeptor-Agonisten, Glukagon-Antagonisten, Insulin-Sensitizer, CCK 1-Rezeptor-Agonisten, Leptin-Rezeptor-Agonisten, Inhibitoren von Leberenzymen, die an der Stimulation der Glukoneogenese und/ oder Glykogenolyse beteiligt sind, Modulatoren der Glukoseaufnahme sowie der Kaliumkanalöffner, wie z.B. denjenigen, die in WO 97/26265 und WO 99/03861 offenbart sind;
- den Blutdruck senkenden Wirkstoffen, beispielhaft und vorzugsweise aus der Gruppe der Calcium-Antagonisten, Angiotensin AII-Antagonisten, ACE-Hemmer, Renin-Inhibitoren, beta-Rezeptoren-Blocker, alpha-Rezeptoren-Blocker, Aldosteron-Antagonisten, Mineralocorticoid-Rezeptor-Antagonisten, ECE-Inhibitoren, ACE/NEP Inhibitoren sowie der Vasopeptidase-Inhibitoren; und/oder
- antithrombotisch wirkenden Mitteln, beispielhaft und vorzugsweise aus der Gruppe der Thrombozytenaggregationshemmer oder der Antikoagulantien
- Diuretika;
- Vasopressin-Rezeptor-Antagonisten;
- organischen Nitraten und NO-Donatoren;
- positiv-inotrop wirksamen Verbindungen;
- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil und Tadalafil sowie PDE 3-Inhibitoren wie Milrinone;
- natriuretischen Peptiden, wie z.B. "atrial natriuretic peptide" (ANP, Anaritide), "B-type natriuretic peptide" oder "brain natriuretic peptide" (BNP, Nesiritide), "C-type natriuretic peptide" (CNP) sowie Urodilatin;
- Agonisten des Prostacyclin-Rezeptors (IP-Rezeptors), wie beispielsweise Iloprost, Beraprost, Cicaprost;
- Hemmer des I_{f} (funny channel) Kanals, wie beispielsweise Ivabradine;
- Calcium-Sensitizern, wie beispielhaft und vorzugsweise Levosimendan;
- Kalium-Supplements;
- NO-unabhängigen, jedoch Häm-abhängigen Stimulatoren der Guanylatcyclase, wie insbesondere den in WO 00/06568, WO 00/06569, WO 02/42301 und WO 03/095451 beschriebenen Verbindungen;
- NO- und Häm-unabhängigen Aktivatoren der Guanylatcyclase, wie insbesondere den in WO 01/19355, WO 01/19776, WO 01/19778, WO 01/19780, WO 02/070462 und WO 02/070510 beschriebenen Verbindungen;
- Inhibitoren der humanen neutrophilen Elastase (HNE), wie beispielsweise Sivelestat und DX-890 (Reltran);
- die Signaltransduktionskaskade inhibierenden Verbindungen, wie beispielsweise Tyrosinkinase-Inhibitoren, insbesondere Sorafenib, Imatinib, Gefitinib und Erlotinib; und/oder
- den Energiestoffwechsel des Herzens beeinflussenden Verbindungen, wie beispielweise Etomoxir, Dichloracetat, Ranolazine und Trimetazidine

### kombiniert werden.

Unter den Fettstoffwechsel verändernden Wirkstoffen werden vorzugsweise Verbindungen aus der Gruppe der HMG-CoA-Reduktase-Inhibitoren, Squalensynthese-Inhibitoren, ACAT-Inhibitoren, Cholesterin-Absorptionshemmer, MTP-Inhibitoren, Lipase-Inhibitoren, Thyroidhormone und/oder Thyroidmimetika, Niacin-Rezeptor-Agonisten, CETP-Inhibitoren, PPAR-α-Agonisten, PPAR-γ-Agonisten, PPAR-δ-Agonisten, polymeren Gallensäureadsorber, Gallensäure-Reabsorptionshemmer, Antioxidantlen/Radikalfänger sowie der Cannabinoid-Rezeptor 1-Antagonisten verstanden.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin, Cerivastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem Thyroidhormon und/oder Thyroidmimetikum, wie beispielhaft und vorzugsweise D-Thyroxin oder 3,5,3'-Triiodothyronin (T3), verabreicht.

Bei einer bevorzugten Ausführungsform der Erfindung werden die erfindungsgemäßen Verbindungen in Kombination mit einem Agonisten des Niacin-Rezeptors, wie beispielhaft und vorzugsweise Niacin, Acipimox, Acifran oder Radecol, verabreicht.

Bei einer bevorzugten Ausführungsform der Erfindung werden die erfindungsgemäßen Verbindungen in Kombination mit einem CETP-Inhibitor, wie beispielhaft und vorzugsweise Torcetrapib, JTT-705, BAY 60-5521, BAY 78-7499 oder CETP vaccine (Avant), verabreicht.

Bei einer bevorzugten Ausführungsform der Erfindung werden die erfindungsgemäßen Verbindungen in Kombination mit einem PPAR-γ-Agonisten beispielsweise aus der Klasse der Thiazolidindione, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-δ-Agonisten, wie beispielhaft und vorzugsweise GW-501516 oder BAY 68-5042, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Antioxidans/Radikalfänger, wie beispielhaft und vorzugsweise Probucol, AGI-1067, BO-653 oder AEOL-10150, verabreicht.

Bei einer bevorzugten Ausführungsform der Erfindung werden die erfindungsgemäßen Verbindungen in Kombination mit einem Cannabinoid-Rezeptor 1-Antagonisten, wie beispielhaft und vorzugsweise Rimonabant oder SR-147778, verabreicht.

Unter Antidiabetika werden vorzugsweise Insulin und Insulinderivate sowie oral wirksame hypoglykämische Wirkstoffe verstanden. Insulin und Insulinderivate umfasst hierbei sowohl Insuline tierischen, menschlichen oder biotechnologischen Ursprungs als auch Gemische hieraus. Die oral wirksamen hypoglykämischen Wirkstoffe umfassen vorzugsweise Sulphonylharnstoffe, Biguanide, Meglitinid-Derivate, Glukosidase-Inhibitoren und PPAR-gamma-Agonisten.

Bei einer bevorzugten Ausführungsform der Erfindung werden die erfindungsgemäßen Verbindungen in Kombination mit Insulin verabreicht.

Bei einer bevorzugten Ausführungsform der Erfindung werden die erfindungsgemäßen Verbindungen in Kombination mit einem Sulphonylharnstoff, wie beispielhaft und vorzugsweise Tolbutamid, Glibenclamid, Glimepirid, Glipizid oder Gliclazid, verabreicht.

Bei einer bevorzugten Ausführungsform der Erfindung werden die erfindungsgemäßen Verbindungen in Kombination mit einem Biguanid, wie beispielhaft und vorzugsweise Metformin, verabreicht.

Bei einer bevorzugten Ausführungsform der Erfindung werden die erfindungsgemäßen Verbindungen in Kombination mit einem Meglitinid-Derivat, wie beispielhaft und vorzugsweise Repaglinid oder Nateglinid, verabreicht.

Bei einer bevorzugten Ausführungsform der Erfindung werden die erfindungsgemäßen Verbindungen in Kombination mit einem Glukosidase-Inhibitor, wie beispielhaft und vorzugsweise Miglitol oder Acarbose, verabreicht.

Bei einer bevorzugten Ausführungsform der Erfindung werden die erfindungsgemäßen Verbindungen in Kombination mit einem DPP-IV-Inhibitor, wie beispielhaft und vorzugsweise Sitagliptin und Vildagliptin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, beta-Rezeptoren-Blocker, alpha-Rezeptoren-Blocker und Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Valsartan, Candesartan, Embusartan, Olmesartan oder Telmisartan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem alpha-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Bei einer bevorzugten Ausführungsform der Erfindung werden die erfindungsgemäßen Verbindungen in Kombination mit einem Aldosteron- oder Mineralokortikoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Vasopressin-Rezeptor-Antagonisten, wie beispielhaft und vorzugsweise Conivaptan, Tolvaptan, Lixivaptan oder SR-121463, verabreicht.

Bei einer bevorzugten Ausführungsform der Erfindung werden die erfindungsgemäßen Verbindungen in Kombination mit einem organischen Nitrat oder NO-Donator, wie beispielhaft und vorzugsweise Natriumnitroprussid, Nitroglycerin, Isosorbidmononitrat, Isosorbiddinitrat, Molsidomin oder SIN-1, oder in Kombination mit inhalativem NO verabreicht.

Bei einer bevorzugten Ausführungsform der Erfindung werden die erfindungsgemäßen Verbindungen in Kombination mit einer positiv-inotrop wirksamen Verbindung, wie beispielhaft und vorzugsweise Herzglycosiden (Digoxin), beta-adrenergen und dopaminergen Agonisten wie Isoproterenol, Adrenalin, Noradrenalin, Dopamin oder Dobutamin, verabreicht.

Bei einer bevorzugten Ausführungsform der Erfindung werden die erfindungsgemäßen Verbindungen in Kombination mit Antisympathotonika wie Reserpin, Clonidin oder alpha-Methyl-Dopa, mit Kaliumkanal-Agonisten wie Minoxidil, Diazoxid, Dihydralazin oder Hydralazin, oder mit Stickoxid freisetzenden Stoffen wie Glycerinnitrat oder Nitroprussidnatrium verabreicht.

Unter antithrombotisch wirkenden Mitteln werden vorzugsweise Verbindungen aus der Gruppe der Thrombozytenaggregationshemmer oder der Antikoagulantien verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Dabigatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Besonders bevorzugt im Rahmen der vorliegenden Erfindung sind Kombinationen enthaltend mindestens eine der erfindungsgemäßen Verbindungen sowie einen oder mehrere weitere Wirkstoffe ausgewählt aus der Gruppe bestehend aus HMG-CoA-Reduktase-Inhibitoren (Statine), Diuretika, beta-Rezeptoren-Blocker, organische Nitrate und NO-Donatoren, ACE-Inhibitoren, Angiotensin AII-Antagonisten, Aldosteron- und Mineralokortikoid-Rezeptor-Antagonisten, Vasopressin-Rezeptor-Antagonisten, Thrombozytenaggregationshemmer und Antikoagulantien, sowie deren Verwendung zur Behandlung und/oder Prävention der zuvor genannten Erkrankungen.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Verwendete Abkürzungen:

- aq.: wässrig
- br s: breites Singulett (bei NMR)
- Bsp.: Beispiel
- c: Konzentration
- d: Dublett (bei NMR)
- dd: Dublett von Dublett (bei NMR)
- DBU: 1,8-Diazabicyclo[5.4.0]undec-7-en
- DC: Dünnschichtchromatographie
- DCI: direkte chemische Ionisation (bei MS)
- DMF: *N,N*-Dimethylformamid
- DMSO: Dimethylsulfoxid
- d. Th.: der Theorie (bei Ausbeute)
- ee: Enantiomerenüberschuss
- EI: Elektronenstoß-Ionisation (bei MS)
- ESI: Elektrospray-Ionisation (bei MS)
- Et: Ethyl
- Fp.: Schmelzpunkt
- h: Stunde(n)
- HPLC: Hochdruck-, Hochleistungsflüssigchromatographie
- kat.: katalytisch
- konz.: konzentriert
- LC-MS: Flüssigchromatographie-gekoppelte Massenspektrometrie
- Lit.: Literatur(stelle)
- MeCN: Acetonitril
- min: Minute(n)
- MS: Massenspektrometrie
- NMR: Kernresonanzspektrometrie
- q: Quartett (bei NMR)
- rac.: racemisch
- RP-HPLC: reverse phase HPLC
- RT: Raumtemperatur
- Rₜ: Retentionszeit (bei HPLC)
- s: Singulett (bei NMR)
- t: Triplett (bei NMR)
- t-Bu: tert.-Butyl
- TFA: Trifluoressigsäure
- THF: Tetrahydrofuran
- verd.: verdünnt

### HPLC-, LC-MS- und GC-MS-Methoden:

Methode 1 (LC-MS): Gerätetyp MS: Micromass ZQ; Gerätetyp HPLC: Waters Alliance 2795; Säule: Phenomenex Synergi 2.5 µ MAX-RP 100A Mercury 20 mm x 4mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90% A → 0.1 min 90% A → 3.0 min 5% A → 4.0 min 5% A → 4.01 min 90% A; Fluss: 2 ml/min; Ofen: 50°C; UV-Detektion: 210 nm.
Methode 2 (LC-MS): Gerätetyp MS: Micromass ZQ; Gerätetyp HPLC: HP 1100 Series; UV DAD; Säule: Phenomenex Gemini 3µ 30 mm x 3.00 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90% A → 2.5 min 30% A → 3.0 min 5% A → 4.5 min 5% A; Fluss: 0.0 min 1 ml/min, 2.5 min/3.0 min/4.5 min. 2 ml/min; Ofen: 50°C; UV-Detektion: 210 nm.
Methode 3 (LC-MS): Instrument: Micromass QuattroPremier mit Waters UPLC Acquity ; Säule: Thermo Hypersil GOLD 1,9µ 50 x 1mm; Eluent A: 1 1 Wasser + 0.5ml 50%ige Ameisensäure , Eluent B: 1 1 Acetonitril + 0.5ml 50%ige Ameisensäure; Gradient: 0.0 min 90% A → 0.1 min 90% A → 1.5 min 10% A → 2.2 min 10% A Ofen: 50°C; Fluss: 0.33ml/min; UV-Detektion: 210 nm.
Methode 4 (LC-MS): Gerätetyp MS: Micromass ZQ; Gerätetyp HPLC: Waters Alliance 2795; Säule: Phenomenex Synergi 2µ Hydro-RP Mercury 20 mm x 4mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90% A → 2.5 min 30% A → 3.0 min 5% A → 4.5 min 5% A; Fluss: 0.0 min 1 ml/min, 2.5 min/3.0 min/4.5 min 2 ml/min; Ofen: 50°C; UV-Detektion: 210 nm.
Methode 5 (LC-MS): Gerätetyp MS: Waters (Micromass) Quattro Micro; Gerätetyp HPLC: Agilent 1100 Serie; Säule : Thermo Hypersil GOLD 3µ 20 x 4 mm; Eluent A: 1 1 Wasser + 0.5ml 50%ige Ameisensäure, Eluent B: 1 1 Acetonitril + 0.5ml 50%ige Ameisensäure; Gradient: 0.0 min 100% A → 3.0 min 10% A → 4.0 min 10% A → 4.01 min 100% A (Flow 2.5ml) → 5.00 min 100% A; Ofen: 50°C; Fluss: 2 ml/min; UV-Detektion: 210nm.
Methode 6 (LC-MS): Gerätetyp MS: Waters ZQ; Gerätetyp HPLC: Agilent 1100 Series; UV DAD; Säule: Thermo Hypersil GOLD 3µ 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 100% A → 3.0 min 10% A → 4.0 min 10% A → 4.1 min 100% B Fluss: 2.5 ml/min, Ofen: 55°C; UV-Detektion: 210 nm.

### Methode 7 (LC-MS):

Instrument: Waters ACQUITY SQD UPLC System; Säule: Waters Acquity UPLC HSS T3 1,8µ 50 x 1mm; Eluent A: 1 1 Wasser + 0.25 ml 99%ige Ameisensäure , Eluent B: 1 1 Acetonitril + 0.25 ml 99%ige Ameisensäure; Gradient: 0.0 min 90% A → 1.2 min 5% A → 2.0 min 5% A; Ofen: 50°C;
Fluss: 0.40 ml/min; UV-Detektion: 210 - 400 nm.

### Ausgangsverbindungen und Intermediate:

### Beispiel 1A

4-(Hydroxymethyl)-N-methylpyridin-2-carboxamid-Hydrochlorid-Monohydrat

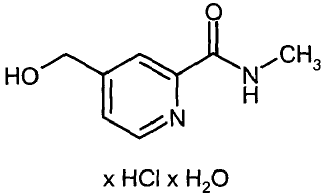

Die Darstellung erfolgte wie in US 6,689,883 für Intermediat H beschrieben.

### Beispiel 2A

4-(Chlormethyl)-*N*-methylpyridin-2-carboxamid-Hydrochlorid

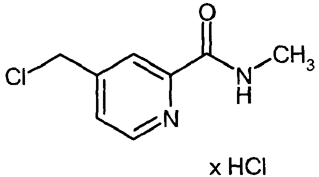

10 g (45.32 mmol) der Verbindung aus Beispiel 1A wurden in 160 ml Dichlormethan suspendiert und auf 0°C abgekühlt. Nach Zugabe von 16.18 g (135.96 mmol) Thionylchlorid wurde das Reaktionsgemisch auf RT erwärmt und über Nacht bei RT gerührt. Der Ansatz wurde danach eingedampft und der Rückstand im Hochvakuum getrocknet.
Ausbeute: 10 g (ca. 100% d. Th.)
LC-MS (Methode 3): Rₜ = 0.71 min; MS (ESIpos): m/z = 185 [M+H]⁺
¹H-NMR (400 MHz, DMSO-d₆): δ = 8.85-8.78 (m, 1H); 8.65 (d, 1H); 8.10 (s, 1H); 7.64 (d, 1H); 4.90 (s, 2H); 2.83 (d, 3H).

### Beispiel 3A

2-Amino-6-sulfanyl-4-(1,3-thiazol-4-yl)pyridin-3,5-dicarbonitril

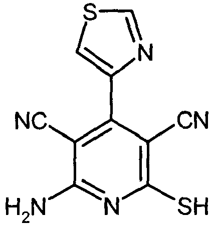

492 mg (4.349 mmol) 1,3-Thiazol-4-carbaldehyd, 871 mg (8.697 mmol) 2-Cyanethanthioamid und 880 mg (8.697 mmol) N-Methylmorpholin wurden in 10 ml Ethanol gelöst. Das Reaktionsgemisch wurde über Nacht bei Raumtemperatur gerührt, das Lösungsmittel wurde im Vakuum entfernt und der Rückstand mit Acetonitril versetzt. Man filtrierte den Feststoff ab, wusch mit Acetonitril und trocknete den Filterkuchen im Vakuum. Es wurden 181 mg (14% d. Th., 86% Reinheit) der Zielverbindung erhalten.

LC-MS (Methode 1): Rₜ = 0.76 min; MS (ESIpos): m/z = 260 [M+H]⁺.

### Beispiel 4A

2-Amino-6-sulfanyl-4-(1,3-thiazol-5-yl)pyridin-3,5-dicarbonitril

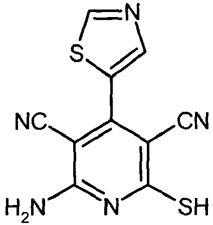

1.136 g (9.539 mmol) 1,3-Thiazol-5-carbaldehyd, 1.910 g (19.077 mmol) 2-Cyanethanthioamid und 1.930 g (19.077 mmol) N-Methylmorpholin wurden in 20 ml Ethanol gelöst. Das Reaktionsgemisch wurde 20h bei Raumtemperatur gerührt bevor man das Lösungsmittel im Vakuum entfernte. Die Reinigung des Rückstands erfolgte mittels Chromatographie an Kieselgel (Eluent: Dichlormethan/ Methanol 10:1). Die produktenthaltenden Fraktionen wurden gesammelt, das Lösungsmittel im Vakkum entfernt und der Rückstand mit Acetonitril verrührt. Nach Abfiltrieren und Trocknung des Feststoffs erhielt man 920 mg (37% d. Th.) der Zielverbindung.

LC-MS (Methode 2): Rₜ = 1.30 min; MS (ESIpos): m/z = 260 [M+H]⁺.

### Beispiel 5A

2-Amino-6-sulfanyl-4-thiophen-2-ylpyridin-3,5-dicarbonitril

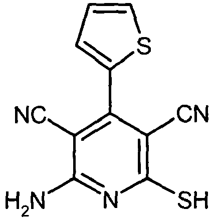

Die Herstellung erfolgte analog Beispiel 4A aus den entsprechenden Edukten.

LC-MS (Methode 1): Rₜ = 1.24 min; MS (ESIpos): m/z = 258 [M]⁺.

### Beispiel 6A

2-Amino-4-(1H-pyrazol-3-yl)-6-sulfanylpyridin-3,5-dicarbonitril

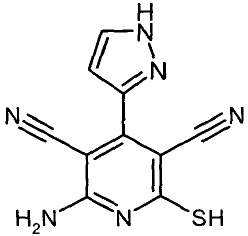

0.75 g (7.81 mmol) 1H-Pyrazol-3-carbaldehyd und 1.56 g (15.61 mmol) 2-Cyanothioacetamid wurden in 26 ml Ethanol mit 1.72 ml (15.61 mmol) N-Methylmorpholin versetzt und 4h unter Rückfluss erhitzt. Der entstandene Niederschlag wurde abgesaugt und mit etwas Ethanol gewaschen.
Ausbeute: 673 mg (36% d. Th.)
LC-MS (Methode 3): Rₜ = 0.44 min; MS (ESIpos): m/z = 243 [M+H]⁺.

Die in Tabelle 1 aufgeführten Beispiele wurden analog zu Beispiel 6A aus den entsprechenden Edukten hergestellt:

**Tabelle 1:**

| **Beispiel Nr.** | **Struktur (Ausbeute)** | **LC-MS: Rₜ [min] (Methode); MS (ESI): m/z [M+H]⁺** |
|---|---|---|
| **7A** | 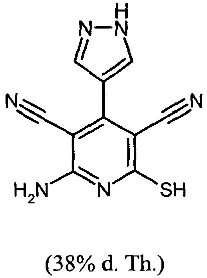 | 0.32 min (Methode 3); m/z = 243 |
| **8A** | 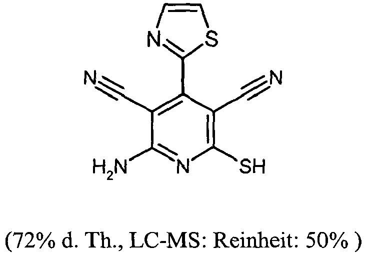 | 0.62 min (Methode 3); m/z = 260 |
| **9A** | 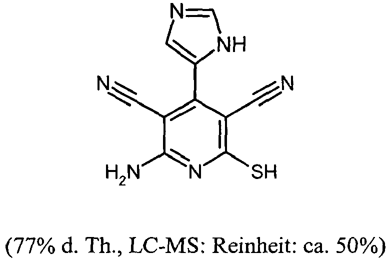 | 0.88 min (Methode 5); m/z = 243. |
| **10A** | 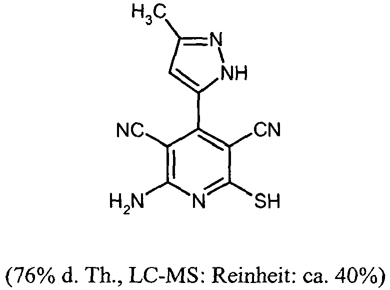 | 1.34 min (Methode 2); m/z = 257 |

### Beispiel 11A

2-Chlor-6-({[2-(4-chlorphenyl)-1,3-thiazol-4-yl]methyl}sulfanyl)-4-(1,3-thiazol-5-yl)pyridin-3,5-dicarbonitril

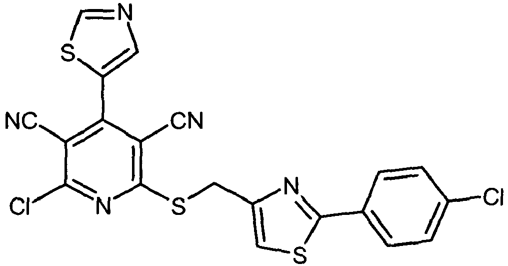

50 mg (0.107 mmol) 2-Amino-6-([2-(4-chlorphenyl)-1,3-thiazol-4-yl]methylsulfanyl)-4-(1,3-thiazol-5-yl)pyridin-3,5-dicarbonitril wurden bei 0 °C in 1.5 ml konzentrierte Salzsäure gelöst und mit 22 mg (0.321 mmol) Natriumnitrit versetzt. Es wurde zunächst eine Stunde bei 0 °C und dann über Nacht bei Raumtemperatur gerührt. Die Reinigung des Reaktionsgemisches erfolgte mittels präparativer HPLC (Acetonitril/ Wasser: 10:90 → 95:5, Zusatz von 0.1% TFA). Man erhielt 9 mg (17 % d. Th.) der Zielverbindung.

¹H-NMR (400 MHz, DMSO-d₆): δ = 9.49 (s, 1H), 8.42 (s, 1H), 7.95 (d, 2H), 7.75 (s, 1H), 7.57 (d, 2H), 4.78 (s, 2H).

LC-MS (Methode 2): Rₜ = 2.99 min; MS (ESIpos): m/z = 486 [M+H]⁺.

### Beispiel 12A

2-Chlor-6-({[2-(4-chlorphenyl)-1,3-thiazol-4-yl]methyl}sulfanyl)-4-(1H-pyrazol-3-yl)pyridin-3,5-dicarbonitril

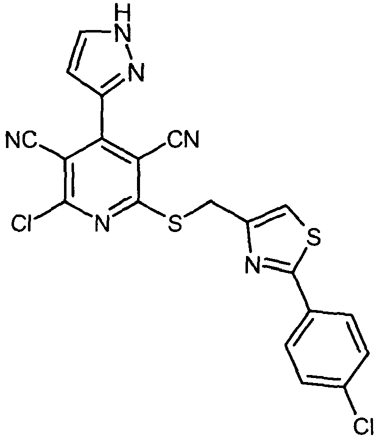

1.7 g (2.42 mmol) der Verbindung aus Beispiel 5, 567 mg (4.84 mmol) Isopentylnitrit und 650 mg (4.84 mmol) Kupfer(II)-chlorid wurden in 24 ml Acetonitril vorgelegt und das Gemisch wurde 3h bei 65°C gerührt. Anschließend wurden 567 mg (4.84 mmol) Isopentylnitrit und 650 mg (4.84 mmol) Kupfer(II)-chlorid hinzugegeben und die Reaktionsmischung wurde nochmals 9h bei 65°C gerührt. Nach Abkühlen auf RT wurden 4.8 ml 1N Salzsäure zugegeben. Die wässrige Phase mit dreimal mit Essigsäureethylester extrahiert. Die vereinigten organischen Phasen wurden mit gesättigter wässriger Natriumchlorid-Lösung gewaschen und über Natriumsulfat getrocknet. Nach Entfernen des Lösungsmittels wurde das Produkt über präparative HPLC (Acetonitril/Wasser, Zusatz von 0.1% TFA) gereinigt.
Ausbeute: 370 mg (33% d. Th.)
LC-MS (Methode 3): Rₜ = 1.49 min; MS (ESIpos): m/z = 469 [M+H]⁺.

Die in Tabelle 2 aufgeführten Beispiele wurden analog zu Beispiel 12A aus den entsprechenden Edukten hergestellt:

**Tabelle 2:**

| **Beispiel Nr.** | **Struktur (Ausbeute)** | **LC-MS: Rt [min] (Methode); MS (ESI): m/z [M+H]+** |
|---|---|---|
| **13A** | 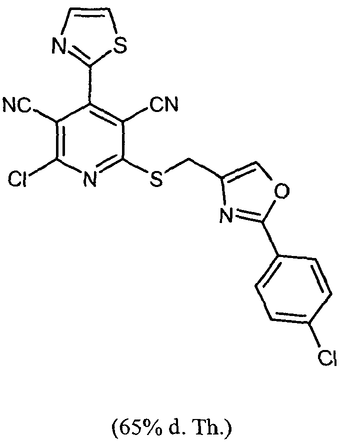 | 2.48 min (Methode 1); m/z= 470 |
| **14A** | 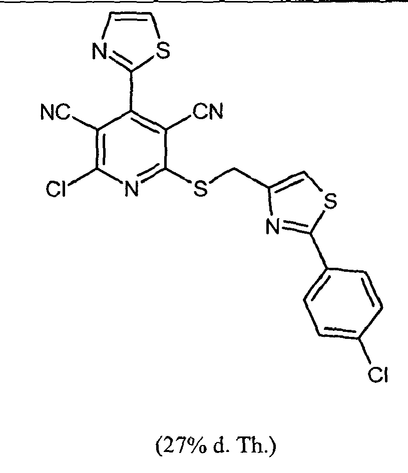 | 1.58 min (Methode 3); m/z = 486 |
| **15A** | 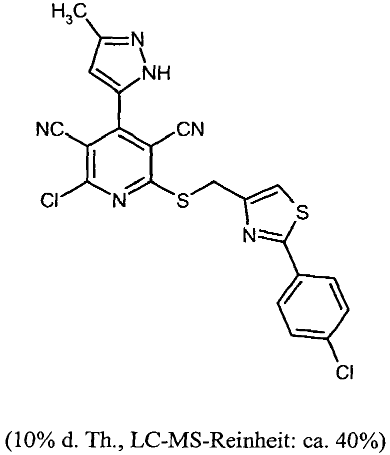 | 3.03 min (Methode 2); m/z = 483 |
| **16A** | 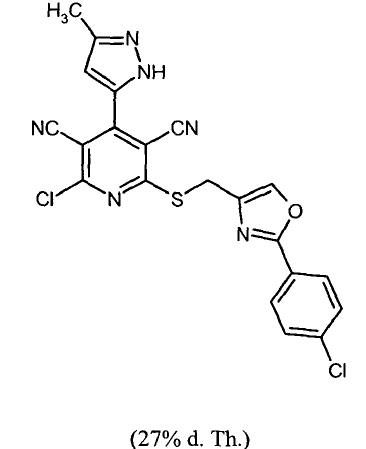 | 2.89 min (Methode 2); m/z = 467 |
| **17A** | 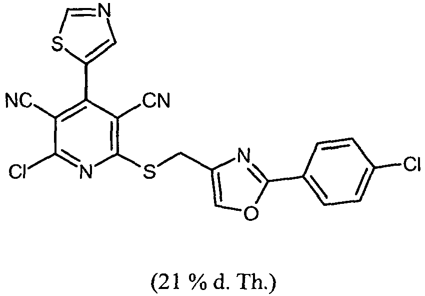 | 2.86 min (Methode 2); m/z = 470 |
| **18A** | 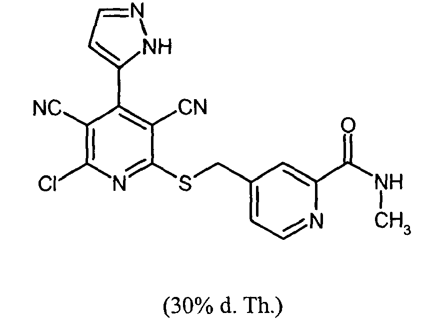 | 1.00 min (Methode 3); m/z = 410 |

### Beispiel 19A

Methyl-{4-[2-amino-6-({[2-(4-chlorphenyl)-1,3-thiazol-4-yl]methyl}sulfanyl)-3,5-dicyanopyridin-4-yl]-1H-pyrazol-1-yl}acetat

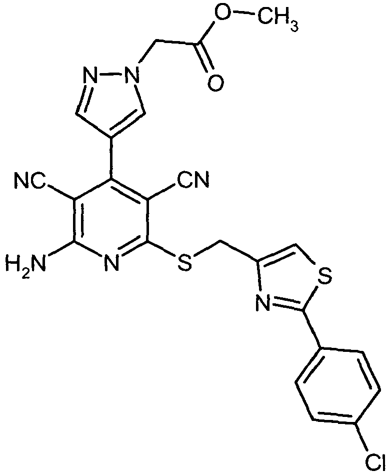

250 mg (0.461 mmol) der Verbindung aus Beispiel 6 wurden in 5.1 ml DMF gelöst, mit 301 mg (0.922 mmol) Cäsiumcarbonat und 71 mg (0.461 mmol) Bromessigsäuremethylester versetzt und über Nacht bei RT gerührt. Der Ansatz wurde mit soviel Wasser/THF versetzt, dass eine klare Lösung entsteht und direkt über präparative HPLC (Acetonitril/Wasser 10:90 → 95:5, Zusatz von 0.1% TFA) gereinigt.
Ausbeute: 122 mg (49% d.Th.)
¹H-NMR (400 MHz, DMSO-d₆): δ = 8.38 (s, 1H), 8.10 (br s, 2H), 7.98-7.7.92 (m, 3H), 7.89 (s, 1H), 7.57 (d, 2H), 5.23 (s, 2H), 4.62 (s, 2H), 3.70 (s, 3H).
LC-MS (Methode 2): Rₜ = 2.62 min; MS (ESIpos): m/z = 522 [M+H]⁺.

### Beispiel 20A

2-Amino-4-(2-brom-1,3-thiazol-4-yl)-6-sulfanylpyridin-3,5-dicarbonitril

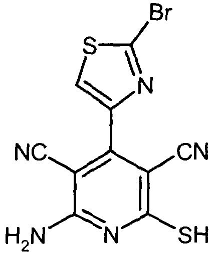

2 g (10.415 mmol) 2-Brom-1,3-thiazol-4-carbaldehyd wurden gemeinsam mit 2.086 g (20.829 mmol) 2-Cyanethanthioamid und 2.29 ml (20.829 mmol) 4-Methylmorpholin 3 h bei 90°C in 20 ml Ethanol refluxiert. Anschließend wurde das Reaktionsgemisch eingeegt. Man erhielt 4.8 g der Zielverbindung in 73%iger Reinheit (Ausbeute: 99% d. Th.).

LC-MS (Methode 3): Rₜ = 0.83 min; MS (ESIpos): m/z = 338 [M+H]⁺.

### Beispiel 21A

2-Amino-4-(2-brom-1,3-thiazol-4-yl)-6-({[2-(4-chlorphenyl)-1,3-thiazol-4-yl]methyl}sulfanyl)pyridin-3,5-dicarbonitril

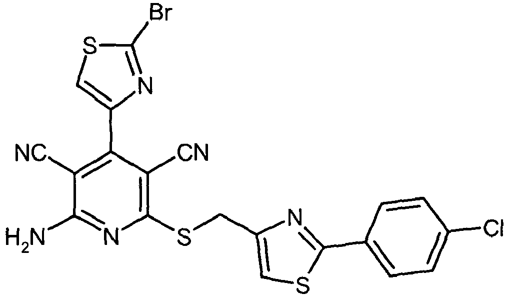

4.78 g (Reinheit 77%, 10.882 mmol) 2-Amino-4-(2-brom-1,3-thiazol-4-yl)-6-sulfanylpyridin-3,5-dicarbonitril wurden in 30 ml DMF vorgelegt, mit 2.923 g (11.971 mmol) 4-(Chlormethyl)-2-(4-chlorphenyl)-1,3-thiazol und 2.742 g (32.647 mmol) Natriumhydrogencarbonat versetzt und über Nacht bei Raumtemperatur gerührt. Das Reaktionsgemisch wurde auf Wasser gegeben, der ausgefallene Feststoff abgesaugt, mit Wasser gewaschen und im Hochvakuum getrocknet. Das verunreinigte Produkt wurde auf Kieselgel aufgezogen und per Chromatographie an Kieselgel (Eluent: Cyclohexan/ Essigsäureethylester 2:1) gereinigt. Die produktenthaltenden Fraktionen wurden eingeengt, der Rückstand mit Dichlormethan ausgerührt, der Feststoff abfiltriert, mit Dichlormethan gewaschen und im Hochvakuum getrocknet. Man erhielt 1.1g der Zielverbindung in 91%iger Reinheit (Ausbeute: 17 % d. Th.).

LC-MS (Methode 3): Rₜ = 1.49 min; MS (ESIpos): m/z = 545 [M+H]⁺.

### Beispiel 22A

2-Amino-4-[2-(3-{[tert.-butyl(dimethyl)silyl]oxy}prop-1-in-1-yl)-1,3-thiazol-4-yl]-6-({2-(4-chlorphenyl)-1,3-thiazol-4-yl]methyl}sulfanyl)pyridin-3,5-dicarbonitril

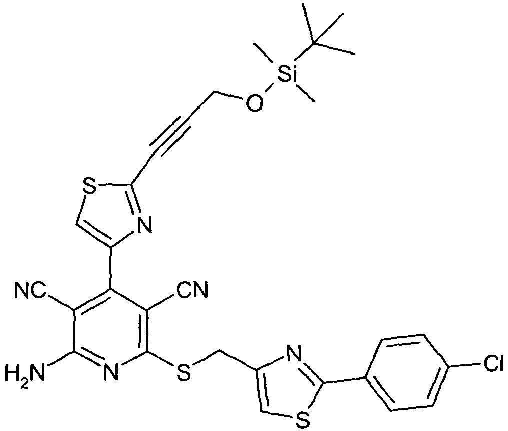

500 mg (0.916 mmol) 2-Amino-4-(2-brom-1,3-thiazol-4-yl)-6-({[2-(4-chlorphenyl)-1,3-thiazol-4-yl]methyl}sulfanyl)pyridin-3,5-dicarbonitril und 0.186 ml (0.916 mmol) tert.-Butyl(dimethyl)(prop-2-in-1-yloxy)silan wurden unter Argon in 10 ml Acetonitril vorgelegt und nacheinander mit 105 mg (0.092 mmol) Tetrakis(triphenylphosphin)palladium(0), 35 mg (0.183 mmol) Kupfer(I)iodid und 0.255 ml (1.832 mmol) Triethylamin versetzt. Anschließend wurde 4 h bei 100°C refluxiert. Das Reaktionsgemsich wurde filtriert und das Filtrat mittels präparativer HPLC gereinigt. Man erhielt 239 mg der Zielverbindung in 38%iger Reinheit (Ausbeute: 16%).

Die auf diesem Weg erhaltene Charge enthielt zu 62% die desilylierte Verbindung. Das Gemisch wurde ohne weitere Reinigung in die nachfolgende Hydrierung eingesetzt.

LC-MS (Methode 7): Rₜ = 1.56 min; MS (ESIpos): m/z = 635 [M+H]⁺.

### Beispiel 23A

2-Amino-6-({[2-(4-chlorphenyl)-1,3-thiazol-4-yl]methyl}sulfanyl)-4-(2-iod-1,3-thiazol-5-yl)pyridin-3,5-dicarbonitril

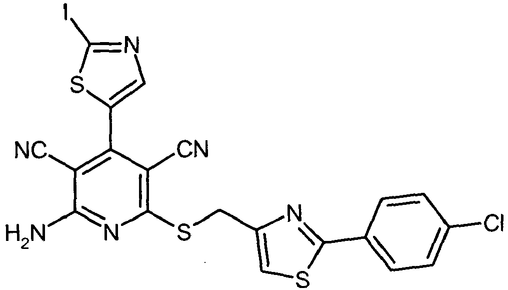

Unter Argon wurden 230 mg (0.493 mmol) 2-Amino-6-({[2-(4-chlorphenyl)-1,3-thiazol-4-yl]methyl}sulfanyl)-4-(1,3-thiazol-5-yl)pyridin-3,5-dicarbonitril in 15 ml THF gelöst und auf -78°C gekühlt. Bei dieser Temperatur wurden 1.182 ml (1.182 mmol) Lithium-1,1,1,3,3,3-hexamethyldisilazan-2-id (1 M in THF) zugetropft. Nachdem 1 h bei -78°C gerührt wurde, wurde eine Lösung aus 0.143 ml (1.084 mmol) 1,2-Diiodethan in 5 ml THF zugetropft und das Reaktionsgemisch eine weitere Stunde bei -78°C gerührt. Die Reaktion wurde durch Zugabe von 20 ml 10%iger wässriger Natriumthiosulfatlösung abgebrochen. Nachdem das Gemisch sich auf Raumtemperatur erwärmt hatte, wurde die wässrige Phase dreimal mit Dichlormethan extrahiert. Die vereinten organischen Phasen wurden über Natriumsulfat getrocknet, filtriert und eingeengt. Der Rückstand wurde mittels präparativer HPLC (Eluent A=Wasser, B=Acetonitril; Gradient: 0.0 min 10% B, 30 min 95% B, 34 min 95% B, 34.01 min 10% B, 38 min 10% B; Fluss: 50 ml/min; +0.1% TFA) gereinigt. Der aus dem Acetonitril/Wasser-Gemisch ausgefallene Feststoff wurde abfiltriert und im Hochvakuum getrocknet. Man erhielt 60 mg der Zielverbindung in 76%iger Reinheit (Ausbeute: 15% d. Th.).

LC-MS (Methode 7): Rₜ = 1.31 min; MS (ESIpos): m/z = 593 [M+H]⁺.

Das in Tabelle 6 aufgeführte Beispiel wurde analog zu Beispiel 12A aus den entsprechenden Edukten hergestellt: Tabelle 6: **Bei- Struktur LC-MS: spiel (Ausbeute) Rt [min] (Methode); MS Nr. (ESI): m/z [M+H]+ 24A** 2.85 min (Methode 2); m/z =

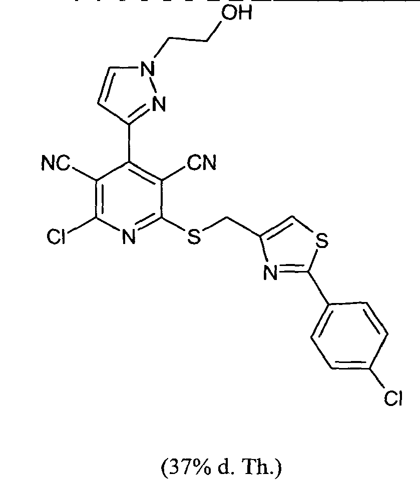

### Ausführungsbeispiele:

### Beispiel 1

2-Amino-6-([2-(4-chlorphenyl)-1,3-thiazol-4-yl]methylsulfanyl)-4-(1,3-thiazol-4-yl)pyridin-3,5-dicarbonitril

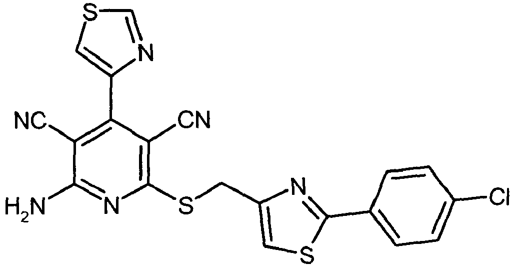

90 mg (ca. 0.298 mmol) 2-Amino-6-sulfanyl-4-(1,3-thiazol-4-yl)pyridin-3,5-dicarbonitril wurden gemeinsam mit 93 mg (0.382 mmol) 4-(Chlormethyl)-2-(4-chlorphenyl)-1,3-thiazol und 88 mg (1.041 mmol) Natriumhydrogencarbonat in 20 ml DMF 2h bei Raumtemperatur gerührt. Die Reaktionsmischung wurde zwei mal mittels präparativer HPLC (Acetonitril/ Wasser: 10:90 → 95:5, Zusatz von 0.1% TFA) gereinigt. Es wurden 36 mg (22% d. Th.) der Zielverbindung erhalten.

¹H-NMR (400 MHz, DMSO-d₆): δ = 9.33 (d, 1H), 8.38 (d, 1H), 8.22 (br s, 2H), 7.98 (s, 1H), 7,93 (d, 2H), 7.57 (d, 2H), 4.65 (s, 2H).

LC-MS (Methode 3): Rₜ = 1.35 min; MS (ESIpos): m/z = 467 [M+H]⁺.

### Beispiel 2

2-Amino-6-([2-(4-chlorphenyl)-1,3-oxazol-4-yl]methylsulfanyl)-4-(1,3-thiazol-4-yl)pyridin-3,5-dicarbonitril 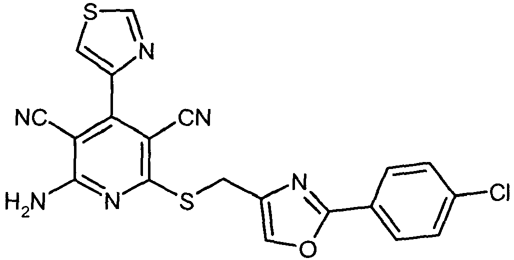 90 mg (ca. 0.298 mmol) 2-Amino-6-sulfanyl-4-(1,3-thiazol-4-yl)pyridin-3,5-dicarbonitril wurden gemeinsam mit 87 mg (0.382 mmol) 4-(Chlormethyl)-2-(4-chlorphenyl)-1,3-Oxazol und 88 mg (1.041 mmol) Natriumhydrogencarbonat in 20 ml DMF über Nacht bei Raumtemperatur gerührt. Die Reaktionsmischung wurde mittels präparativer HPLC (Acetonitril/ Wasser: 10:90 → 95:5, Zusatz von 0.1 % TFA) gereinigt. Es wurden 37 mg (24% d. Th.) der Zielverbindung erhalten.

¹H-NMR (400 MHz, DMSO-d₆): δ = 9.33 (d, 1H), 8.37 (d, 1H), 8.36 (s, 1H), 8.13 (br s, 2H), 7.97 (d, 2H), 7.6 (d, 2H), 4.43 (s, 2H).

LC-MS (Methode 3): Rₜ = 1.29 min; MS (ESIpos): m/z = 451 [M+H]⁺.

### Beispiel 3

2-Amino-6-([2-(4-chlorphenyl)-1,3-thiazol-4-yl]methylsulfanyl)-4-(1,3-thiazol-5-yl)pyridin-3,5-dicarbonitril

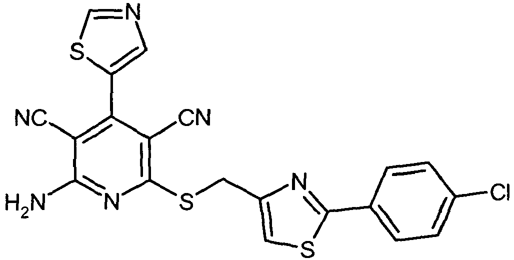

765 mg (2.950 mmol) 2-Amino-6-sulfanyl-4-(1,3-thiazol-5-yl)pyridin-3,5-dicarbonitril wurden gemeinsam mit 792 mg (3.245 mmol) 4-(Chlormethyl)-2-(4-chlorphenyl)-1,3-thiazol und 744 mg (8.850 mmol) Natriumhydrogencarbonat in 15 ml DMF 1h bei Raumtemperatur gerührt. Das Reaktionsgemisch wurde auf 150 ml Acetonitril gegeben und dann mit 100 ml Wasser versetzt. Der ausgefallene Niederschlag wurde abfiltriert, zunächst mit wenig Acetonitril und schließlich mit Diethylether gewaschen. Nach Trocknung im Vakuum erhielt man 889 mg (53% d. Th.) der Zielverbindung.

¹H-NMR (400 MHz, DMSO-d₆): δ = 9.38 (s, 1H), 8.27 (s, 1H), 8.13 (br s, 2H), 7.95 (d, 2H), 7.89 (s, 1H), 7.57 (d, 2H), 4.62 (s, 2H).

LC-MS (Methode 3): Rₜ = 1.35 min; MS (ESIpos): m/z = 467 [M+H]⁺.

### Beispiel 4

2-Amino-6-([2-(4-chlorphenyl)-1,3-oxazol-4-yl]methylsulfanyl)-4-(1,3-thiazol-5-yl)pyridin-3,5-dicarbonitril

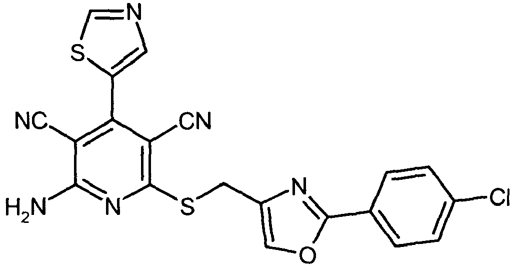

250 mg (0.964 mmol) 2-Amino-6-sulfanyl-4-(1,3-thiazol-5-yl)pyridin-3,5-dicarbonitril wurden gemeinsam mit 242 mg (1.060 mmol) 4-(Chlormethyl)-2-(4-chlorphenyl)-1,3-oxazol und 243 mg (2.892 mmol) Natriumhydrogencarbonat in 5 ml DMF 1h bei Raumtemperatur gerührt. Das Reaktionsgemisch wurde auf Wasser gegeben, der ausgefallene Niederschlag wurde abfiltriert und mit Diethylether gewaschen. Nach Trocknung im Vakuum erhielt man 53 mg (12% d. Th.) der Zielverbindung.

¹H-NMR (400 MHz, DMSO-d₆): δ = 9.38 (s, 1H), 8.35 (s, 1H), 8.28 (s, 1H), 8.26 (br s, 2H), 7.96 (d, 2H), 7.59 (d, 2H), 4.42 (s, 2H).

LC-MS (Methode 2): Rₜ = 2.55 min; MS (ESIpos): m/z = 451 [M+H]⁺.

### Beispiel 5

2-Amino-6-({[2-(4-chlorphenyl)-1,3-thiazol-4-yl]methyl}sulfanyl)-4-(1H-pyrazol-3-yl)pyridine-3,5-dicarbonitril

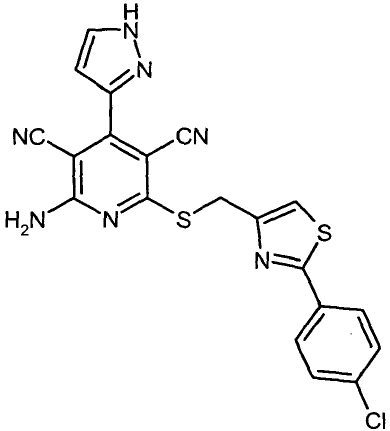

50 mg (0.21 mmol) der Verbindung aus Beispiel 6A und 156 mg (0.64 mmol) 4-(Chlormethyl)-2-(4-chlorphenyl)-1,3-thiazol wurden in 1 ml Dimethylformamid gelöst, mit 52 mg (0.62 mmol) Natriumhydrogencarbonat versetzt und 2.5h bei RT gerührt. Der Ansatz wurde mit ca. 10 ml Wasser versetzt und der entstandene Feststoff wurde über präparative HPLC (Acetonitril/ Wasser 10:90 → 95:5, Zusatz von 0.1% TFA) gereinigt.
Ausbeute: 54 mg (58% d.Th.)
¹H-NMR (400 MHz, DMSO-d₆): δ = 13.59 (br s, 1H), 8.45-7.80 (m, 6H), 7.57 (d, 2H), 6.79 (t, 1H), 4.63 (s, 2H).

LC-MS (Methode 1): Rₜ = 2.08 min; MS (ESIpos): m/z = 450 [M+H]⁺.

Die in Tabelle 3 aufgeführten Beispiele wurden aus den entsprechenden Ausgangsverbindungen analog zu Beispiel 5 mit anschließender Aufreinigung [präparative HPLC (Chromasil, Wasser/Acetonitril + 0.15% konz. Salzsäure)] hergestellt:

**Tabelle 3:**

| **Beispiel Nr.** | **Struktur (Ausbeute)** | **LC-MS: Rₜ [min] (Methode) ; MS (ESI): m/z** [**M**+**H**]⁺ | **¹H-NMR (DMSO-d₆):** |
|---|---|---|---|
| **6** | 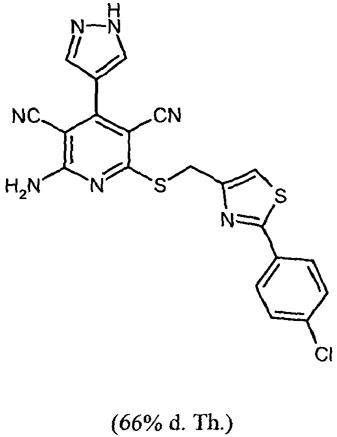 | 2.53 min (Methode 2); m/z = 450 | δ (400 MHz) = 13.4 (br s, 1H), 8.11 (br s, 4H), 7.93 (d, 2H), 7.89 (s, 1H), 7.57 (d, 2H), 4.62 (s, 2H). |
| **7** | 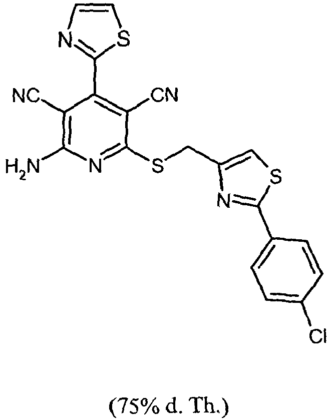 | 1.38 min (Methode 3); m/z = 467 | δ (400 MHz) = 8.40 (br s, 2H), 8.19 (d, 1H), 8.17 (d, 1H), 7.95 (s, 1H), 7.92 (d, 2H), 7.58 (d, 2H), 4.67 (s, 2H). |
| **8** | 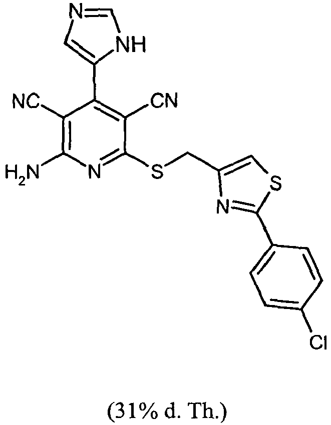 | 1.19min (Methode 3); m/z = 450 | δ (400 MHz) = 8.24 (br s, 1H), 8.09 (br s, 2H), 7.97-7.87 (m, 4H), 7.58 (d, 2H), 4.62 (s, 2H). |
| **9** | 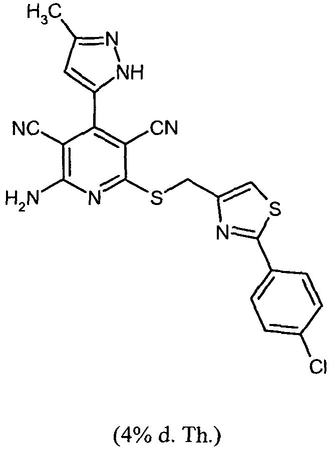 | 1.31 min (Methode 3); m/z = 467 | Ö (400 MHz) = 13.29 (br s, 1H), 8.02 (br s, 2H), 7.94 (d, 2H), 7.89 (s, 1H), 7.58 (d, 2H), 6.51 (s, 1H), 4.62 (s, 2H), 2.32 (s, 3H). |
| **10** | 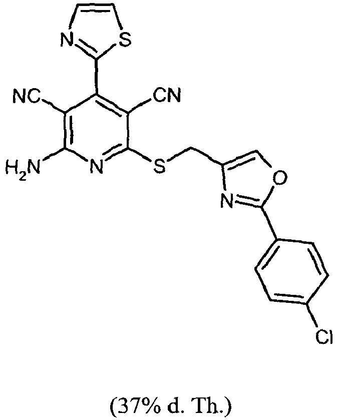 | 2.63 min (Methode 2); m/z = 451 | δ (400 MHz) = 8.37 (s, 1H), 8.25 (br s, 2H), 8.19 (d, 1H), 8.17 (d, 1H), 7.98 (d, 2H), 7.60 (d, 2H), 4.43 (s, 2H). |
| **11** | 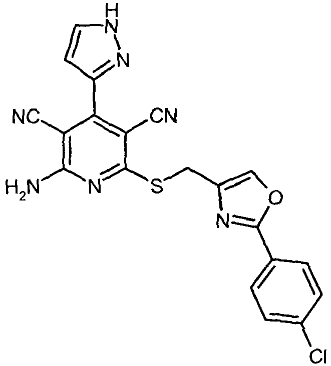 | 1.22 min (Methode 3); m/z = 434 | δ (400 MHz) = 13.59 (br s, 1H), 8.36 (s, 1H), 8.09 (br s, 2H), 7.98-7.95 (m, 3H), 7.60 (d, 2H), 6.78 (br s, 1H), 4.41(s, 2H). |
| **12** | 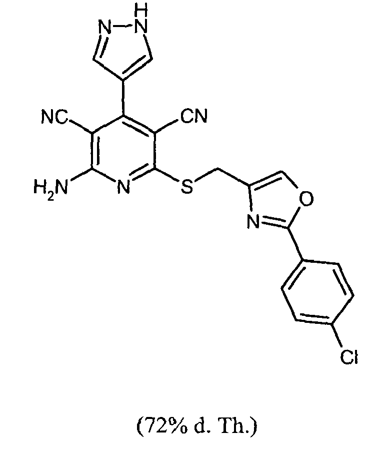 | 1.19 min (Methode 3); m/z = 434 | δ (400 MHz) = 13.50 (br s, 1H), 8.38-8.23 (m, 2H), 8.08 (br s, 2H), 7.98-7.89 (m, 2H), 7.60 (d, 2H), 4.41 (s, 2H). |
| **13** | 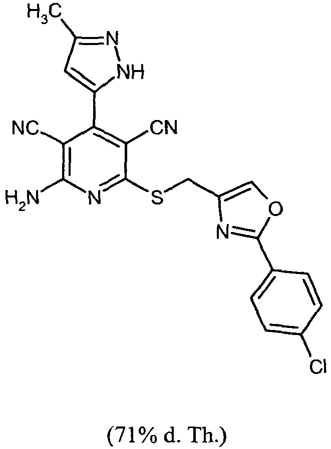 | 1.25 min (Methode 3); m/z = 448 | δ (400 MHz) = 8.34 (s, 1H), 8.10 (br s, 2H), 7.98 (d, 2H), 7.60 (d, 2H), 6.51 (s, 1H), 4.41 (s, 2H), 2.31 (s, 3H). |
| **15** | 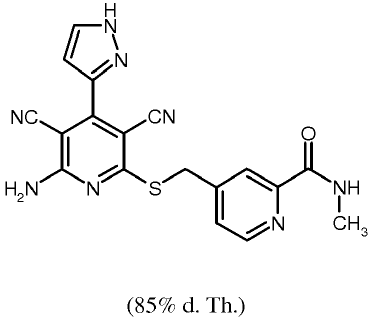 | 1.29 min (Methode 1); m/z = 391 | δ (400 MHz) = 13.60 (br s, 1H), 8.76 (q, 1H), 8.54 (d, 1H), 8.12 (s, 1H), 8.05 (br s, 2H), 7.97 (d, 1H), 7.78 (dd, 1H), 6.78 (d, 1H), 4.60 (s, 2H), 2.81 (d, 3H). |

### Beispiel 16

2-([2-(4-Chlorphenyl)-1,3-thiazol-4-yl]methylsulfanyl)-6-(3-hydroxyazetidin-1-yl)-4-(1,3-thiazol-5-yl)pyridin-3,5-dicarbonitril

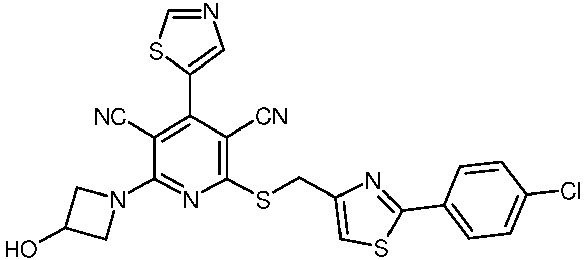

100 mg (0.206 mmol) 2-Chlor-6-({[2-(4-chlorphenyl)-1,3-thiazol-4-yl]methyl}sulfanyl)-4-(1,3-thiazol-5-yl)pyridin-3,5-dicarbonitril wurden gemeinsam mit 45 mg (0.411 mmol) Azetidin-3-olhydrochlorid und 53 mg (0.411 mmol) N-Ethyl-N-(1-methylethyl)propan-2-amin in 1.5 ml THF gelöst. Nach zweistündigem Rühren des Gemischs bei Raumtemperatur erfolgt eine Reinigung mittels präparativer HPLC (Acetonitril/ Wasser: 10:90 → 95:5, Zusatz von 0.1% TFA). Es wurden 40 mg (37% d. Th.) der Zielverbindung erhalten.

¹H-NMR (400 MHz, DMSO-d₆): δ = 9.40 (d, 1H), 8.28 (d, 1H), 7.95 (d, 2H), 7.68 (s, 1H), 7.57 (d, 2H), 5.9 (d, 1H), 4.80-4.67 (m, 3H), 4.62-4.55 (m, 2H), 4.17-4.11 (m, 2H).

LC-MS (Methode 2): Rₜ = 2.66 min; MS (ESIpos): m/z = 523 [M+H]⁺.

### Beispiel 17

2-([2-(4-Chlorphenyl)-1,3-thiazol-4-yl]methylsulfanyl)-6-[(2R)-2,3-dihydroxypropyl]amino-4-(1,3-thiazol-5-yl)pyridin-3,5-dicarbonitril

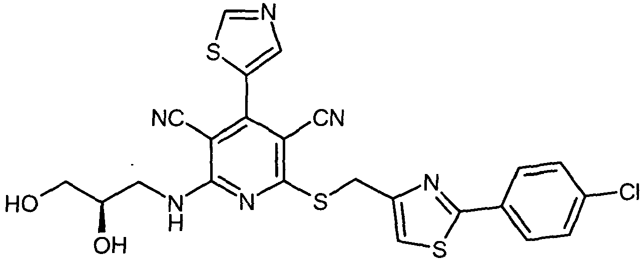

100 mg (0.206 mmol) 2-Chlor-6-({[2-(4-chlorphenyl)-1,3-thiazol-4-yl]methyl}sulfanyl)-4-(1,3-thiazol-5-yl)pyridin-3,5-dicarbonitril wurden gemeinsam mit 38 mg (0.411 mmol) (2R)-3-Aminopropan-1,2-diol in 1.5 ml THF gelöst. Nach zweistündigem Rühren des Gemischs bei Raumtemperatur erfolgt eine Reinigung mittels präparativer HPLC (Acetonitril/ Wasser: 10:90 → 95:5, Zusatz von 0.1 % TFA). Es wurden 64 mg (58 % d. Th.) der Zielverbindung erhalten.

¹H-NMR (500 MHz, DMSO-d₆): δ = 9.41 (d, 1H), 8.31 (d, 1H), 8.10 (t, 1H), 7.96 (d, 2H), 7.73 (s, 1H), 7.57 (d, 2H), 4.92 (d, 1H), 4.78-4.68 (m, 3H), 3.81-3.71 (m, 2H), 3.58-3.48 (m, 1H), 3.44-3.40 (m, 2H).

LC-MS (Methode 2): Rₜ = 2.42 min; MS (ESIpos): m/z = 541 [M+H]⁺.

### Beispiel 18

2-([2-(4-Chlorphenyl)-1,3-oxazol-4-yl]methylsulfanyl)-6-(3-hydroxyazetidin-1-yl)-4-(1,3-thiazol-5-yl)pyridin-3,5-dicarbonitril

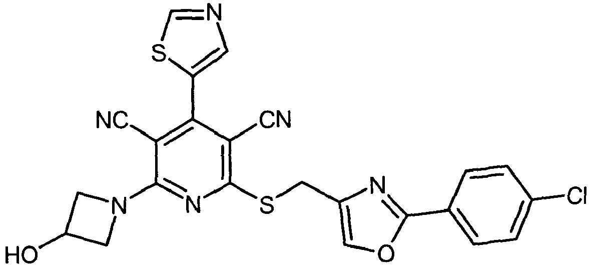

80 mg (0.170 mmol) 2-Chlor-6-({[2-(4-chlorphenyl)-1,3-oxazol-4-yl]methyl}sulfanyl) -4-(1,3-thiazol-5-yl)pyridin-3,5-dicarbonitril wurden gemeinsam mit 37 mg (0.340 mmol) Azetidin-3-olhydrochlorid und 44 mg (0.340 mmol) N-Ethyl-N-(1-methylethyl)propan-2-amin in 1 ml THF gelöst. Nach zweistündigem Rühren des Gemischs bei Raumtemperatur erfolgt eine Reinigung mittels präparativer (Acetonitril/ Wasser: 10:90 → 95:5, Zusatz von 0.1% TFA). Es wurden 40 mg (46 % d. Th.) der Zielverbindung erhalten.

¹H-NMR (400 MHz, DMSO-d₆): δ = 9.40 (s, 1H), 8.27 (s, 1H), 8.16 (s, 1H), 7.97 (d, 2H), 7.61 (d, 2H), 5.92 (br s, 1H), 4.68-4.60 (m, 3H), 4.48 (s, 2H), 4.18 (m, 2H).

LC-MS (Methode 2): Rₜ = 2.54 min; MS (ESIpos): m/z = 507 [M+H]⁺.

### Beispiel 19

2-({[2-(4-Chlorophenyl)-1,3-oxazol-4-yl]methyl}sulfanyl)-6-[(2-hydroxyethyl)amino]-4-(1,3-thiazol-2-yl)pyridin-3,5-dicarbonitril

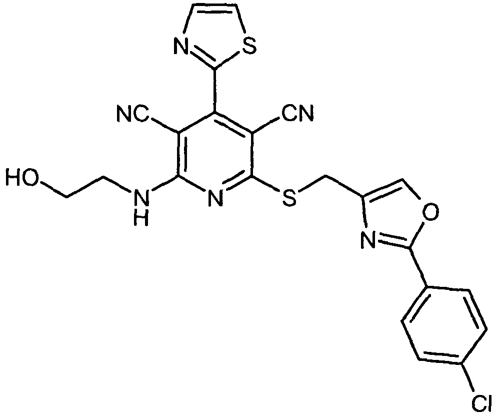

50 mg (0.11 mmol) der Verbindung aus Beispiel 13A wurden in 1.5 ml DMF gelöst und mit 13 mg (0.213 mmol) 2-Aminoethanol versetzt und über Nacht bei RT gerührt. Der Ansatz über präparative HPLC (Acetonitril/ Wasser 10:90 → 95:5) gereinigt.
Ausbeute: 23 mg (43% d.Th.)
1H-NMR (400 MHz, DMSO-d6): δ = 8.25-8.17 (m, 3H), 7.98 (d, 2H), 7.61 (d, 2H), 4.83 (t, 1H), 4.52 (s, 2H), 3.69-3.62 (m, 2H), 3.61-3.53 (m, 2H).

LC-MS (Methode 1): Rt = 2.05 min; MS (ESIpos): m/z = 495 [M+H]+.

Die in Tabelle 4 aufgeführten Beispiele wurden aus den entsprechenden Ausgangsverbindungen analog zu Beispiel 19 mit anschließender Aufreinigung [präparative HPLC (Chromasil, Wasser/Acetonitril ggf. mit Säurezusatz)] hergestellt:

**Tabelle 4:**

| **Beispiel Nr.** | **Struktur (Ausbeute)** | **LC-MS: Rₜ [min] (Methode) ; MS (ESI): m/z [M+H]⁺** | **¹H-NMR (DMSO-d₆):** |
|---|---|---|---|
| **20** | 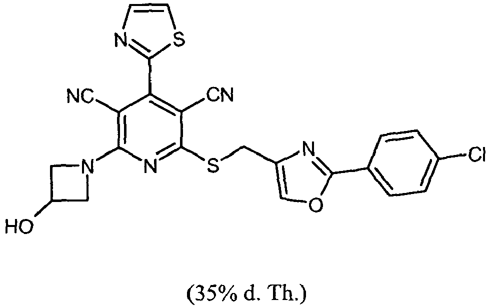 | 2.10 min (Methode 1); m/z = 507 | δ(400MHz)=8.22-8.15 (m, 3H), 7.98 (d, 2H), 7.61 (d, 2H), 5.89 (br s, 1H), 4.70 (br s, 2H), 4.62 (br s, 1H), 4.49 (s, 2H), 4.19 (br s, 2H). |
| **21** | 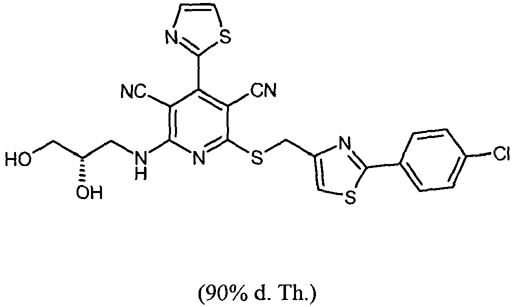 | 1.26 min (Methode 3); **m**/**z** = 541 | δ (400 MHz) = 8.19 (dd, 2H), 8.09 (t, 1H), 7.97 (d, 2H), 7.73 (s, 1H), 7.56 (d, 2H), 4.96 (d, 1H), 4.80-4.69 (m, 3H), 3.81-3.70 (m, 2H), 3.58-3.46 (m, 1H), 3.45-3.34 (m, 2H). |
| **22** | 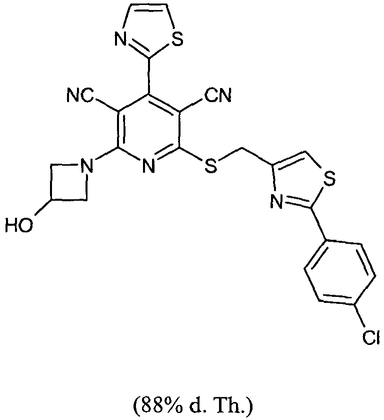 | 1.37 min (Methode 3); **m**/**z** = 523 | δ (400 MHz) = 8.19 (dd, 2H), 7.95 (d, 2H), 7.69 (s, 1H), 7.58 (d, 2H), 5.89 (d, 1H), 4.80-4.55 (m, 5 H), 4.19 (br s, 2H). |
| **23** | 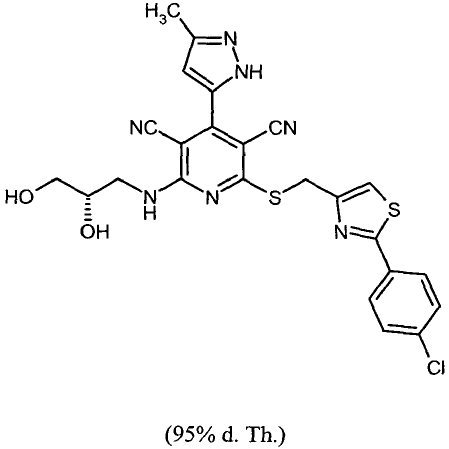 | 1.22 min (Methode 3); m/z = 538 | δ (400 MHz) = 13.30 (br s, 1H), 7.97 (d, 2H), 7.79-7.70 (m, 2H), 7.58 (d, 2H), 6.52 (s, 1H), 4.71 (dd, 2H), 4.62 (s, 2H), 2.32 (s, 3H) 3.90-3.62 (m, 4H), 3.53-3.45 (m, 1H), 3.43-3.31 (m, 2H), 2.32 (s, 3H). |
| **24** | 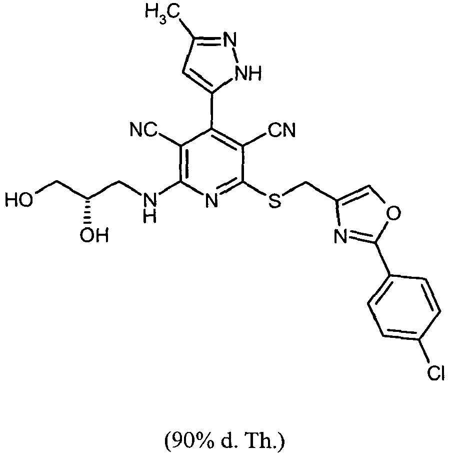 | 1.17 min (Methode 3); m/z = 522 | δ(400 MHz) = 13.29 (br s, 1H), 8.20 (s, 1H), 7.98 (d, 2H), 7.79-7.70 (m, 1H), 7.61 (d, 2H), 6.52 (s, 1H), 4.94 (br s, 1H), 4.72 (br s, 1H), 4.52 (dd, 2H), 3.70-3.68 (m, 2H), 3.57-3.47 (m, 1H), 3.43-3.30 (m, 2H), 2.32 (s, 3H). |
| **25** | 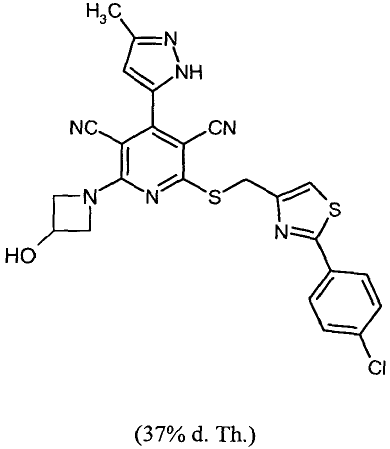 | 1.31 min (Methode 3); m/z = 520 | δ (400 MHz) = 13.28 (s, 1H), 7.96 (d, 2H), 7.68 (s, 1H), 7.57 (d, 2H), 6.48 (s, 1H), 5.84 (br s, 1H), 4.71-4.52 (m, 5H), 4.12 (br s, 2H), 2.30 (s, 3H). |
| **26** | 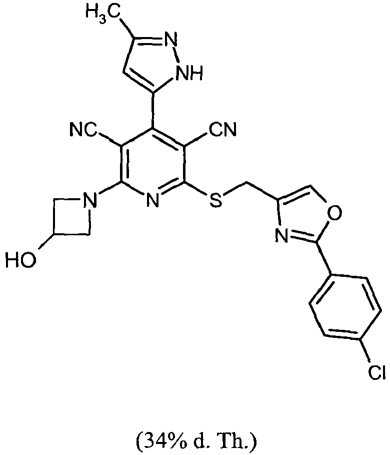 | 1.25 min (Methode 3); **m**/**z** = 504 | δ (400 MHz) = 13.28 (s, 1H), 8.18 (s, 1H), 7.98 (d, 2H), 7.61 (d, 2H), 6.46 (s, 1H), 5.84 (d, 1H), 4.72-4.52 (m, 3H), 4.48 (s, 2H), 4.14 (d, 2H), 2.31 (s, 3H). |
| **27** | 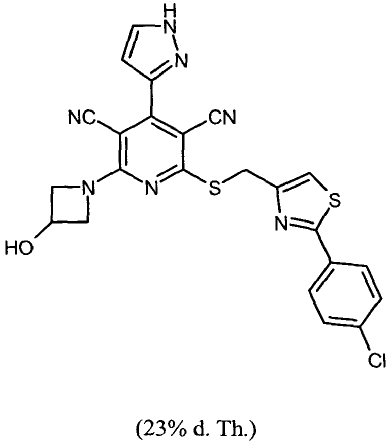 | 2.05 min (Methode 3); m/z = 506 | 5 (400 MHz) = 13.59 (br s, 1H), 7.99-7.92 (m, 3H), 7.68 (s, 1H), 7.58 (d, 2H), 6.75 (s, 1H), 5.87 (d, 1H), 4.72-4.53 (m, 5H), 4.15 (d, 2H). |
| **28** | 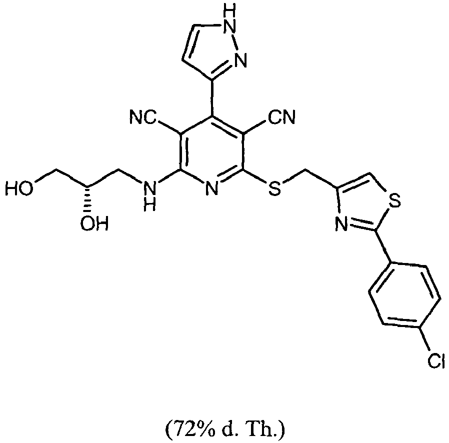 | 1.18 min (Methode 3); m/z = 524 | δ (400 MHz) = 13.61 (br s, 1H), 7.99-7.92 (m, 3H), 7.83-7.77 (m, 1H), 7.73 (s, 1H), 7.57 (d, 2H), 6.79 (d, 1H), 4.71 (dd, 2H), 3.90-3.62 (m, 4H), 3.55-3.47 (m, 1H), 3.43-3.32 (m, 2H). |
| **29** | 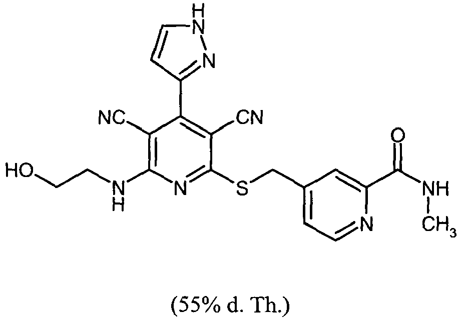 | 0.84 min (Methode 3); m/z = 435 | δ (400 MHz) = 13.60 (br s, 1H), 8.79 (q, 1H), 8.58 (d, 1H), 8.12 (s, 1H), 7.98 (s, 1H), 7.91-7.83 (m, 1H), 7.64 (dd, 1H), 6.79 (d, 1H), 4.64 (s, 2H), 3.53-3.41 (m, 4H), 2.82 (d, 3H). |

### Beispiel 30

2-Amino-6-([2-(4-chlorphenyl)-1,3-thiazol-4-yl]methylsulfanyl)-4-[1-(2-hydroxypropyl)-1H-pyrazol-3-yl]pyridin-3,5-dicarbonitril

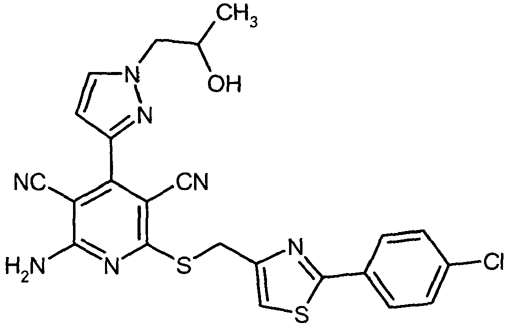

10 mg (0.022 mmol) 2-Amino-6-({[2-(4-chlorphcnyl)-1,3-thiazol-4-yl]methyl}sulfanyl)-4-(1H-pyrazol-3-yl)pyridin-3,5-dicarbonitril wurden in 1 ml 2-Methyloxiran gelöst und mit einer Spatelspitze Cäsiumcarbonat versetzt. Das Reaktionsgemisch wurde über Nacht bei Raumtemperatur gerührt, eingeengt, mit etwas Methanol aufgenommen und mittels präparativer HPLC (Acetonitril/ Wasser: 10:90 → 95:5, Zusatz von 0.1% TFA) gereinigt. Es wurden 10 mg (86 % d. Th.) der Zielverbindung erhalten.

¹H-NMR (400 MHz, DMSO-d₆): δ = 8.26 (br s, 2H), 7.95 (d, 2H), 7.88 (d, 1H), 7.71 (s, 1H), 7.56 (d, 2H), 6.74 (d, 1H), 4.62 (s, 2H), 4.13 (d, 2H), 4.05-3.99 (m, 1H), 1.04 (d, 3H), 3.37 (d, 2H).

LC-MS (Methode 2): Rₜ = 2.58 min; MS (ESIpos): m/z = 508 [M+H]⁺.

### Beispiel 31

2-Amino-6-({[2-(4-chlorphenyl)-1,3-thiazol-4-yl]methyl}sulfanyl)-4-[1-(2-hydroxyethyl)-1H-pyrazol-3-yl]pyridin-3,5-dicarbonitril

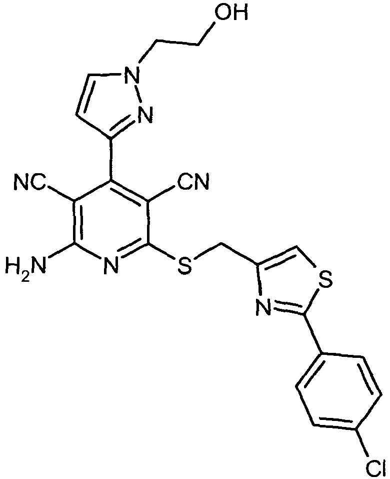

100 mg (0.18 mmol) der Verbindung aus Beispiel 5 wurden in 1.5 ml DMF gelöst, mit 90 mg (0.28 mmol) Cäsiumcarbonat und 158 mg (0.92 mmol) Iodethanol versetzt und über Nacht bei 80°C gerührt. Anschließend wurden nochmals 90 mg (0.28 mmol) Cäsiumcarbonat hinzugegeben und die Reaktionsmischung wurde nochmals 4h bei 80°C gerührt. Der Ansatz wurde mit wenig Wasser und THF versetzt, so dass eine klare Lösung entsteht, und über präparative HPLC (Acetonitril/Wasser 10:90 → 95:5, Zusatz von 0.1% TFA) gereinigt.
Ausbeute: 41 mg (40% d.Th.)
¹H-NMR (400 MHz, DMSO-d₆): δ = 8.05 (br s, 2H), 7.96-7.88 (m, 4H), 7.57 (d, 2H), 6.77 (d, 1H), 4.62 (s, 2H), 4.23 (t, 2H), 3.78 (t, 2H).

LC-MS (Methode 1): Rₜ = 2.05 min; MS (ESIpos): m/z = 494 [M+H]⁺.

### Beispiel 32

2-Amino-6-({[2-(4-chlorphenyl)-1,3-thiazol-4-yl]methyl}sulfanyl)-4-thiophen-2-ylpyridin-3,5-dicarbonitril

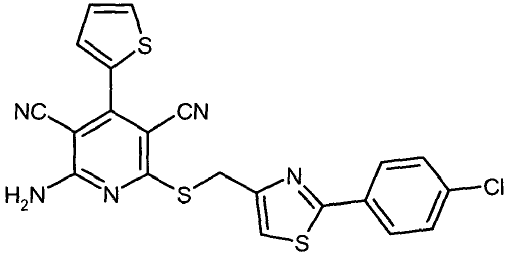

70 mg (0.271 mmol) 2-Amino-6-sulfanyl-4-thiophen-2-ylpyridin-3,5-dicarbonitril wurden gemeinsam mit 79 mg (0.325 mmol) 4-(Chlormethyl)-2-(4-chlorphenyl)-1,3-thiazol und 91 mg (1.084 mmol) Natriumhydrogencarbonat in 2 ml DMF über Nacht bei Raumtemperatur gerührt. Die Reaktionsmischung wurde filtriert und das Filtrat mittels präparativer HPLC (Chromasil, Wasser/Acetonitril - ohne Säure) gereinigt. Es wurden 94 mg (71% d. Th.) der Zielverbindung erhalten.

¹H-NMR (400 MHz, DMSO-d₆): δ = 8.15 (br s, 2H), 7.96-7-91 (m, 4H), 7.58-7.55 (m, 3H), 7.27 (dd, 1H), 3.33 (s, 2H).

LC-MS (Methode 4): Rₜ = 2.79 min; MS (ESIpos): m/z = 466 [M+H]⁺.

### Beispiel 33

2-Amino-6-({[2-(4-chlorphenyl)-1,3-thiazol-4-yl]methyl}sulfanyl)-4-[1-(3-hydroxypropyl)-1H-pyrazol-3-yl]pyridin-3,5-dicarbonitril

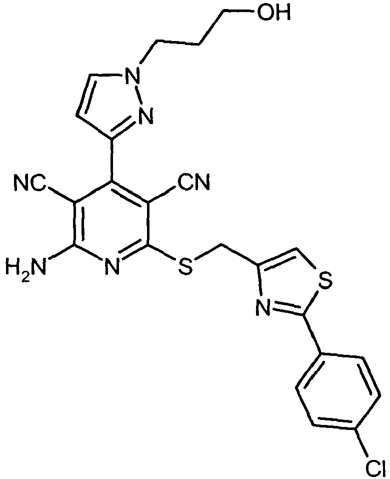

50 mg (0.092 mmol) der Verbindung aus Beispiel 5 wurden in 0.8 ml DMF gelöst, mit 15 mg (0.101 mmol) DBU und 21 mg (0.111 mmol) 3-Iodpropanol versetzt und über Nacht bei 120°C gerührt. Der Ansatz wurde mit wenig Wasser und Essigsäureethylester versetzt und die beiden entstandenen Phasen wurden voneinander getrennt. Die wässrige Phase wurde zweimal mit Essigsäureethylester extrahiert. Die vereinten organischen Phasen wurden einrotiert und der Rückstand wurde mittels Dickschichtchromatographie gereinigt (Toluol:Acetonitril = 3:1).
Ausbeute: 18 mg (38% d.Th.)
¹H-NMR (400 MHz, DMSO-d₆): δ = 8.10 (br s, 2H), 7.96-7.90 (m, 4H), 7.57 (d, 2H), 6.74 (d, 1H), 4.62 (s, 2H), 4.60 (t, 1H), 4.26 (t, 2H), 3.40 (q, 2H), 1.96 (Quintett, 2H).

LC-MS (Methode 2): Rₜ = 2.54 min; MS (ESIpos): m/z = 508 [M+H]⁺.

Die in Tabelle 5 aufgeführten Beispiele wurden aus den entsprechenden Ausgangsverbindungen analog zu Beispiel 33 mit anschließender Aufreinigung [präparative HPLC (Chromasil, Wasser/Acetonitril ggf. mit Säurezusatz)] hergestellt:

**Tabelle 5:**

| **Beispiel Nr.** | **Struktur (Ausbeute)** | **LC-MS: Rₜ [min] (Methode) ; MS (ESI): m/z** [**M**+**H**]⁺ | **¹H-NMR (DMSO-d₆):** |
|---|---|---|---|
| **34** | 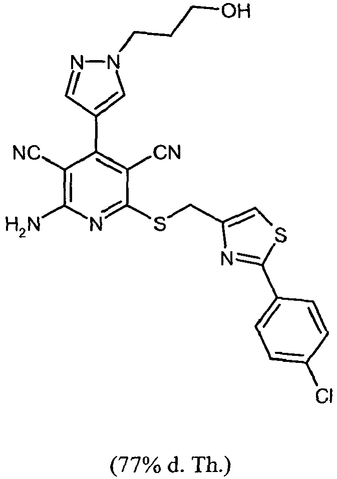 | 2.01 min (Methode 1); m/z = 508 | δ(400 MHz) = 8.31 (s, 1H), 8.09 (br s, 2H), 7.95 (d, 2H), 7.90 (d, 2H), 7.58 (d, 2H), 4.62 (s, 2H), 4.28 (t, 2H), 3.41 (t, 2H), 1.94 (Quintett, 2H). |
| **35** | 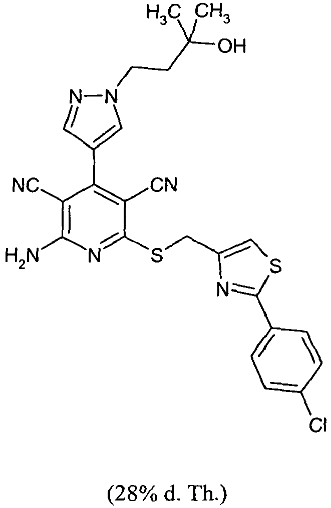 | 2.14 min (Methode 1); m/z = 536 | δ(400MHz)=8.32 (s, 1H), 8.09 (br s, 2H), 7.95 (d, 2H), 7.90 (s, 2H), 7.58 (d, 2H), 4.62 (s, 2H), 4.49 (s, 1H), 4.29 (t, 2H), 1.92 (t, 2H). |

### Beispiel 36

2-Amino-6-({[2-(4-chlorophenyl)-1,3-thiazol-4-yl]methyl}sulfanyl)-4-[1-(2-hydroxyethyl)-1H-pyrazol-4-yl]pyridin-3,5-dicarbonitril

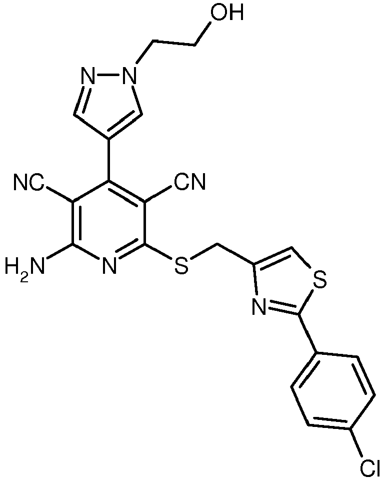

80 mg (0.153 mmol) der Verbindung aus Beispiel 19A wurden in 3 ml Methanol und 3 ml THF vorgelegt und mit 17 mg (0.460 mmol) Natriumborhydrid versetzt. Die Reaktionsmischung wurde 1 h bei RT gerührt und anschließend wurden nochmals 17 mg (0.460 mmol) Natriumborhydrid hinzugegeben und 1h bei RT gerührt. Der Ansatz wurde mit wenig Wasser versetzt und direkt über eine präparative HPLC (Acetonitril/Wasser 10:90 → 95:5, Zusatz von 0.1% TFA) gereinigt.
Ausbeute: 70 mg (92% d.Th.)
¹H-NMR (400 MHz, DMSO-d₆): δ = 8.30 (s, 1H), 8.09 (br s, 2H), 7.97-7.91 (m, 3H), 7.89 (s, 1H), 7.57 (d, 2H), 4.62 (s, 2H), 4.26 (t, 2H), 3.76 (t, 2H).

LC-MS (Methode 2): Rₜ = 2.40 min; MS (ESIpos): m/z = 494 [M+H]⁺.

### Beispiel 38

2-({[2-(4-Chlorphenyl)-1,3-thiazol-4-yl]methyl}sulfanyl)-6-(methylamino)-4-(1H-pyrazol-3-yl)pyridin-3,5-dicarbonitril

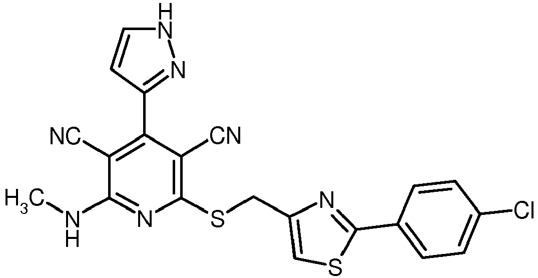

300 mg (0.639 mmol) 2-Chlor-6-({[2-(4-chlorphenyl)-1,3-thiazol-4-yl]methyl}sulfanyl)-4-(1H-pyrazol-3-yl)pyridin-3,5-dicarbonitril wurden in 2 ml Tetrahydrofuran gelöst und mit 0.639 ml (1.278 mmol) 2 M Methylaminlösung in Tetrahydrofuran versetzt. Nach 30 minütigem Rühren wurde Methanol zugegeben, filtriert und das Filtrat mittels präparativer HPLC gereinigt. Man erhielt 173 mg (58 % d. Th.) der Zielverbindung.

¹H-NMR (400 MHz, DMSO-d₆): δ = 13.59 (s, 1H), 8.08 (m, 1H), 7.98 (d, 1H), 7.95 (d, 2H), 7.69 (s, 1H), 7.57 (d, 2H), 6.79 (d, 1H), 4.72 (s, 2H), 3.01 (s, 3H).

LC-MS (Methode 3): Rₜ = 1.42 min; MS (ESIpos): m/z = 464 [M+H]⁺.

### Beispiel 39

2-({[2-(4-Chlorphenyl)-1,3-thiazol-4-yl]methyl}sulfanyl)-6-[(2-hydroxyethyl)amino]-4-(1H-pyrazol-3-yl)pyridin-3,5-dicarbonitril

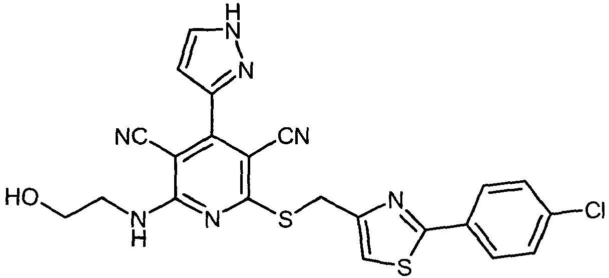

58 mg (0.124 mmol) 2-Chlor-6-({[2-(4-chlorphenyl)-1,3-thiazol-4-yl]methyl}sulfanyl)-4-(1H-pyrazol-3-yl)pyridin-3,5-dicarbonitril wurden in 1 ml Tetrahydrofuran gelöst und mit 15 mg (0.247 mmol) 2-Aminoethanol versetzt. Nach 30 minütigem Rühren wurde Methanol zugegeben, filtriert und das Filtrat mittels präparativer HPLC gereinigt. Man erhielt 63 mg (100 % d. Th.) der Zielverbindung.

¹H-NMR (400 MHz, DMSO-d₆): δ = 13.60 (s, 1H), 7.99 (m, 1H), 7.96 (d, 2H), 7.91 (m, 1H), 7.71 (s, 1H), 7.57 (d, 2H), 6.78 (d, 1H), 4.80 (t, 1H), 4.70 (s, 2H), 3.60 (m, 2H), 3.54 (m, 2H).

LC-MS (Methode 2): Rₜ = 2.45 min; MS (ESIpos): m/z = 494 [M+H]⁺.

### Beispiel 40

2-({[2-(4-Chlorphenyl)-1,3-thiazol-4-yl]methyl}sulfanyl)-6-[(2-hydroxyethyl)amino]-4-(1,3-thiazol-5-yl)pyridin-3,5-dicarbonitril

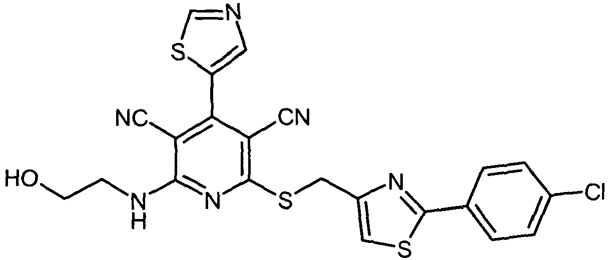

100 mg (Reinheit 91%, 0.186 mmol) 2-Chlor-6-({[2-(4-chlorphenyl)-1,3-thiazol-4-yl]methyl}sulfanyl)-4-(1,3-thiazol-5-yl)pyridin-3,5-dicarbonitril wurden in 5 ml THF vorgelegt, mit 0.023 ml (0.373 mmol) 2-Aminoethanol versetzt und 30 min bei Raumtemperatur gerührt. Anschließend wurde das Produkt mittels präparativer HPLC isoliert. Man erhielt 64 mg (67% d. Th.) der Zielverbindung.

¹H-NMR (400 MHz, DMSO-d₆): 9.41 (s, 1H), 8.31 (s, 1H), 8.20 (t, 1H), 7.93 (d, 2H), 7.71 (s, 1H), 7.57 (d, 2H), 4.81 (m, 1H), 4.71 (s, 2H), 3.61 (m, 2H), 3.53 (m, 2H).

LC-MS (Methode 3): Rₜ = 1.33 min; MS (ESIpos): m/z = 511 [M+H]⁺.

### Beispiel 41

2-Amino-6-({[2-(4-chlorphenyl)-1,3-thiazol-4-yl]methyl}sulfanyl)-4-[2-(3-hydroxypropyl)-1,3-thiazol-4-yl]pyridin-3,5-dicarbonitril

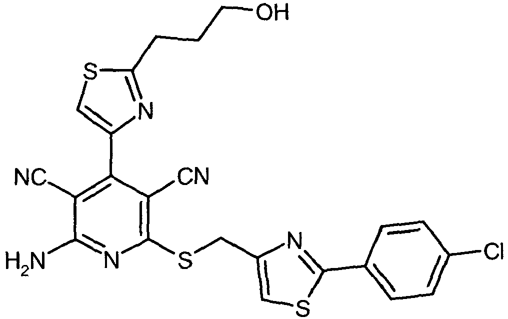

Unter Argon wurden 220 mg des Gemisches aus Beispiel 22A in 10 ml Essigsäureethylester vorgelegt, mit 110 mg Palladium (10 %-ig auf Aktivkohle) versetzt und über Nacht bei Wasserstoffnormaldruck und Raumtemperatur hydriert. Der Katalysator wurde über Celite abfiltriert, der Filterkuchen wurde mit Essigsäureethylester gewaschen und das Filtrat wurde eingeengt. Der Rückstand wurde mittels präparativer HPLC gereinigt. Man erhielt 31 mg (17% d. Th.) der Zielverbindung.

¹H-NMR (400 MHz, DMSO-d₆): 8.40-8.00 (m br, 2H), 8.16 (s, 1H), 7.95 (d, 2H), 7.90 (s, 1H), 7.56 (d, 2H), 4.64 (s, 2H), 3.49 (t, 2H), 3.08 (t, 2H), 1.91 (m, 2H).

LC-MS (Methode 7): Rₜ = 1.17 min; MS (ESIpos): m/z = 525 [M+H]⁺.

### Beispiel 42

2-Amino-6-({[2-(4-chlorphenyl)-1,3-thiazol-4-yl]methyl}sulfanyl)-4-(2-{[(2S)-2,3-dihydroxypropyl]amino}-1,3-thiazol-4-yl)pyridin-3,5-dicarbonitril

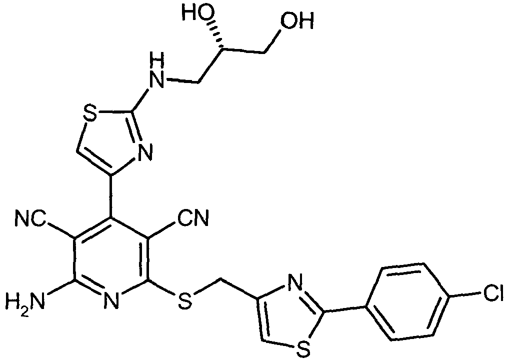

100 mg (0.183 mmol) 2-Amino-4-(2-brom-1,3-thiazol-4-yl)-6-({[2-(4-chlorphenyl)-1,3-thiazol-4-yl]methyl}sulfanyl)pyridin-3,5-dicarbonitril wurden in 2 ml Aceton vorgelegt, mit 834 mg (9.159 mmol) (2S)-3-Aminopropan-1,2-diol versetzt und 12h bei 80°C gerührt. Anschließend wurden weitere 834 mg (9.159 mmol) (2S)-3-Aminopropan-1,2-diol nachgegeben und weitere 12h bei 80°C gerührt. Das Reaktionsgemisch wurde eingeengt und das Produkt mittels präparativer HPLC isoliert. Man erhielt 19 mg (19% d. Th.) der Zielverbindung.

¹H-NMR (400 MHz, DMSO-d₆): 8.30-7.99 (m, 2H), 7.94 (d, 2H), 7.89 (s, 1H), 7.86 (m, 1H), 7.56 (d, 2H), 7.23 (s, 1H), 4.62 (s, 2H), 3.68 (m, 2H), 3.60-3.40 (m, 3H), 3.36 (d, 1H), 3.17 (m, 1H).

LC-MS (Methode 7): Rₜ = 1.09 min; MS (ESIpos): m/z = 556 [M+H]⁺.

### Beispiel 43

2-Amino-6-({[2-(4-chlorphenyl)-1,3-thiazol-4-yl]methyl}sulfanyl)-4-{2-[(2-hydroxyethyl)(methyl)amino]-1,3-thiazol-5-yl}pyridin-3,5-dicarbonitril

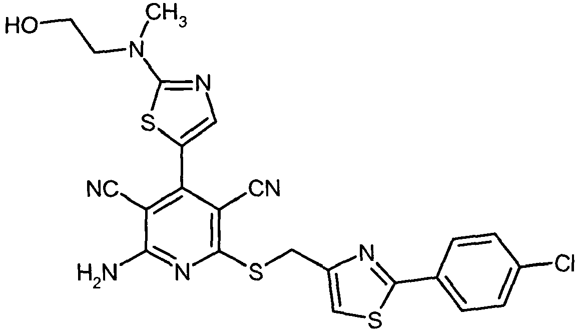

130 mg (0.219 mmol) 2-Amino-6-({[2-(4-chlorphenyl)-1,3-thiazol-4-yl]methyl}sulfanyl)-4-(2-iod-1,3-thiazol-5-yl)pyridin-3,5-dicarbonitril wurden in 2.5 ml Aceton vorgelegt, mit 0.885 ml (10.963 mmol) 2-(Methylamino)ethanol versetzt und über Nacht bei 80°C gerührt. Das Reaktionsgemisch wurde eingeengt und das Produkt mittels präparativer HPLC isoliert. Man erhielt 74 mg (63% d. Th.) der Zielverbindung.

¹H-NMR (400 MHz, DMSO-d₆): 8.26-8.01 (m, 2H), 7.94 (d, 2H), 7.88 (s, 1H), 7.74 (s, 1H), 7.56 (d, 2H), 4.89 (t, 1H), 4.61 (s, 2H), 3.64 (m, 2H), 3.58 (m, 2H), 3.15 (s, 3H).

LC-MS (Methode 7): Rₜ = 1.18 min; MS (ESIpos): m/z = 540 [M+H]⁺.

### Beispiel 44

2-Amino-6-({[2-(4-chlorphenyl)-1,3-thiazol-4-yl]methyl}sulfanyl)-4-{2-[(2-hydroxyethyl)amino]-1,3-thiazol-5-yl}pyridin-3,5-dicarbonitril

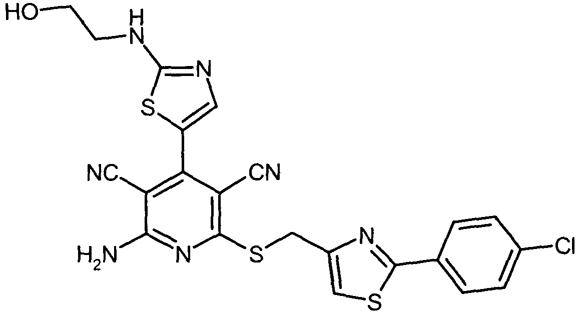

94 mg (0.159 mmol) 2-Amino-6-({[2-(4-chlorphenyl)-1,3-thiazol-4-yl]methyl}sulfanyl)-4-(2-iod-1,3-thiazol-5-yl)pyridin-3,5-dicarbonitril wurden in 2.5 ml Aceton vorgelegt, mit 0.479 ml (7.927 mmol) 2-Aminoethanol versetzt und über Nacht bei 80°C gerührt. Das Reaktionsgemisch wurde eingeengt und das Produkt mittels präparativer HPLC isoliert. Man erhielt 7 mg (8% d. Th.) der Zielverbindung.

¹H-NMR (400 MHz, DMSO-d₆): 8.39 (m, 1H), 8.34-7.99 (m, 2H), 7.94 (d, 2H), 7.88 (s, 1H), 7.64 (s, 1H), 7.56 (d, 2H), 4.83 (t, 1H), 4.61 (s, 2H), 3.56 (m, 2H), 3.37 (m, 2H).

LC-MS (Methode 7): Rₜ = 1.11 min; MS (ESIpos): m/z = 526 [M+H]⁺.

Die in Tabelle 7 aufgeführten Beispiele wurden aus den entsprechenden Ausgangsverbindungen analog zu Beispiel 19 mit anschließender Aufreinigung [präparative HPLC (Chromasil, Wasser/Acetonitril ggf. mit Säurezusatz)] hergestellt:

**Tabelle 7:**

| | | | |
|---|---|---|---|
| **Beispiels Nr.** | **Struktur (Ausbeute)** | **LC-MS: Rₜ [min] (Methode) ; MS (ESI): m/z [M+H]⁺** | **¹H-NMR (DMSO-d₆):** |
| **45** | 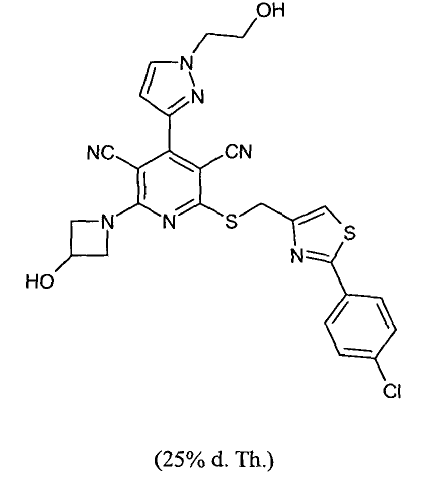 | 2.01 min (Methode 1); m/z = 550 | δ (400 MHz) = 7.96 (d, 2H), 7.91 (d, 1H), 7.68 (s, 1H), 7.58 (d, 2H), 6.73 (d, 1H), 5.87 (d, 1H), 4.92 (t, 1H), 4.73-4.53 (m, 5H), 4.28-4.08 (m, 4H), 3.78 (t, 2H). |
| **46** | 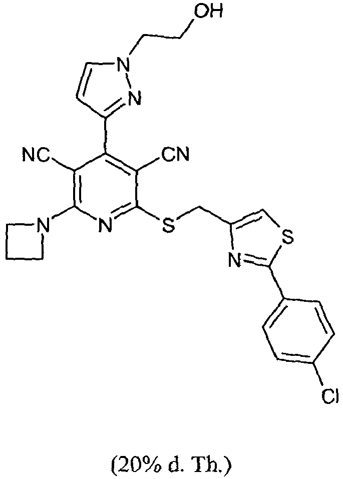 | 2.59 min (Methode 5); m/z = 534 | δ (400 MHz) = 7.96 (d, 2H), 7.91 (d, 1H), 7.68 (s, 1H), 7.58 (d, 2H), 6.72 (d, 1H), 4.92 (t, 1H), 4.67 (s, 2H), 4.58-4.32 (m, 4H), 4.25 (t, 2H), 3.76 (q, 2H), 2.39-2.30 (m, 2H). |

### Beispiel 47

2-({[2-(4-Chlorphenyl)-1,3-thiazol-4-yl]methyl}sulfanyl)-6-[(3R)-3-hydroxypyrrolidin-1-yl]-4-(1,3-thiazol-5-yl)pyridin-3,5-dicarbonitril

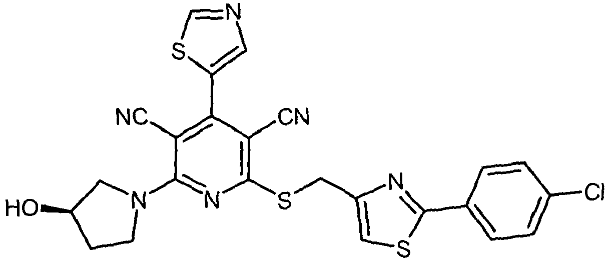

100 mg (ca. 0.186 mmol) 2-Chlor-6-({[2-(4-chlorphenyl)-1,3-thiazol-4-yl]methyl}sulfanyl)-4-(1,3-thiazol-5-yl)pyridin-3,5-dicarbonitril wurden in 5 ml THF vorgelegt, mit 33 mg (0.373 mmol) (R)-(+)-3-Pyrrolidinol versetzt und 30 min bei Raumtemperatur gerührt. Anschließend wurde das Produkt mittels präparativer HPLC isoliert. Man erhielt 78 mg (78% d. Th.) der Zielverbindung.

¹H-NMR (400 MHz, DMSO-d₆): δ = 9.40 (s, 1H), 8.28 (s, 1H), 7.95 (d, 2H), 7.70 (s, 1H), 7.57 (d, 2H), 5.15 (d, 1H), 4.71 (s, 2H), 4.40 (m, 1H), 3.92 (m, 3H), 3.76 (d, 1H), 2.10-1.80 (d, 2H).

LC-MS (Methode 3): Rₜ = 1.36 min; MS (ESIpos): m/z = 537 [M+H]⁺.

### B. Bewertung der pharmakologischen und physiologischen Wirksamkeit

Die pharmakologische und physiologische Wirkung der erfindungsgemäßen Verbindungen kann in folgenden Assays gezeigt werden:

### B-1. Indirekte Bestimmung des Adenosin-Agonismus über Genexpression

Zellen der permanenten Linie CHO (Chinese Hamster Ovary) werden stabil mit der cDNA für die Adenosin-Rezeptor-Subtypen A1, A2a und A2b transfiziert. Die Adenosin-A1-Rezeptoren sind über Gᵢ-Proteine und die Adenosin-A2a- und A2b-Rezeptoren über Gₛ-Proteine an die Adenylatcyclase gekoppelt. Entsprechend wird die cAMP-Bildung in der Zelle inhibiert bzw. stimuliert. Über einen cAMP-abhängigen Promotor wird danach die Expression der Luziferase moduliert. Der Luziferase-Test wird mit dem Ziel hoher Sensitivität und Reproduzierbarkeit, geringer Varianz und guter Eignung für die Durchführung auf einem Robotersystem optimiert durch Variation mehrerer Testparameter, wie z.B. Zelldichte, Dauer der Anzuchtphase und der Testinkubation, Forskolin-Konzentration und Medium-Zusammensetzung. Zur pharmakologischen Charakterisierung der Zellen und zum Roboter-gestützten Substanz-Screening wird das folgende Testprotokoll verwendet:
Die Stammkulturen werden in DMEM/F12-Medium mit 10% FCS (fötales Kälberserum) bei 37°C unter 5% CO₂ gezüchtet und jeweils nach 2-3 Tagen 1:10 gesplittet. Testkulturen werden mit 2000 Zellen pro Napf in 384-well-Platten ausgesät und ca. 48 Stunden bei 37°C angezogen. Dann wird das Medium durch eine physiologische Kochsalzlösung (130 mM Natriumchlorid, 5 mM Kaliumchlorid, 2 mM Calciumchlorid, 20 mM HEPES, 1 mM Magnesiumchlorid-Hexahydrat, 5 mM Natriumhydrogencarbonat, pH 7.4) ersetzt. Die in DMSO gelösten zu testenden Substanzen werden in einer Verdünnungsreihe von 5 x 10⁻¹¹ M bis 3 x 10⁻⁶ M (Endkonzentration) zu den Testkulturen pipettiert (maximale DMSO-Endkonzentration im Testansatz: 0.5%). 10 Minuten später wird Forskolin zu den A1-Zellen zugegeben und anschließend werden alle Kulturen für vier Stunden bei 37°C inkubiert. Danach wird zu den Testkulturen 35 µl einer Lösung, bestehend zu 50% aus Lyse-Reagenz (30 mM Dinatriumhydrogenphosphat, 10% Glycerin, 3% TritonX100, 25 mM TrisHCl, 2 mM Dithiotreitol (DTT), pH 7.8) und zu 50% aus Luciferase-Substrat-Lösung (2.5 mM ATP, 0.5 mM Luciferin, 0.1 mM Coenzym A, 10 mM Tricin, 1.35 mM Magnesiumsulfat, 15 mM DTT, pH 7.8) zugegeben, ca. 1 Minute geschüttelt und die Luciferase-Aktivität mit einem Kamerasystem gemessen. Bestimmt werden die EC₅₀-Werte, d.h. die Konzentrationen, bei denen bei der A1-Zelle 50% der Luciferase-Antwort inhibiert bzw. bei den A2b- und A2a-Zellen 50% der maximalen Stimulierbarkeit mit der entsprechenden Substanz erreicht sind. Als Referenzverbindung dient in diesen Experimenten die Adenosin-analoge Verbindung NECA (5-*N*- Ethylcarboxamido-adenosin), die mit hoher Affinität an alle Adenosin-Rezeptor-Subtypen bindet und eine agonistische Wirkung besitzt [Klotz, K.N., Hessling, J., Hegler, J., Owman, C., Kull, B., Fredholm, B.B., Lohse, M.J., "Comparative pharmacology of human adenosine receptor subtypes - characterization of stably transfected receptors in CHO cells", Naunyn Schmiedebergs Arch. Pharmacol. 357, 1-9 (1998)].

In der folgenden Tabelle 1 sind die EC₅₀-Werte repräsentativer Ausführungsbeispiele für die Rezeptorstimulation an Adenosin A1-, A2a- und A2b-Rezeptor-Subtypen aufgeführt:

**Tabelle 6**

| **Beispiel Nr.** | **EC50 A1 [nM] (1 µM Forskolin)** | **EC50 A2a [nM]** | **EC50 A2b [nM]** |
|---|---|---|---|
| **1** | 0.5 | 3000 | 1570 |
| **4** | 0.9 | 3000 | 3000 |
| **8** | 1.2 | 275 | 715 |
| **9** | 1.4 | 3000 | 3000 |
| **12** | 2.4 | 3000 | 3000 |
| **16** | 0.4 | 3000 | 3000 |
| **20** | 0.5 | 3000 | 1800 |
| **21** | 0.5 | 378 | 3000 |
| **22** | 0.6 | 2470 | 1280 |
| **27** | 1.1 | 6500 | 6500 |
| **30** | 1.9 | 2000 | 1410 |
| **32** | 0.3 | 184 | 34 |
| **37** | 0.05 | 88 | 28 |
| **47** | 0.5 | 3000 | 3000 |

### B-2. Untersuchung an isolierten Gefäßen

Aus narkotisierten Ratten wird die Arteria caudalis präpariert und in eine konventionelle Apparatur zur Messung isolierter Gefäße eingespannt. Die Gefäße werden in einem Wärmebad perfundiert und mit Phenylephrin kontrahiert. Das Maß der Kontraktion wird über einen Kontraktionsmesser ermittelt. Zu den vorkontrahierten Gefäßen werden Testsubstanzen gegeben und die Abnahme der Kontraktion der Gefäße gemessen. Eine Abnahme der Kontraktion entspricht einer Dilatation der Gefäße. Als EC₅₀-Wert einer Testsubstanz bzgl. ihrer relaxierenden Eigenschaften wird die Konzentration angegeben, bei der die Kontraktion der Gefäße um 50% verringert ist.

### B-3. Blutdruck- und Herzfrequenz-Messungen an wachen Ratten

Wachen SHR (spontaneously hypertensive rats)-Ratten, die einen internen Sender tragen, der dauerhaft sowohl Blutdruck als auch Herzfrequenz messen kann (telemetrische Erfassung von hämodynamischen Parametern), werden Testsubstanzen in verschiedenen Dosierungen oral verabreicht. Anschließend werden über 24 Stunden Blutdruck und Herzfrequenz und deren Veränderungen aufgezeichnet.

### B-4. Blutdruck- und Herzfrequenz-Messungen an wachen Krallenaffen

Wachen Krallenaffen, die einen internen Sender tragen, der dauerhaft sowohl Blutdruck als auch Herzfrequenz messen kann (telemetrische Erfassung von hämodynamischen Parametern), werden Testsubstanzen in verschiedenen Konzentrationen oral verabreicht. Anschließend werden über 6-24 Stunden Blutdruck und Herzfrequenz und deren Veränderungen aufgezeichnet.

### B-5. Indirekte Bestimmung des Adenosin-Antagonismus über Genexpression

Zellen der permanenten Linie CHO K1 (Chinese Hamster Ovary) werden stabil mit einem Reporterkonstrukt (CRE-Luciferase) und der cDNA für die Adenosin-Rezeptorsubtypen A2a oder A2b transfiziert. A2a- bzw. A2b-Rezeptoren sind über Gas-Proteine an die Adenylatcyclase gekoppelt. Durch Rezeptoraktivierung wird die Adenylatcyclase aktiviert und damit das cAMP-Level in der Zelle erhöht. Über das Reporterkonstrukt, einem cAMP-abhängigen Promotor, ist die Änderung des cAMP-Levels an die Luciferase-Expression gekoppelt.

Für die Bestimmung von Adenosin-Antagonismus am Adenosin-Rezeptorsubtyp A1 werden ebenfalls CHO K1-Zellen stabil transfiziert, diesmal allerdings mit einem Ca²⁺-sensitiven Reporterkonstrukt (NFAT-TA-Luc; Clontech) und einem A1-Gα16 Fusionskonstrukt. Diese Rezeptorchimäre ist im Gegensatz zum nativen A1-Rezeptor (Gai-Kopplung) an die Phospholipase C gekoppelt. Die Luciferase wird hier in Abhängigkeit von der cytosolischen Ca²⁺-Konzentration exprimiert.

Die permanenten Zelllinien werden in DMEM/F12 (Cat.-No BE04-687Q; BioWhittaker) mit 10% FCS (Fetales Kälberserum) und diversen Zusätzen (20 ml/Liter IM HEPES (Cat.-No 15630; Gibco), 20 ml/Liter GlutaMAX (Cat.-No 35050-038, Gibco), 14 ml/Liter MEM Natriumpyruvat (Cat.-No 11360-039; Gibco), 10 ml/Liter PenStrep (Cat.-No 15070-063; Gibco)) bei 37°C unter 5% Kohlendioxid kultiviert und zweimal pro Woche gesplittet.

Für die Testung im 384-well Plattenformat werden die Zellen mit 2000 Zellen/Well in 25µl/Well Aussaatmedium ausgesät und bis zur Substanztestung bei 37°C unter 5% Kohlendioxid kultiviert. Die A2a- und A2b-Zellen werden 24 h vor der Substanztestung in Medium mit Zusätzen und 5% FCS ausgesät, wobei für die A2a-Zellen als Basismedium DMEM/F12 und für die A2b-Zellen OptiMEM (Cat.-No 31985-047; Gibco) verwendet wird. Die A1-Gα16-Zellen werden 48 h vor Substanztestung in OptiMEM mit 2.5% dialysiertem FCS und Zusätzen ausgesät. Am Testtag wird vor der Substanzzugabe das Medium durch 25 µl Cafty-Puffer (Cat.-No T21-154; PAA) mit 2 mM Calciumchlorid und 0.1% BSA (Rinderserumalbumin) ersetzt. Von den zu testenden, in DMSO gelösten Substanzen werden Verdünnungsreihen in Cafty-Puffer mit 2 mM Calciumchlorid und 0.1% BSA (Rinderserumalbumin) und einer geeigneten Konzentration des Agonisten angesetzt. Die Substanzen werden in einer Endkonzentration von 5 x 10⁻⁵ M bis 2.56 x 10⁻¹¹ M zu den Testkulturen pipettiert, wobei der DMSO-Gehalt auf den Zellen 0.5% nicht überschreiten darf. Als Agonist wird für die A2a- und A2b-Zellen NECA (5-N-Ethylcarboxamido-adenosin) in einer Endkonzentration von 30 nM, was etwa der EC₅₀-Konzentration entspricht, eingesetzt. Für die A1-Gα16-Zellen wird 25 nM CPA (N6-cyclopentyl-adenosine) als Agonist eingesetzt, was etwa der EC₇₅-Konzentration entspricht. Nach der Substanzzugabe werden die Zellplatten 3-4 h bei 37°C unter 5% Kohlendioxid inkubiert. Anschließend werden die Zellen direkt vor der Messung mit 25 µl einer Lösung, bestehend zu 50% aus Lyse-Reagenz (30 mM Dinatriumhydrogenphosphat, 10% Glycerin, 3% Triton X-100, 25 mM TrisHCl, 2 mM Dithiotreitol (DTT), pH 7.8) und zu 50% aus Luciferase-Substrat-Lösung (2.5 mM ATP, 0.5 mM Luciferin, 0.1 mM Coenzym A, 10 mM Tricin, 1.35 mM Magnesiumsulfat, 15 mM DTT, pH 7.8) versetzt. Die Luciferase-Aktivität wird mit einem Lumineszenz-Reader detektiert. Bestimmt werden die 1C₅₀-Werte, d.h. die Konzentration, bei denen die Luciferase-Antwort, hervorgerufen durch den jeweiligen Agonisten zu 50% inhibiert wird. Als Referenz-Antagonist wird für die A2a- und A2b-Zellen ZM241385 und für die A1-Gα16-Zellen DPCPX (1,3-dipropyl-8-cyclopentylxanthine) verwendet.

**Tabelle 7**

| **Beispiel Nr.** | **IC50 A1 [nM]** | **EC50 A2a [nM]** | **EC50 A2b [nM]** |
|---|---|---|---|
| **15** | 0.5 | 50000 | 40 |
| **29** | 3.6 | 306 | 267 |

### B-6. Bestimmung pharmakokinetischer Kenngrößen nach intravenöser und oraler Gabe

Die zu untersuchende Substanz wird Tieren (z.B. Maus, Ratte, Hund) intravenös als Lösung appliziert, die orale Applikation erfolgt als Lösung oder Suspension über eine Schlundsonde. Nach Substanzgabe wird den Tieren zu festgelegten Zeitpunkten Blut entnommen. Dieses wird heparinisiert, anschließend wird daraus durch Zentrifugation Plasma gewonnen. Die Substanz wird im Plasma über LC/MS-MS analytisch quantifiziert. Aus den so ermittelten Plasmakonzentration-Zeit-Verläufen werden die pharmakokinetischen Kenngrößen wie AUC (Fläche unter der Konzentration-Zeit-Kurve), Cₘₐₓ (maximale Plasmakonzentration), T_{1/2} (Halbwertszeit) und CL (Clearance) mittels eines validierten pharmakokinetischen Rechenprogramms berechnet.

### B-7. Bestimmung der Löslichkeit

### Benötigte Reagenzien:

- PBS-Puffer pH 6.5: 90.00 g NaCl p.a. (z.B. Fa. Merck, Art.-Nr. 1.06404.1000), 13.61 g KH₂PO₄ p.a. (z.B. Fa. Merck, Art.-Nr. 1.04873.1000) und 83.35 g 1 N Natronlauge (z.B. Fa. Bernd Kraft GmbH, Art.-Nr. 01030.4000) werden in einen 1 Liter-Messkolben eingewogen, mit destilliertem Wasser auf 1 Liter aufgefüllt und für 1 Stunde gerührt. Danach wird mit 1 N Salzsäure (z.B. Fa. Merck, Art.-Nr. 1.09057.1000) der pH-Wert auf 6.5 eingestellt.
- PEG/Wasser-Lösung (70:30 v/v): 70 ml Polyethylenglykol 400 (z.B. Fa. Merck, Art.-Nr. 8.17003.1000) und 30 ml destilliertes Wasser werden in einem 100 ml-Messkolben homogenisiert.
- PEG/PBS-Puffer pH 6.5 (20:80 v/v): 20 ml Polyethylenglykol 400 (z.B. Fa. Merck, Art.-Nr. 8.17003.1000) und 80 ml PBS-Puffer pH 6.5 werden in einem 100 ml-Messkolben homogenisiert.
- Dimethylsulfoxid (z.B. Fa. Baker, Art.-Nr. 7157.2500)
- destilliertes Wasser.

### Herstellung der Ausgangslösung (Urlösung):

Mindestens 4 mg der Testsubstanz werden in ein Weithals-10 mm Screw V-Vial (Fa. Glastechnik Gräfenroda GmbH, Art.-Nr. 8004-WM-H/V15µ) mit passender Schraubkappe und Septum genau eingewogen, in einem Pipettierroboter mit DMSO bis zu einer Konzentration von 50 mg/ml versetzt und 10 Minuten geschüttelt.

### Herstellung der Kalibrierlösungen:

*Herstellung der Ausgangslösung für Kalibrierlösungen* (*Stammlösung*)*:* In eine Mikrotiterplatte werden 10 µl der Urlösung mit Hilfe eines Pipettierroboters überführt und mit DMSO bis zu einer Konzentration von 600 µg/ml ausgefüllt. Die Probe wird bis zu ihrer vollständigen Lösung geschüttelt.

*Kalibrierlösung 1 (20 µg*/*ml):* 34.4 µl der Stammlösung werden mit 1000 µl DMSO versetzt und homogenisiert.

*Kalibrierlösung 2 (2.5 µg*/*ml):* 100 µl der Kalibrierlösung 1 werden mit 700 µl DMSO versetzt und homogenisiert.

### Herstellung der Probenlösungen:

*Probenlösung für Löslichkeit bis 5 g*/*Liter in PBS-Puffer pH 6.5:* In eine Mikrotiterplatte werden 10 µl Urlösung transferiert und mit 1000 µl PBS-Puffer pH 6.5 versetzt.

*Probenlösung für Löslichkeit bis 5 g*/*Liter in PEG*/*Wasser (70:30):* In eine Mikrotiterplatte werden 10 µl Urlösung transferiert und mit 1000 µl PEG/Wasser (70:30) versetzt.

*Probenlösung für Löslichkeit bis 5 g*/*Liter in PEG*/*PBS-Puffer pH 6.5 (20:80):* In eine Mikrotiterplatte werden 10 µl Urlösung transferiert und mit 1000 µl PEG/PBS-Puffer pH 6.5 (20:80) versetzt.

### Durchführung:

Die so hergestellten Probenlösungen werden 24 Stunden bei 1400 rpm mittels eines temperierbaren Schüttlers (z.B. Fa. Eppendorf Thermomixer comfort Art.-Nr. 5355 000.011 mit Wechselblock Art.-Nr. 5362.000.019) bei 20°C geschüttelt. Von diesen Lösungen werden jeweils 180 µl abgenommen und in Beckman Polyallomer Centrifuge Tubes (Art.-Nr. 343621) überführt. Diese Lösungen werden 1 Stunde mit ca. 223.000 x g zentrifugiert (z.B. Fa. Beckman Optima L-90K Ultracentrifuge mit Type 42.2 Ti Rotor bei 42.000 rpm). Von jeder Probenlösung werden 100 µl des Überstandes abgenommen und mit DMSO zu 1:5 und 1:100 verdünnt. Es wird von jeder Verdünnung eine Abfüllung in ein geeignetes Gefäß für die HPLC-Analytik vorgenommen.

### Analytik:

Die Proben werden mittels RP-HPLC analysiert. Quantifiziert wird über eine Zwei-Punkt-Kalibrationskurve der Testverbindung in DMSO. Die Löslichkeit wird in mg/Liter ausgedrückt. Analysensequenz: 1) Kalibrierlösung 2.5 mg/ml; 2) Kalibrierlösung 20 µg/ml; 3) Probenlösung 1:5; 4) Probenlösung 1:100.

### HPLC-Methode für Säuren:

Agilent 1100 mit DAD (G1315A), quat. Pumpe (G1311A), Autosampler CTC HTS PAL, Degaser (G1322A) und Säulenthermostat (G1316A); Säule: Phenomenex Gemini C18, 50 mm x 2 mm, 5 µ; Temperatur: 40°C; Eluent A: Wasser/Phosphorsäure pH 2; Eluent B: Acetonitril; Flussrate: 0.7 ml/min; Gradient: 0-0.5 min 85% A, 15% B; Rampe: 0.5-3 min 10% A, 90% B; 3-3.5 min 10% A, 90% B; Rampe: 3.5-4 min 85% A, 15% B; 4-5 min 85% A, 15% B.

### HPLC-Methode für Basen:

Agilent 1100 mit DAD (G1315A), quat. Pumpe (G1311A), Autosampler CTC HTS PAL, Degaser (G1322A) und Säulenthermostat (G1316A); Säule: VDSoptilab Kromasil 100 C18, 60 mm x 2.1 mm, 3.5 µ; Temperatur: 30°C; Eluent A: Wasser + 5 ml Perchlorsäure/Liter; Eluent B: Acetonitril; Flussrate: 0.75 ml/min; Gradient: 0-0.5 min 98% A, 2% B; Rampe: 0.5-4.5 min 10% A, 90% B; 4.5-6 min 10% A, 90% B; Rampe: 6.5-6.7 min 98% A, 2% B; 6.7-7.5 min 98% A, 2% B.

### B-8. Bestimmung der metabolischen Stabilität

Zur Bestimmung der metabolischen Stabilität von Testverbindungen werden diese *in vitro* mit Lebermikrosomen oder bevorzugt mit primären frischen Hepatozyten verschiedener Tierspezies (z.B. von Ratte und Hund) als auch humanen Ursprungs inkubiert, um Metabolitenprofile eines möglichst kompletten hepatischen Phase I- und Phase II-Metabolismus zu erhalten und zu vergleichen.

Die Testverbindungen werden mit einer Konzentration von 10-20 µM inkubiert. Dazu werden Stammlösungen der Substanzen mit einer Konzentration von 1-2 mM in Acetonitril hergestellt und dann mit einer 1:100-Verdünnung in den Inkubationsansatz pipettiert. Die Lebermikrosomen werden in 50 mM Kaliumphosphat-Puffer (pH 7.4) mit und ohne NADPH-generierendem System, bestehend aus 1 mM NADP⁺, 10 mM Glucose-6-phosphat und 1 Einheit Glucose-6-phosphat-Dehydrogenase, bei 37°C inkubiert. Primäre Hepatozyten werden in Suspension in Williams E-Medium ebenfalls bei 37°C inkubiert. Nach einer Inkubationszeit von 0-4 Stunden werden die Inkubationsansätze mit Acetonitril abgestoppt (Endkonzentration ca. 30%) und das Protein bei ca. 15000 x g abzentrifugiert. Die so abgestoppten Proben werden entweder direkt analysiert oder bis zur Analyse bei -20°C gelagert.

Die Analyse erfolgt mittels Hochleistungsflüssigkeits-Chromatographie mit Ultraviolett- und massenspektrometrischer Detektion (HPLC-UV-MS/MS). Dazu werden die Überstände der Inkubationsproben mit geeigneten C 18-reversed-phase-Säulen und variablen Eluenten-Gemischen aus Acetonitril und 10 mM wässriger Ammoniumformiat-Lösung chromatographiert. Die UV-Chromatogramme in Verbindung mit massenspektrometrischen MS/MS-Daten dienen zur Identifizierung und Strukturaufklärung der Metabolite.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 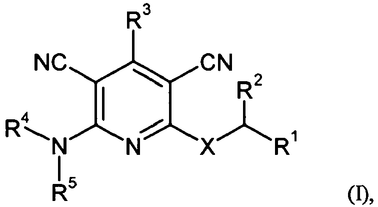 in welcher
X für O oder S steht,
R¹ für 5- oder 6-gliedriges Heteroaryl, steht,
wobei 5- oder 6-gliedriges Heteroaryl mit 1 oder 2 Substituenten unabhängig voneinander ausgewählt aus der Gruppe Halogen, Nitro, Cyano, (C₁-C₆)-Alkyl, Trifluormethyl, Hydroxy, (C₁-C₆)-Alkoxy, Amino, Mono-(C₁-C₆)-alkylamino, Di-(C₁-C₆)-alkylamino, Hydroxycarbonyl, (C₁-C₆)-Alkoxycarbonyl, Aminocarbonyl, Mono-(C₁-C₆)-alkylaminocarbonyl, Di-(C₁-C₆)-alkyl-aminocarbonyl, (C₃-C₇)-Cycloalkylaminocarbonyl, Aminosulfonyl, Mono-(C₁-C₆)-alkylaminosulfonyl, Di-(C₁-C₆)-alkylaminosulfonyl, (C₁-C₆)-Alkylsulfonylamino, Pyrrolidino, Piperidino, Morpholino, Piperazino, *N'*-(C₁-C₄)-Alkylpiperazino, Pyrrolidinocarbonyl, Piperidinocarbonyl, Morpholinocarbonyl, Piperazinocarbonyl, *N'*-(C₁-C₄)-Alkylpiperazinocarbonyl, Phenyl und 5- oder 6-gliedriges Heteroaryl substituiert ist,
worin Phenyl und 5- oder 6-gliedriges Heteroaryl mit 1 bis 3 Substituenten unabhängig voneinander ausgewählt aus der Gruppe Halogen, Nitro, Cyano, (C₁-C₆)-Alkyl, Difluormethyl, Trifluormethyl, Hydroxy, (C₁-C₆)-Alkoxy, Difluormethoxy, Trifluormethoxy, Amino, Mono-(C₁-C₆)-alkylamino, Di-(C₁-C₆)-alkylamino, Hydroxycarbonyl und (C₁-C₆)-Alkoxycarbonyl substituiert sein können,
R² für Wasserstoff oder (C₁-C₄)-Alkyl steht,
R³ für Thienyl, Pyrrolyl, Oxazolyl, Thiazolyl, Isoxazolyl, Isothiazolyl, Pyrazolyl, Imidazolyl, Triazolyl, Oxadiazolyl, Thiadiazolyl oder Tetrazolyl steht,
wobei Thienyl, Pyrrolyl, Oxazolyl, Thiazolyl, Isoxazolyl, Isothiazolyl, Pyrazolyl, Imidazolyl, Triazolyl, Oxadiazolyl, Thiadiazolyl und Tetrazolyl mit 1 oder 2 Substituenten unabhängig voneinander ausgewählt aus der Gruppe Halogen, Cyano, Hydroxy, (C₁-C₆)-Alkyl, (C₁-C₆)-Alkoxy, (C₃-C₇)-Cycloalkoxy und -NR^{A}R^{B} substituiert sein können,
worin (C₁-C₆)-Alkyl und (C₁-C₆)-Alkoxy mit 1 bis 3 Substituenten unabhängig voneinander ausgewählt aus der Gruppe Fluor, Trifluormethyl, (C₃-C₇)-Cycloalkyl, Oxo, Hydroxy, (C₁-C₄)-Alkoxy, Hydroxycarbonyl, Amino, Mono-(C₁-C₄)-alkylamino und Di-(C₁-C₄)-alkylamino substituiert sein können, und
worin (C₃-C₇)-Cycloalkoxy mit 1 oder 2 Substituenten unabhängig voneinander ausgewählt aus der Gruppe (C₁-C₄)-Alkyl, Hydroxy, Oxo und (C₁-C₄)-Alkoxy substituiert sein kann,
und
worin
R^{A} für Wasserstoff oder (C₁-C₆)-Alkyl steht, worin (C₁-C₆)-Alkyl seinerseits mit 1 bis 3 Substituenten Fluor substituiert sein kann,
und
worin (C₁-C₆)-Alkyl seinerseits mit einem Substituenten ausgewählt aus der Gruppe Hydroxy und (C₁-C₄)-Alkoxy substituiert sein kann,
R^{B} für Wasserstoff, (C₁-C₆)-Alkyl, (C₃-C₇)-Cycloalkyl, (C₁-C₆)-Alkylcarbonyl, (C₁-C₄)-Alkylsulfonyl oder (C₃-C₇)-Cycloalkylsulfonyl steht,
worin (C₁-C₆)-Alkyl seinerseits mit 1 bis 3 Substituenten Fluor substituiert sein kann,
und
worin (C₁-C₆)-Alkyl seinerseits mit 1 oder 2 Substituenten unabhängig voneinander ausgewählt aus der Gruppe (C₃-C₇)-Cycloalkyl, Oxo, Hydroxy, (C₁-C₄)-Alkoxy, Hydroxycarbonyl, Amino, Mono-(C₁-C₄)-alkylamino, Di-(C₁-C₄)-alkylamino und (C₃-C₇)-Cycloalkylamino substituiert sein kann,
und
worin (C₃-C₇)-Cycloalkyl seinerseits mit 1 oder 2 Substituenten unabhängig voneinander ausgewählt aus der Gruppe (C₁-C₄)-Alkyl, Hydroxy, Oxo und (C₁-C₄)-Alkoxy substituiert sein kann, oder
R⁴ R^{A} und R^{B} zusammen mit dem Stickstoffatom, an das sie gebunden sind, einen 4- bis 7-gliedrigen Heterocyclus bilden, der ein weiteres Ring-Heteroatom aus der Reihe N, O oder S enthalten und mit 1 oder 2 Substituenten unabhängig voneinander ausgewählt aus der Gruppe (C₁-C₄)-Alkyl, Hydroxy, Oxo und (C₁-C₄)-Alkoxy substituiert sein kann, für Wasserstoff oder (C₁-C₆)-Alkyl steht, wobei (C₁-C₆)-Alkyl mit 1 oder 2 Substituenten unabhängig voneinander ausgewählt aus der Gruppe Hydroxy, (C₁-C₄)-Alkoxy, Hydroxycarbonyl, (C₁-C₄)-Alkoxycarbonyl, Amino, Mono-(C₁-C₄)-alkylamino und Di-(C₁-C₄)-alkylamino substituiert sein kann,
R⁵ für Wasserstoff oder (C₁-C₆)-Alkyl steht,
wobei (C₁-C₆)-Alkyl mit 1 oder 2 Substituenten unabhängig voneinander ausgewählt aus der Gruppe Hydroxy, (C₁-C₄)-Alkoxy, Hydroxycarbonyl, (C₁-C₄)-Alkoxycarbonyl, Amino, Mono-(C₁-C₄)-alkylamino und Di-(C₁-C₄)-alkylamino substituiert sein kann,
R⁴ und R⁵ zusammen mit dem Stickstoffatom, an das sie gebunden sind, einen 4- bis 7-gliedrigen Heterocyclus bilden, der ein weiteres Ring-Heteroatom aus der Reihe N, O oder S enthalten und mit 1 oder 2 Substituenten unabhängig voneinander ausgewählt aus der Gruppe (C₁-C₄)-Alkyl, Hydroxy, Oxo und (C₁-C₄)-Alkoxy substituiert sein kann,
sowie ihre N-Oxide, Salze, Solvate, Salze der N-Oxide und Solvate der N-Oxide und Salze.

2. Verbindung der Formel (I) nach Anspruch 1, in welcher
X für O oder S steht,
R¹ für 5- oder 6-gliedriges Heteroaryl steht,
wobei 5- oder 6-gliedriges Heteroaryl mit 1 oder 2 Substituenten unabhängig voneinander ausgewählt aus der Gruppe Fluor, Chlor, Cyano, (C₁-C₄)-Alkyl, Trifluormethyl, (C₁-C₄)-Alkoxy, Mono-(C₁-C₄)-alkylamino, Di-(C₁-C₄)-alkylamino, Hydroxycarbonyl, (C₁-C₄)-Alkoxycarbonyl, Aminocarbonyl, Mono-(C₁-C₄)-alkylaminocarbonyl, Di-(C₁-C₄)-alkylaminocarbonyl, Pyrrolidino, Piperidino, Morpholino, Piperazino, *N*'-(C₁-C₄)-Alkylpiperazino, Phenyl und 5- oder 6-gliedriges Heteroaryl substituiert ist,
worin Phenyl und 5- oder 6-gliedriges Heteroaryl mit 1 oder 2 Substituenten unabhängig voneinander ausgewählt aus der Gruppe Fluor, Chlor, Cyano, (C₁-C₄)-Alkyl, Difluormethyl, Trifluormethyl, (C₁-C₄)-Alkoxy, Difluormethoxy, Trifluormethoxy, Hydroxycarbonyl und (C₁-C₄)-Alkoxycarbonyl substituiert sein können,
R² für Wasserstoff oder Methyl steht,
R³ für Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isoxazol-5-yl, Pyrazol-1-yl, Pyrazol-3-yl, Pyrazol-4-yl, Pyrazol-5-yl, Imidazol-1-yl, Imidazol-2-yl, Imidazol-4-yl oder Imidazol-5-yl steht,
wobei Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isoxazol-5-yl, Pyrazol-1-yl, Pyrazol-3-yl, Pyrazol-4-yl, Pyrazol-5-yl, Imidazol-1-yl, Imidazol-2-yl, Imidazol-4-yl und Imidazol-5-yl mit 1 oder 2 Substituenten unabhängig voneinander ausgewählt aus der Gruppe Fluor, Trifluormethyl (C₁-C₆)-Alkyl, (C₁-C₄)-Alkoxy und -NR^{A}R^{B} substituiert sein können,
worin (C₁-C₆)-Alkyl und (C₂-C₄)-Alkoxy mit 1 oder 2 Substituenten unabhängig voneinander ausgewählt aus der Gruppe Fluor, Trifluormethyl, Hydroxy, Methoxy, Ethoxy, Hydroxycarbonyl, Amino, Methylamino, Ethylamino, N,N-Dimethylamino und N,N-Diethylamino substituiert sein können,
und
worin
R^{A} für Wasserstoff oder (C₁-C₄)-Alkyl steht, worin (C₁-C₄)-Alkyl seinerseits mit einem Substituenten ausgewählt aus der Gruppe Hydroxy und (C₁-C₄)-Alkoxy substituiert sein kann,
R^{B} für Wasserstoff oder (C₁-C₄)-Alkyl steht,
worin (C₁-C₄)-Alkyl seinerseits mit 1 oder 2 Substituenten unabhängig voneinander ausgewählt aus der Gruppe Hydroxy, (C₁-C₄)-Alkoxy und Hydroxycarbonyl substituiert sein kann,
R⁴ für Wasserstoff oder Methyl steht,
R⁵ für Wasserstoff oder (C₁-C₄)-Alkyl steht,
wobei (C₁-C₄)-Alkyl mit 1 oder 2 Substituenten unabhängig voneinander ausgewählt aus der Gruppe Hydroxy, Methoxy und Ethoxy substituiert sein kann,
oder
R⁴ und R⁵ zusammen mit dem Stickstoffatom, an das sie gebunden sind, einen 4- bis 6-gliedrigen Heterocyclus bilden, der ein weiteres Ring-Heteroatom aus der Reihe N, O oder S enthalten und mit 1 oder 2 Substituenten unabhängig voneinander ausgewählt aus der Gruppe (C₁-C₄)-Alkyl, Hydroxy, Methoxy und Ethoxy substituiert sein kann,
sowie ihre Salze, Solvate und Solvate der Salze.

3. Verbindung der Formel (I) nach Anspruch 1 oder 2, in welcher
X für S steht,
R¹ für Oxazolyl, Thiazolyl oder Pyridyl steht, wobei Pyridyl mit einem Substituenten ausgewählt aus der Gruppe Cyano, Methoxy, Ethoxy, Hydroxycarbonyl, Methoxycarbonyl, Ethoxycarbonyl, Aminocarbonyl, Methylaminocarbonyl und Dimethylaminocarbonyl substituiert ist, und wobei Pyridyl mit einem Substituenten ausgewählt aus der Gruppe Fluor, Chlor, Methyl, Ethyl und Trifluormethyl substituiert sein kann, und wobei Oxazolyl und Thiazolyl mit einem Substituenten Phenyl substituiert sind, worin Phenyl mit einem Substituenten ausgewählt aus der Gruppe Fluor, Chlor, Cyano, Methyl, Methoxy, Hydroxycarbonyl und Methoxycarbonyl substituiert sein kann, und wobei Oxazolyl und Thiazolyl mit einem Substituenten ausgewählt aus der Gruppe Fluor, Methyl, Ethyl, Methoxy, Hydroxycarbonyl und Methoxycarbonyl substituiert sein können,
R² für Wasserstoff steht,
R³ für eine Gruppe der Formel 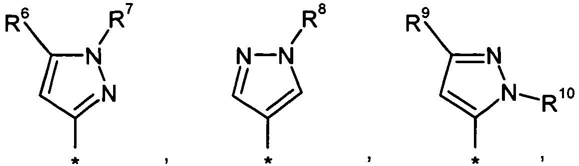 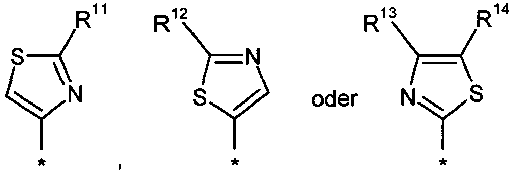 steht,
wobei
* die Anknüpfstelle an das Pyridin bedeutet, und
wobei
R⁶ für Wasserstoff, Methyl oder Ethyl steht,
R⁷ für Wasserstoff oder (C₁-C₆)-Alkyl steht,
worin (C₁-C₆)-Alkyl mit 1 oder 2 Substituenten unabhängig voneinander ausgewählt aus der Gruppe Hydroxy und Methoxy substituiert sein kann,
R⁸ für Wasserstoff oder (C₁-C₆)-Alkyl steht,
worin (C₁-C₆)-Alkyl mit 1 oder 2 Substituenten unabhängig voneinander ausgewählt aus der Gruppe Hydroxy und Methoxy substituiert sein kann,
R⁹ für Wasserstoff, Methyl oder Ethyl steht,
R¹⁰ für Wasserstoff steht,
R¹¹ für Wasserstoff oder (C₁-C₆)-Alkyl steht,
worin (C₁-C₆)-Alkyl mit 1 oder 2 Substituenten unabhängig voneinander ausgewählt aus der Gruppe Hydroxy und Methoxy substituiert sein kann,
R¹² für Wasserstoff oder (C₁-C₆)-Alkyl steht,
worin (C₁-C₆)-Alkyl mit 1 oder 2 Substituenten unabhängig voneinander ausgewählt aus der Gruppe Hydroxy und Methoxy substituiert sein kann,
R¹³ für Wasserstoff, Methyl oder Ethyl steht, und
R¹⁴ für Wasserstoff oder (C₁-C₆)-Alkyl steht,
worin (C₁-C₆)-Alkyl mit 1 oder 2 Substituenten unabhängig voneinander ausgewählt aus der Gruppe Hydroxy und Methoxy substituiert sein kann,
R⁴ für Wasserstoff steht,
R⁵ für Wasserstoff, Ethyl, n-Propyl oder sec.-Butyl steht, wobei Ethyl, n-Propyl und sec.-Butyl mit 1 oder 2 Substituenten Hydroxy substituiert sein können,
oder
R⁴ und R⁵ zusammen mit dem Stickstoffatom, an das sie gebunden sind, einen Azetidinyl- oder Pyrrolidinyl-Ring bilden, der mit einem Substituenten Hydroxy substituiert sein kann,
sowie ihre Salze, Solvate und Solvate der Salze.

4. Verbindung der Formel (I) nach Anspruch 1, in welcher
X für O oder S steht,
R¹ für Oxazolyl, Thiazolyl oder Pyridyl steht,
wobei Oxazolyl, Thiazolyl und Pyridyl mit 1 oder 2 Substituenten unabhängig voneinander ausgewählt aus der Gruppe Fluor, Chlor, Cyano, (C₁-C₄)-Alkyl, Trifluormethyl, (C₁-C₄)-Alkoxy, Hydroxycarbonyl, (C₁-C₄)-Alkoxycarbonyl, Aminocarbonyl, Mono-(C₁-C₄)-alkylaminocarbonyl, Di-(C₁-C₄)-alkylaminocarbonyl, Pyrrolidino, Piperidino, Morpholino, Piperazino, N'-(C₁-C₄)-Alkylpiperazino, Phenyl und 5- oder 6-gliedriges Heteroaryl, substituiert sind,
worin Phenyl und 5- oder 6-gliedriges Heteroaryl mit 1 oder 2 Substituenten unabhängig voneinander ausgewählt aus der Gruppe Fluor, Chlor, Cyano, (C₁-C₄)-Alkyl, Difluormethyl, Trifluormethyl, (C₁-C₄)-Alkoxy, Difluormethoxy, Trifluormethoxy, Hydroxycarbonyl und (C₁-C₄)-Alkoxycarbonyl substituiert sein können,
R² für Wasserstoff oder (C₁-C₄)-Alkyl steht,
R³ für Thien-2-yl oder Thien-3-yl steht,
wobei Thien-2-yl und Thien-3-yl mit 1 oder 2 Substituenten unabhängig voneinander ausgewählt aus der Gruppe Fluor, (C₁-C₆)-Alkyl, (C₁-C₄)-Alkoxy und NR^{A}R^{B} substituiert sein können,
worin (C₁-C₆)-Alkyl und (C₂-C₄)-Alkoxy mit 1 oder 2 Substituenten unabhängig voneinander ausgewählt aus der Gruppe Fluor, Trifluormethyl, Hydroxy, Methoxy, Ethoxy, Hydroxycarbonyl, Amino, Methylamino, Ethylamino, *N,N*-Dimethylamino und *N,N-*Diethylamino substituiert sein können,
und
worin
R^{A} für Wasserstoff oder (C₁-C₄)-Alkyl steht, worin (C₁-C₄)-Alkyl seinerseits mit einem Substituenten ausgewählt aus der Gruppe Hydroxy und (C₁-C₄)-Alkoxy substituiert sein kann,
R^{B} für Wasserstoff oder (C₁-C₄)-Alkyl steht, worin (C₁-C₄)-Alkyl seinerseits mit 1 oder 2 Substituenten unabhängig voneinander ausgewählt aus der Gruppe Hydroxy, (C₁-C₄)-Alkoxy und Hydroxycarbonyl substituiert sein kann,
R⁴ für Wasserstoff oder Methyl steht,
R⁵ für Wasserstoff oder (C₁-C₄)-Alkyl steht, wobei (C₁-C₄)-Alkyl mit 1 oder 2 Substituenten unabhängig voneinander ausgewählt aus der Gruppe Hydroxy, Methoxy und Ethoxy substituiert sein kann,
oder
R⁴ und R⁵ zusammen mit dem Stickstoffatom, an das sie gebunden sind, einen 4- bis 6-gliedrigen Heterocyclus bilden, der ein weiteres Ring-Heteroatom aus der Reihe N, O oder S enthalten und mit 1 oder 2 Substituenten unabhängig voneinander ausgewählt aus der Gruppe (C₁-C₄)-Alkyl, Hydroxy, Methoxy und Ethoxy substituiert sein kann,
sowie ihre Salze, Solvate und Solvate der Salze.

5. Verbindung der Formel (I) nach Anspruch 4, in welcher
X für S steht,
R¹ für Oxazolyl, Thiazolyl oder Pyridyl steht,
wobei Pyridyl mit einem Substituenten ausgewählt aus der Gruppe Cyano, Methoxy, Ethoxy, Hydroxycarbonyl, Methoxycarbonyl, Ethoxycarbonyl, Aminocarbonyl, Methylaminocarbonyl und Dimethylaminocarbonyl substituiert ist,
und
wobei Pyridyl mit einem Substituenten ausgewählt aus der Gruppe Fluor, Chlor, Methyl, Ethyl und Trifluormethyl substituiert sein kann,
und
wobei Oxazolyl und Thiazolyl mit einem Substituenten Phenyl substituiert sind,
worin Phenyl mit einem Substituenten ausgewählt aus der Gruppe Fluor, Chlor, Cyano, Methyl, Methoxy, Hydroxycarbonyl und Methoxycarbonyl substituiert sein kann, und
wobei Oxazolyl und Thiazolyl mit einem Substituenten ausgewählt aus der Gruppe Fluor, Methyl, Ethyl, Methoxy, Hydroxycarbonyl und Methoxycarbonyl substituiert sein können,
R² für Wasserstoff steht,
R³ für eine Gruppe der Formel 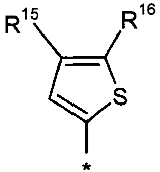 steht,
wobei
* die Anknüpfstelle an das Pyridin bedeutet,
und wobei
R¹⁵ für Wasserstoff, Methyl oder Ethyl steht,
R¹⁶ für Wasserstoff oder (C₁-C₄)-Alkyl steht, worin (C₁-C₄)-Alkyl mit 1 oder 2 Substituenten unabhängig voneinander ausgewählt aus der Gruppe Hydroxy und Methoxy substituiert sein kann,
R⁴ für Wasserstoff steht,
R⁵ für Wasserstoff, Ethyl, n-Propyl oder sec.-Butyl steht,
wobei Ethyl, n-Propyl und sec.-Butyl mit 1 oder 2 Substituenten Hydroxy substituiert sein können,
oder
R⁴ und R⁵ zusammen mit dem Stickstoffatom, an das sie gebunden sind, einen Azetidinyl- oder Pyrrolidinyl-Ring bilden, der mit einem Substituenten Hydroxy substituiert sein kann,
sowie ihre Salze, Solvate und Solvate der Salze.

6. Verfahren zur Herstellung von Verbindungen der Formel (I), wie in den Ansprüchen 1 bis 5 definiert, **dadurch gekennzeichnet, dass** man
[A] eine Verbindung der Formel (II) 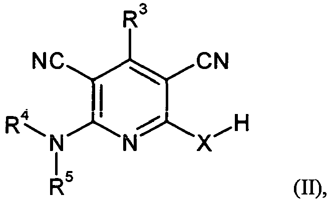 in welcher X, R³, R⁴ und R⁵ jeweils die in den Ansprüchen 1 bis 5 angegebenen Bedeutungen haben,
in einem inerten Lösungsmittel in Gegenwart einer Base mit einer Verbindung der Formel (III) 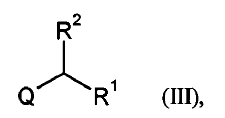 in welcher R¹ und R² jeweils die in den Ansprüchen 1 bis 5 angegebenen Bedeutungen haben und
Q für eine geeignete Abgangsgruppe, vorzugsweise für Halogen, insbesondere Chlor, Brom oder Iod, oder für Mesylat, Tosylat oder Triflat steht,
umsetzt
oder
[B] im Fall, dass X für O steht, eine Verbindung der Formel (IV) 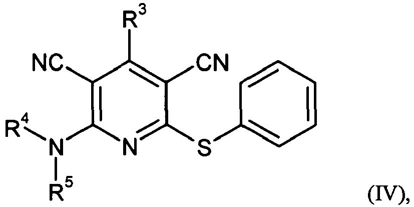 in welcher R³, R⁴ und R⁵ jeweils die in den Ansprüchen 1 bis 5 angegebenen Bedeutungen haben,
in einem inerten Lösungsmittel in Gegenwart einer Base mit einer Verbindung der Formel (V) 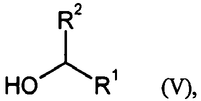 in welcher R¹ und R² die in den Ansprüchen 1 bis 5 angegebenen Bedeutungen haben, umsetzt
oder
[C] eine Verbindung der Formel (I-A) 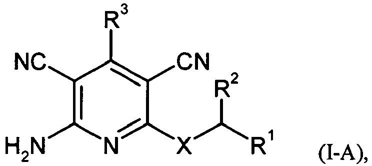 in welcher R¹, R² und R³ jeweils die in den Ansprüchen 1 bis 5 angegebenen Bedeutungen haben,
zunächst mit Kupfer(II)chlorid und Isoamylnitrit in einem geeigneten Lösungsmittel in eine Verbindung der Formel (XV) 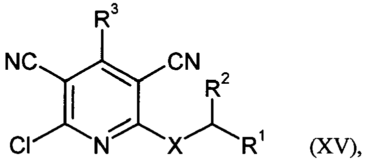 in welcher R¹, R² und R³ jeweils die in den Ansprüchen 1 bis 5 angegebenen Bedeutungen haben,
überführt und diese anschließend mit einer Verbindung der Formel (VIII) 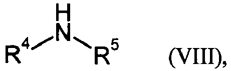 in welcher R⁴ und R⁵ jeweils die in den Ansprüchen 1 bis 5 angegebenen Bedeutungen haben, und
wobei mindestens einer der beiden Reste R⁴ und R⁵ von Wasserstoff verschieden ist,
zu einer Verbindung der Formel (I-B) 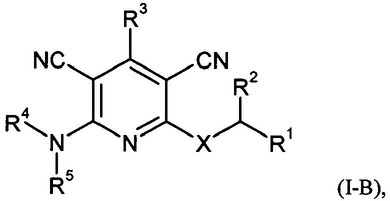 in welcher R¹, R², R³, R⁴ und R⁵ jeweils die in den Ansprüchen 1 bis 5 angegebenen Bedeutungen haben,
und
wobei mindestens einer der beiden Reste R⁴ und R⁵ von Wasserstoff verschieden ist, umsetzt,
anschließend gegebenenfalls vorhandene Schutzgruppen abspaltet und die resultierenden Verbindungen der Formel (I) gegebenenfalls mit den entsprechenden (*i*) Lösungsmitteln und/oder (*ii*) Basen oder Säuren in ihre Solvate, Salze und/oder Solvate der Salze überführt.

7. Verbindung der Formel (I), wie in einem der Ansprüche 1 bis 5 definiert, zur Behandlung und/oder Prävention von Krankheiten.

8. Verbindung der Formel (I), wie in einem der Ansprüche 1 bis 5 definiert, zur Verwendung in einem Verfahren zur Behandlung und/oder Prophylaxe von koronarer Herzerkrankung, akutem Koronarsyndrom, Angina pectoris, Herzinsuffizienz, Myokardinfarkt und Vorhofflimmern.

9. Verbindung der Formel (I), wie in einem der Ansprüche 1 bis 5 definiert, zur Verwendung in einem Verfahren zur Behandlung und/oder Prophylaxe von Diabetes, Metabolischem Syndrom und Dyslipidämien.

10. Verwendung einer Verbindung der Formel (I), wie in einem der Ansprüche 1 bis 5 definiert, zur Herstellung eines Arzneimittels zur Behandlung und/oder Prävention von koronarer Herzerkrankung, akutem Koronarsyndrom, Angina pectoris, Herzinsuffizienz, Myokardinfarkt und Vorhofflimmern.

11. Arzneimittel enthaltend eine Verbindung der Formel (I), wie in einem der Ansprüche 1 bis 5 definiert, in Kombination mit einem inerten, nicht-toxischen, pharmazeutisch geeigneten Hilfsstoff.

12. Arzneimittel enthaltend eine Verbindung der Formel (I), wie in einem der Ansprüche 1 bis 5 definiert, in Kombination mit einem oder mehreren weiteren Wirkstoffen ausgewählt aus der Gruppe bestehend aus den Fettstoffwechsel verändernden Wirkstoffen, Antidiabetika, blutdrucksenkenden Wirkstoffen und antithrombotisch wirkenden Mitteln.

13. Arzneimittel nach Anspruch 11 oder 12 zur Behandlung und/oder Prävention von koronarer Herzerkrankung, akutem Koronarsyndrom, Angina pectoris, Herzinsuffizienz, Myokardinfarkt und Vorhofflimmern.

14. Arzneimittel nach Anspruch 11 oder 12 zur Behandlung und/oder Prävention von Diabetes, Metabolischem Syndrom und Dyslipidämien.

## Claims

1. Compound of the formula (I) 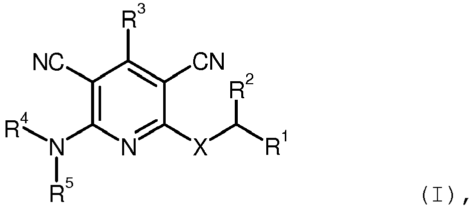 in which
X represents O or S,
R¹ represents 5- or 6-membered heteroaryl,
where 5- or 6-membered heteroaryl is substituted by 1 or 2 substituents independently of one another selected from the group consisting of halogen, nitro, cyano, (C₁-C₆)-alkyl, trifluoromethyl, hydroxyl, (C₁-C₆)-alkoxy, amino, mono- (C₁-C₆)-alkylamino, di-(C₁-C₆)-alkylamino, hydroxycarbonyl, (C₁-C₆)-alkoxycarbonyl, aminocarbonyl, mono-(C₁-C₆)-alkylaminocarbonyl, di-(C₁-C₆)-alkylaminocarbonyl, (C₃-C₇)-cycloalkylaminocarbonyl, aminosulfonyl, mono-(C₁-C₆)-alkylaminosulfonyl, di- (C₁-C₆)-alkyl-aminosulfonyl, (C₁-C₆)-alkylsulfonylamino, pyrrolidino, piperidino, morpholino, piperazino, *N'*-(C₁-C₄)-alkylpiperazino, pyrrolidinocarbonyl, piperidinocarbonyl, morpholinocarbonyl, piperazinocarbonyl, *N'-*(C₁-C₄)-alkylpiperazinocarbonyl, phenyl and 5-or 6-membered heteroaryl,
where phenyl and 5- or 6-membered heteroaryl may be substituted by 1 to 3 substituents independently of one another selected from the group consisting of halogen, nitro, cyano, (C₁-C₆)-alkyl, difluoromethyl, trifluoromethyl, hydroxyl, (C₁-C₆)-alkoxy, difluoromethoxy, trifluoromethoxy, amino, mono-(C₁-C₆)-alkylamino, di-(C₁-C₆)-alkylamino, hydroxycarbonyl and (C₁-C₆)-alkoxycarbonyl,
R² represents hydrogen or (C₁-C₄)-alkyl,
R³ represents thienyl, pyrrolyl, oxazolyl, thiazolyl, isoxazolyl, isothiazolyl, pyrazolyl, imidazolyl, triazolyl, oxadiazolyl, thiadiazolyl or tetrazolyl, where thienyl, pyrrolyl, oxazolyl, thiazolyl, isoxazolyl, isothiazolyl, pyrazolyl, imidazolyl, triazolyl, oxadiazolyl, thiadiazolyl and tetrazolyl may be substituted by 1 or 2 substituents independently of one another selected from the group consisting of halogen, cyano, hydroxyl, (C₁-C₆)-alkyl, (C₁-C₆)-alkoxy, (C₃-C₇)-cycloalkoxy and -NR^{A}R^{B},
where (C₁-C₆)-alkyl and (C₁-C₆)-alkoxy may be substituted by 1 to 3 substituents independently of one another selected from the group consisting of fluorine, trifluoromethyl, (C₃-C₇)-cycloalkyl, oxo, hydroxyl, (C₁-C₄)-alkoxy, hydroxycarbonyl, amino, mono-(Cl-C4)-alkylamino and di- (C₁-C₄)-alkylamino,
and
where (C₃-C₇)-cycloalkoxy may be substituted by 1 or 2 substituents independently of one another selected from the group consisting of (C₁-C₄)-alkyl, hydroxyl, oxo and (C₁-C₄)-alkoxy,
and
where
R^{A} represents hydrogen or (C₁-C₆)-alkyl,
where (C₁-C₆)-alkyl for its part may be substituted by 1 to 3 fluorine substituents,
and
where (C₁-C₆)-alkyl for its part may be substituted by a substituent selected from the group consisting of hydroxyl and (C₁-C₄)-alkoxy,
R^{B} represents hydrogen, (C₁-C₆)-alkyl, (C₃-C₇)-cycloalkyl, (C₁-C₆)-alkylcarbonyl, (C₁-C₄)-alkylsulfonyl or (C₃-C₇)-cycloalkylsulfonyl,
where (C₁-C₆)-alkyl for its part may be substituted by 1 to 3 fluorine substituents,
and
where (C₁-C₆)-alkyl for its part may be substituted by 1 or 2 substituents independently of one another selected from the group consisting of (C₃-C₇)-cycloalkyl, oxo, hydroxyl, (C₁-C₄)-alkoxy, hydroxycarbonyl, amino, mono-(C₁-C₄)-alkylamino, di-(C₁-C₄)-alkylamino and (C₃-C₇)-cycloalkylamino,
and
where (C₃-C₇)-cycloalkyl for its part may be substituted by 1 or 2 substituents independently of one another selected from the group consisting of (C₁-C₄)-alkyl, hydroxyl, oxo and (C₁-C₄)-alkoxy, or
R^{A} and R^{B} together with the nitrogen atom to which they are attached form a 4-to 7-membered heterocycle which may contain a further ring heteroatom from the group consisting of N, O and S and may be substituted by 1 or 2 substituents independently of one another selected from the group consisting of (C₁-C₄)-alkyl, hydroxyl, oxo and (C₁-C₄)-alkoxy,
R⁴ represents hydrogen or (C₁-C₆)-alkyl,
where (C₁-C₆)-alkyl may be substituted by 1 or 2 substituents independently of one another selected from the group consisting of hydroxyl, (C₁-C₄)-alkoxy, hydroxycarbonyl, (C₁-C₄)-alkoxycarbonyl, amino, mono-(C₁-C₄)-alkylamino and di-(C₁-C₄)-alkylamino,
R⁵ represents hydrogen or (C₁-C₆)-alkyl,
where (C₁-C₆)-alkyl may be substituted by 1 or 2 substituents independently of one another selected from the group consisting of hydroxyl, (C₁-C₄)-alkoxy, hydroxycarbonyl, (C₁-C₄)-alkoxycarbonyl, amino, mono-(C₁-C₄)-alkylamino and di-(C₁-C₄)-alkylamino,
or
R⁴ and R⁵ together with the nitrogen atom to which they are attached form a 4- to 7-membered heterocycle which may contain a further ring heteroatom from the group consisting of N, O and S and may be substituted by 1 or 2 substituents independently of one another selected from the group consisting of (C₁-C₄)-alkyl, hydroxyl, oxo and (C₁-C₄)-alkoxy,
or an N-oxide, salt, solvate, salt of an N-oxide or solvate of an N-oxide or salt thereof.

2. Compound of the formula (I) according to Claim 1 in which
X represents O or S,
R¹ represents 5- or 6-membered heteroaryl,
where 5- or 6-membered heteroaryl is substituted by 1 or 2 substituents independently of one another selected from the group consisting of fluorine, chlorine, cyano, (C₁-C₄) -alkyl, trifluoromethyl, (C₁-C₄)-alkoxy, mono- (C₁-C₄) -alkylamino, di-(C₁-C₄)-alkylamino, hydroxycarbonyl, (C₁-C₄)-alkoxy-carbonyl, aminocarbonyl, mono-(C₁-C₄)-alkylaminocarbonyl, di-(C₁-C₄)-alkylaminocarbonyl, pyrrolidino, piperidino, morpholino, piperazino, *N*'-(C₁-C₄)-alkylpiperazino, phenyl and 5- or 6-membered heteroaryl,
where phenyl and 5- or 6-membered heteroaryl may be substituted by 1 or 2 substituents independently of one another selected from the group consisting of fluorine, chlorine, cyano, (C₁-C₄) -alkyl, difluoromethyl, trifluoromethyl, (C₁-C₄)-alkoxy, difluoromethoxy, trifluoromethoxy, hydroxycarbonyl and (C₁-C₄)-alkoxycarbonyl,
R² represents hydrogen or methyl,
R³ represents pyrrol-1-yl, pyrrol-2-yl, pyrrol-3-yl, oxazol-2-yl, oxazol-4-yl, oxazol-5-yl, thiazol-2-yl, thiazol-4-yl, thiazol-5-yl, isoxazol-5-yl, pyrazol-1-yl, pyrazol-3-yl, pyrazol-4-yl, pyrazol-5-yl, imidazol-1-yl, imidazol-2-yl, imidazol-4-yl or imidazol-5-yl,
where pyrrol-1-yl, pyrrol-2-yl, pyrrol-3-yl, oxazol-2-yl, oxazol-4-yl, oxazol-5-yl, thiazol-2-yl, thiazol-4-yl, thiazol-5-yl, isoxazol-5-yl, pyrazol-1-yl, pyrazol-3-yl, pyrazol-4-yl, pyrazol-5-yl, imidazol-1-yl, imidazol-2-yl, imidazol-4-yl and imidazol-5-yl may be substituted by 1 or 2 substituents independently of one another selected from the group consisting of fluorine, trifluoromethyl (C₁-C₆)-alkyl, (C₁-C₄)-alkoxy and -NR^{A}R^{B},
where (C₁-C₆)-alkyl and (C₂-C₄)-alkoxy may be substituted by 1 or 2 substituents independently of one another selected from the group consisting of fluorine, trifluoromethyl, hydroxyl, methoxy, ethoxy, hydroxycarbonyl, amino, methylamino, ethylamino, N,N-dimethylamino and N,N-diethylamino,
and
where
R^{A} represents hydrogen or (C₁-C₄)-alkyl,
where (C₁-C₄)-alkyl for its part may be substituted by a substituent selected from the group consisting of hydroxyl and (C₁-C₄)-alkoxy,
R^{B} represents hydrogen or (C₁-C₄)-alkyl,
where (C₁-C₄)-alkyl for its part may be substituted by 1 or 2 substituents independently of one another selected from the group consisting of hydroxyl, (C₁-C₄)-alkoxy and hydroxycarbonyl,
R⁴ represents hydrogen or methyl,
R⁵ represents hydrogen or (C₁-C₄)-alkyl,
where (C₁-C₄)-alkyl may be substituted by 1 or 2 substituents independently of one another selected from the group consisting of hydroxyl, methoxy and ethoxy,
or
R⁴ and R⁵ together with the nitrogen atom to which they are attached form a 4- to 6-membered heterocycle which may contain a further ring heteroatom from the group consisting of N, O and S and may be substituted by 1 or 2 substituents independently of one another selected from the group consisting of (C₁-C₄) -alkyl, hydroxyl, methoxy and ethoxy,
or a salt, solvate or solvate of a salt thereof.

3. Compound of the formula (I) according to Claim 1 or 2 in which
X represents S,
R¹ represents oxazolyl, thiazolyl or pyridyl,
where pyridyl is substututed by a substituent selected from the group consisting of cyano, methoxy, ethoxy, hydroxycarbonyl, methoxycarbonyl, ethoxycarbonyl, aminocarbonyl, methylaminocarbonyl and dimethylaminocarbonyl,
and
where pyridyl may be substituted by a substituent selected from the group consisting of fluorine, chlorine, methyl, ethyl and trifluoromethyl,
and
where oxazolyl and thiazolyl are substituted by a phenyl substituent,
where phenyl may by substituted by a substituent selected from the group consisting of fluorine, chlorine, cyano, methyl, methoxy, hydroxycarbonyl and methoxycarbonyl,
and
where oxazolyl and thiazolyl may by substituted by a substituent selected from the group consisting of fluorine, methyl, ethyl, methoxy, hydroxycarbonyl and methoxycarbonyl,
R² represents hydrogen,
R³ represents a group of the formula 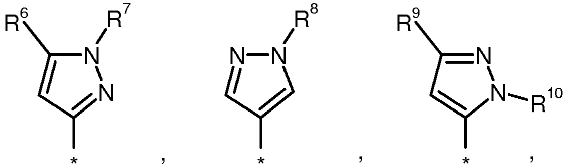 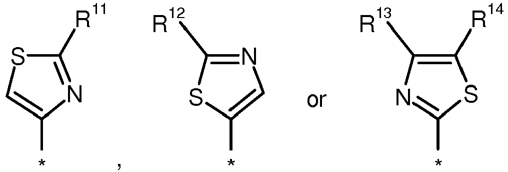 where
* denotes the point of attachment to the pyridine,
and
where
R⁶ represents hydrogen, methyl or ethyl,
R⁷ represents hydrogen or (C₁-C₆)-alkyl,
where (C₁-C₆)-alkyl may be substituted by 1 or 2 substituents independently of one another selected from the group consisting of hydroxyl and methoxy,
R⁸ represents hydrogen or (C₁-C₆)-alkyl,
where (C₁-C₆)-alkyl may be substituted by 1 or 2 substituents independently of one another selected from the group consisting of hydroxyl and methoxy,
R⁹ represents hydrogen, methyl or ethyl,
R¹⁰ represents hydrogen,
R¹¹ represents hydrogen or (C₁-C₆)-alkyl,
where (C₁-C₆)-alkyl may be substituted by 1 or 2 substituents independently of one another selected from the group consisting of hydroxyl and methoxy,
R¹² represents hydrogen or (C₁-C₆)-alkyl,
where (C₁-C₆)-alkyl may be substituted by 1 or 2 substituents independently of one another selected from the group consisting of hydroxyl and methoxy,
R¹³ represents hydrogen, methyl or ethyl, and
R¹⁴ represents hydrogen or (C₁-C₆)-alkyl,
where (C₁-C₆)-alkyl may be substituted by 1 or 2 substituents independently of one another selected from the group consisting of hydroxyl and methoxy,
R⁴ represents hydrogen,
R⁵ represents hydrogen, ethyl, n-propyl or secbutyl,
where ethyl, n-propyl and sec-butyl may be substituted by 1 or 2 hydroxyl substituents,
or
R⁴ and R⁵ together with the nitrogen atom to which they are attached form an azetidinyl or pyrrolidinyl ring which may be substituted by a hydroxyl substituent,
or a salt, solvate or solvate of a salt thereof.

4. Compound of the formula (I) according to Claim 1 in which
X represents O or S,
R¹ represents oxazolyl, thiazolyl or pyridyl,
where oxazolyl, thiazolyl and pyridyl are substituted by 1 or 2 substituents independently of one another selected from the group consisting of fluorine, chlorine, cyano, (C₁-C₄) -alkyl, trifluoromethyl, (C₁-C₄)-alkoxy, hydroxycarbonyl, (C₁-C₄)-alkoxycarbonyl, aminocarbonyl, mono-(C₁-C₄)-alkylaminocarbonyl, di-(C₁-C₄)-alkylaminocarbonyl, pyrrolidino, piperidino, morpholino, piperazino, N'-(C₁-C₄)-alkylpiperazino, phenyl and 5- or 6-membered heteroaryl,
where phenyl and 5- or 6-membered heteroaryl may be substituted by 1 or 2 substituents independently of one another selected from the group consisting of fluorine, chlorine, cyano, (C₁-C₄) -alkyl, difluoromethyl, trifluoromethyl, (C₁-C₄)-alkoxy, difluoromethoxy, trifluoromethoxy, hydroxycarbonyl and (C₁-C₄)-alkoxycarbonyl,
R² represents hydrogen or (C₁-C₄) -alkyl,
R³ represents thien-2-yl or thien-3-yl,
where thien-2-yl and thien-3-yl may be substituted by 1 or 2 substituents independently of one another selected from the group consisting of fluorine, (C₁-C₆)-alkyl, (C₁-C₄)-alkoxy and -NR^{A}R^{B},
where (C₁-C₆)-alkyl and (C₂-C₄)-alkoxy may be substituted by 1 or 2 substituents independently of one another selected from the group consisting of fluorine, trifluoromethyl, hydroxyl, methoxy, ethoxy, hydroxycarbonyl, amino, methylamino, ethylamino, *N,N-*dimethylamino and *N,N-*diethylamino,
and
where
R^{A} represents hydrogen or (C₁-C₄)-alkyl,
where (C₁-C₄)-alkyl for its part may be substituted by a substituent selected from the group consisting of hydroxyl and (C₁-C₄)-alkoxy,
R^{B} represents hydrogen or (C₁-C₄)-alkyl,
where (C₁-C₄)-alkyl for its part may be substituted by 1 or 2 substituents independently of one another selected from the group consisting of hydroxyl, (C₁-C₄)-alkoxy and hydroxycarbonyl,
R⁴ represents hydrogen or methyl,
R⁵ represents hydrogen or (C₁-C₄) -alkyl,
where (C₁-C₄)-alkyl may be substituted by 1 or 2 substituents independently of one another selected from the group consisting of hydroxyl, methoxy and ethoxy,
or
R⁴ and R⁵ together with the nitrogen atom to which they are attached form a 4- to 6-membered heterocycle which may contain a further ring heteroatom from the group consisting of N, O and S and may be substituted by 1 or 2 substituents independently of one another selected from the group consisting of (C₁-C₄)-alkyl, hydroxyl, methoxy and ethoxy,
or a salt, solvate or solvate of a salt thereof.

5. Compound of the formula (I) according to Claim 4 in which
X represents S,
R¹ represents oxazolyl, thiazolyl or pyridyl,
where pyridyl is substituted by a substituent selected from the group consisting of cyano, methoxy, ethoxy, hydroxycarbonyl, methoxycarbonyl, ethoxycarbonyl, aminocarbonyl, methylaminocarbonyl and dimethylaminocarbonyl,
and
where pyridyl may be substituted by a substituent selected from the group consisting of fluorine, chlorine, methyl, ethyl and trifluoromethyl,
and
where oxazolyl and thiazolyl are substituted by a phenyl substituent,
where phenyl may be substituted by a substituent selected from the group consisting of fluorine, chlorine, cyano, methyl, methoxy, hydroxycarbonyl and methoxycarbonyl,
and
where oxazolyl and thiazolyl may be substituted by a substituent selected from the group consisting of fluorine, methyl, ethyl, methoxy, hydroxycarbonyl and methoxycarbonyl,
R² represents hydrogen,
R³ represents a group of the formula 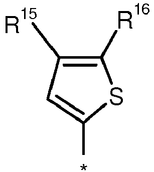 where
* denotes the point of attachment to the pyridine,
and where
R¹⁵ represents hydrogen, methyl or ethyl,
R¹⁶ represents hydrogen or (C₁-C₄)-alkyl,
where (C₁-C₄)-alkyl may be substituted by 1 or 2 substituents independently of one another selected from the group consisting of hydroxyl and methoxy,
R⁴ represents hydrogen,
R⁵ represents hydrogen , ethyl, n-propyl or sec-butyl,
where ethyl, n-propyl and sec-butyl may be substituted by 1 or 2 hydroxyl substituents,
or
R⁴ and R⁵ together with the nitrogen atom to which they are attached form an azetidinyl or pyrrolidinyl ring which may be substituted by a hydroxyl substituent,
or a salt, solvate or solvate of a salt thereof.

6. Process for preparing compounds of the formula (I) as defined in Claims 1 to 5, **characterized in that**
[A] a compound of the formula (II) 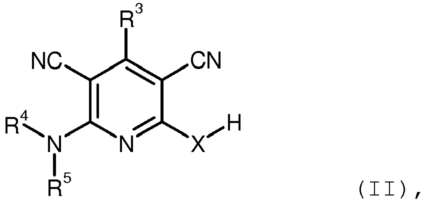 in which X, R³, R⁴ and R⁵ each have the meanings given in any of Claims 1 to 5,
is reacted in an inert solvent in the presence of a base with a compound of the formula (III) 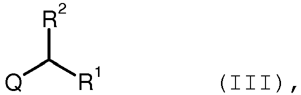 in which R¹ and R² each have the meanings given in any of Claims 1 to 5 and
Q represents a suitable leaving group, preferably halogen, in particular chlorine, bromine or iodine, or represents mesylate, tosylate or triflate,
or
[B] if X represents O, a compound of the formula (IV) 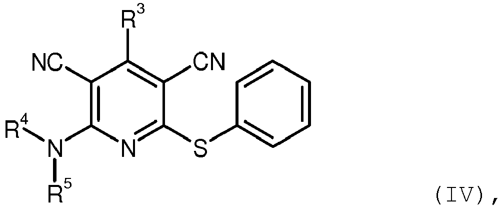 in which R³, R⁴ and R⁵ each have the meanings given in any of Claims 1 to 5,
is reacted in an inert solvent in the presence of a base with a compound of the formula (V) 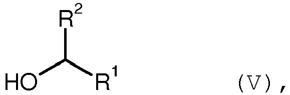 in which R¹ and R² have the meanings given in any of Claims 1 to 5,
or
[C] a compound of the formula (I-A) 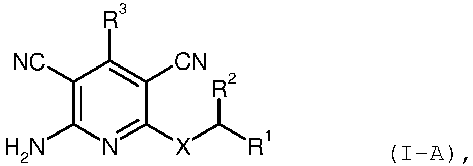 in which R¹, R² and R³ each have the meanings given in any of Claims 1 to 5,
is initially converted with copper(II) chloride and isoamyl nitrite in a suitable solvent into a compound of the formula (XV) 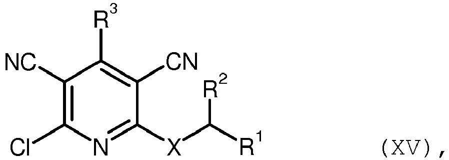 in which R¹, R² and R³ each have the meanings given in any of Claims 1 to 5,
and this is then reacted with a compound of the formula (VIII) 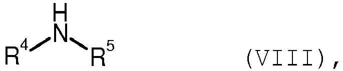 in which R⁴ and R⁵ each have the meanings given in any of Claims 1 to 5,
and
in which at least one of the two radicals R⁴ and R⁵ is different from hydrogen,
to give a compound of the formula (I-B) 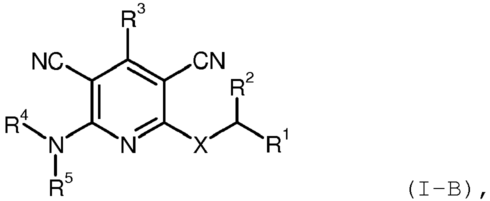 in which R¹, R², R³, R⁴ and R⁵ each have the meanings given in any of Claims 1 to 5,
and
in which at least one of the two radicals R⁴ and R⁵ is different from hydrogen,
any protective groups present are then removed and the resulting compounds of the formula (I) are, if appropriate, converted with the appropriate (i) solvents and/or (ii) bases or acids into their solvates, salts and/or solvates of the salts.

7. Compound of the formula (I) as defined in any of Claims 1 to 5 for the treatment and/or prophylaxis of diseases.

8. Compound of the formula (I) as defined in any of Claims 1 to 5 for use in a method for the treatment and/or prophylaxis of coronary heart disease, acute coronary syndrome, angina pectoris, heart failure, myocardial infarction and atrial fibrillation.

9. Compound of the formula (I) as defined in any of Claims 1 to 5 for use in a method for the treatment and/or prophylaxis of diabetes, metabolic syndrome and dyslipidemias.

10. Use of a compound of the formula (I) as defined in any of Claims 1 to 5 for preparing a medicament for the treatment and/or prophylaxis of coronary heart disease, acute coronary syndrome, angina pectoris, heart failure, myocardial infarction and atrial fibrillation.

11. Medicament, comprising a compound of the formula (I) as defined in any of Claims 1 to 5 in combination with an inert nontoxic pharmaceutically suitable auxiliary.

12. Medicament, comprising a compound of the formula (I) as defined in any of Claims 1 to 5 in combination with one or more further active ingredients selected from the group consisting of lipid metabolism-modifying active ingredients, antidiabetics, antihypertensive drugs and antithrombotic drugs.

13. Medicament according to Claim 11 or 12 for the treatment and/or prophylaxis of coronary heart disease, acute coronary syndrome, angina pectoris, heart failure, myocardial infarction and atrial fibrillation.

14. Medicament according to Claim 11 or 12 for the treatment and/or prophylaxis of diabetes, metabolic syndrome and dyslipidemias.

## Revendications

1. Composé de formule (I) 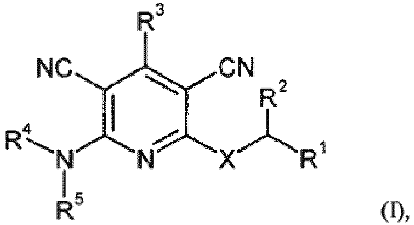 dans laquelle
X représente O ou S,
R¹ représente un groupe hétéroaryle à 5 ou 6 chaînons,
le groupe hétéroaryle à 5 ou 6 chaînons étant substitué par 1 ou 2 substituants choisis indépendamment l'un de l'autre dans le groupe constitué par halogéno, nitro, cyano, alkyle en C₁-C₆, trifluorométhyle, hydroxy, alcoxy en C₁-C₆, amino, monoalkyl(C₁-C₆)amino, dialkyl(C₁-C₆)amino, hydroxycarbonyle, alcoxy(C₁-C₆)carbonyle, aminocarbonyle, monoalkyl(C₁-C₆)aminocarbonyle, dialkyl(C₁-C₆)aminocarbonyle, cycloalkyl(C₃-C₇)-aminocarbonyle, aminosulfonyle, monoalkyl(C₁-C₆)-aminosulfonyle, dialkyl(C₁-C₆)aminosulfonyle, alkyl(C₁-C₆)sulfonylamino, pyrrolidino, pipéridino, morpholino, pipérazino, *N*'-alkyl(C₁-C₄)pipérazino, pyrrolidinocarbonyle, pipéridinocarbonyle, morpholinocarbonyle, pipérazinocarbonyle, *N*'-alkyl(C₁-C₄)pipérazinocarbonyle, phényle et hétéroaryle à 5 ou 6 chaînons,
les groupes phényle et hétéroaryle à 5 ou 6 chaînons pouvant être substitués par 1 à 3 substituants choisis chacun indépendamment dans le groupe constitué par halogéno, nitro, cyano, alkyle en C₁-C₆, difluorométhyle, trifluorométhyle, hydroxy, alcoxy en C₁-C₆, difluorométhoxy, trifluorométhoxy, amino, monoalkyl(C₁-C₆)amino, dialkyl(C₁-C₆)amino, hydroxycarbonyle et alcoxy(C₁-C₆)carbonyle,
R² représente un atome d'hydrogène ou un groupe alkyle en C₁-C₄,
R³ représente un groupe thiényle, pyrrolyle, oxazolyle, thiazolyle, isoxazolyle, isothiazolyle, pyrazolyle, imidazolyle, triazolyle, oxadiazolyle, thiadiazolyle ou tétrazolyle
les groupes thiényle, pyrrolyle, oxazolyle, thiazolyle, isoxazolyle, isothiazolyle, pyrazolyle, imidazolyle, triazolyle, oxadiazolyle, thiadiazolyle et tétrazolyle pouvant être substitués par 1 ou 2 substituants choisis indépendamment l'un de l'autre dans le groupe constitué par halogéno, cyano, hydroxy, alkyle en C₁-C₆, alcoxy en C₁-C₆, cycloalcoxy en C₃-C₇ et -NR^{A}R^{B},
les groupes alkyle en C₁-C₆ et alcoxy en C₁-C₆ pouvant être substitués par 1 à 3 substituants choisis chacun indépendamment dans le groupe constitué par fluoro, trifluorométhyle, cycloalkyle en C₃-C₇, oxo, hydroxy, alcoxy en C₁-C₄, hydroxycarbonyle, amino, monoalkyl(C₁-C₄)amino et dialkyl(C₁-C₄)-amino,
et
le groupe cycloalcoxy en C₃-C₇ pouvant être substitué par 1 ou 2 substituants choisis indépendamment l'un de l'autre dans le groupe constitué par alkyle en C₁-C₄, hydroxy, oxo et alcoxy en C₁-C₄ ; et
R^{A} représentant un atome d'hydrogène ou un groupe alkyle en C₁-C₆, le groupe alkyle en C₁-C₆ pouvant pour sa part être substitué par 1 à 3 substituants fluoro, et le groupe alkyle en C₁-C₆ pouvant pour sa part être substitué par un substituant choisi dans le groupe constitué par hydroxy et alcoxy en C₁-C₄,
R^{B} représentant un atome d'hydrogène, un groupe alkyle en C₁-C₆, cycloalkyle en C₃-C₇, alkyl(C₁-C₆)-carbonyle, alkyl(C₁-C₄)sulfonyle ou cycloalkyl(C₃-C₇)sulfonyle, le groupe alkyle en C₁-C₆ pouvant pour sa part être substitué par 1 à 3 substituants fluoro, et le groupe alkyle en C₁-C₆ pouvant pour sa part être substitué par 1 ou 2 substituants choisis indépendamment l'un de l'autre dans le groupe constitué par cycloalkyle en C₃-C₇, oxo, hydroxy, alcoxy en C₁-C₄, hydroxycarbonyle, amino, monoalkyl(C₁-C₄)amino, dialkyl(C₁-C₄)amino et cycloalkyl(C₃-C₇)amino et le groupe cycloalkyle en C₃-C₇ pouvant pour sa part être substitué par 1 ou 2 substituants choisis indépendamment l'un de l'autre dans le groupe constitué par alkyle en C₁-C₄, hydroxy, oxo et alcoxy en C₁-C₄,
ou
R^{A} et R^{B} formant ensemble avec l'atome d'azote, auquel ils sont liés, un hétérocycle à 4 à 7 chaînons, qui peut contenir un autre hétéroatome dans le cycle, choisi dans la série consistant en N, O ou S, et être substitué par 1 ou 2 substituants choisis indépendamment l'un de l'autre dans le groupe constitué par alkyle en C₁-C₄, hydroxy, oxo et alcoxy en C₁-C₄,
R⁴ représente un atome d'hydrogène ou un groupe alkyle en C₁-C₆,
le groupe alkyle en C₁-C₆ pouvant être substitué par 1 ou 2 substituants choisis indépendamment l'un de l'autre dans le groupe constitué par hydroxy, alcoxy en C₁-C₄, hydroxycarbonyle, alcoxy(C₁-C₄)carbonyle, amino, monoalkyl(C₁-C₄)-amino et dialkyl(C₁-C₄)amino,
R⁵ représente un atome d'hydrogène ou un groupe alkyle en C₁-C₆,
le groupe alkyle en C₁-C₆ pouvant être substitué par 1 ou 2 substituants choisis indépendamment l'un de l'autre dans le groupe constitué par hydroxy, alcoxy en C₁-C₄, hydroxycarbonyle, alcoxy(C₁-C₄)carbonyle, amino, monoalkyl(C₁-C₄)-amino et dialkyl(C₁-C₄)amino,
ou
R⁴ et R⁵ forment ensemble avec l'atome d'azote, auquel ils sont liés, un hétérocycle à 4 à 7 chaînons, qui peut contenir un autre hétéroatome dans le cycle, choisi dans la série consistant en N, O ou S, et être substitué par 1 ou 2 substituants choisis indépendamment l'un de l'autre dans le groupe constitué par alkyle en C₁-C₄, hydroxy, oxo et alcoxy en C₁-C₄,
ainsi que ses N-oxydes, sels, produits de solvatation, sels des N-oxydes et produits de solvatation des N-oxydes et sels.

2. Composé de formule (I) selon la revendication 1, dans lequel
X représente O ou S,
R¹ représente un groupe hétéroaryle à 5 ou 6 chaînons, le groupe hétéroaryle à 5 ou 6 chaînons étant substitué par 1 ou 2 substituants choisis indépendamment l'un de l'autre dans le groupe constitué par fluoro, chloro, cyano, alkyle en C₁-C₄, trifluorométhyle, alcoxy en C₁-C₄, monoalkyl(C₁-C₄)amino, dialkyl(C₁-C₄)amino, hydroxycarbonyle, alcoxy(C₁-C₄)carbonyle, aminocarbonyle, monoalkyl(C₁-C₄)aminocarbonyle, dialkyl(C₁-C₄)aminocarbonyle, pyrrolidino, pipéridino, morpholino, pipérazino, *N'-*alkyl(C₁-C₄)pipérazino, phényle et hétéroaryle à 5 ou 6 chaînons,
les groupes phényle et hétéroaryle à 5 ou 6 chaînons pouvant être substitués par 1 ou 2 substituants choisis indépendamment l'un de l'autre dans le groupe constitué par fluoro, chloro, cyano, alkyle en C₁-C₄, difluorométhyle, trifluorométhyle, alcoxy en C₁-C₄, difluorométhoxy, trifluorométhoxy, hydroxycarbonyle et alcoxy(C₁-C₄)carbonyle,
R² représente un atome d'hydrogène ou le groupe méthyle,
R³ représente le groupe pyrrol-1-yle, pyrrol-2-yle, pyrrol-3-yle, oxazol-2-yle, oxazol-4-yle, oxazol-5-yle, thiazol-2-yle, thiazol-4-yle, thiazol-5-yle, isoxazol-5-yle, pyrazol-1-yle, pyrazol-3-yle, pyrazol-4-yle, pyrazol-5-yle, imidazol-1-yle, imidazol-2-yle, imidazol-4-yle ou imidazol-5-yle,
les groupes pyrrol-1-yle, pyrrol-2-yle, pyrrol-3-yle, oxazol-2-yle, oxazol-4-yle, oxazol-5-yle, thiazol-2-yle, thiazol-4-yle, thiazol-5-yle, isoxazol-5-yle, pyrazol-1-yle, pyrazol-3-yle, pyrazol-4-yle, pyrazol-5-yle, imidazol-1-yle, imidazol-2-yle, imidazol-4-yle et imidazol-5-yle pouvant être substitués par 1 ou 2 substituants choisis indépendamment l'un de l'autre dans le groupe constitué par fluoro, trifluorométhyle, alkyle en C₁-C₆, alcoxy en C₁-C₄ et -NR^{A}R^{B},
les groupes alkyle en C₁-C₆ et alcoxy en C₂-C₄ pouvant être substitués par 1 ou 2 substituants choisis indépendamment l'un de l'autre dans le groupe constitué par fluoro, trifluorométhyle, hydroxy, méthoxy, éthoxy, hydroxycarbonyle, amino, méthylamino, éthylamino, N,N-diméthylamino et N,N-diéthylamino, et
R^{A} représentant un atome d'hydrogène ou un groupe alkyle en C₁-C₄, le groupe alkyle en C₁-C₄ pouvant pour sa part être substitué par un substituant choisi dans le groupe constitué par hydroxy et alcoxy en C₁-C₄,
R^{B} représentant un atome d'hydrogène, un groupe alkyle en C₁-C₄,
le groupe alkyle en C₁-C₄ pouvant pour sa part être substitué par 1 ou 2 substituants choisis indépendamment l'un de l'autre dans le groupe constitué par hydroxy, alcoxy en C₁-C₄ et hydroxycarbonyle,
R⁴ représente un atome d'hydrogène ou le groupe méthyle,
R⁵ représente un atome d'hydrogène ou un groupe alkyle en C₁-C₄,
le groupe alkyle en C₁-C₄ pouvant être substitué par 1 ou 2 substituants choisis indépendamment l'un de l'autre dans le groupe constitué par hydroxy, méthoxy et éthoxy,
ou
R⁴ et R⁵ forment ensemble avec l'atome d'azote, auquel ils sont liés, un hétérocycle à 4 à 6 chaînons, qui peut contenir un autre hétéroatome dans le cycle, choisi dans la série consistant en N, O ou S, et être substitué par 1 ou 2 substituants choisis indépendamment l'un de l'autre dans le groupe constitué par alkyle en C₁-C₄, hydroxy, méthoxy et éthoxy,
ainsi que ses sels, produits de solvatation et produits de solvatation des sels.

3. Composés de formule (I) selon la revendication 1 ou 2, dans lequel
X représente S,
R¹ représente un groupe oxazolyle, thiazolyle ou pyridyle,
le groupe pyridyle étant substitué par un substituant choisi dans le groupe constitué par cyano, méthoxy, éthoxy, hydroxycarbonyle, méthoxycarbonyle, éthoxycarbonyle, aminocarbonyle, méthylaminocarbonyle et diméthylaminocarbonyle,
et
le groupe pyridyle pouvant être substitué par un substituant choisi dans le groupe constitué par fluoro, chloro, méthyle, éthyle et trifluorométhyle,
et
les groupes oxazolyle et thiazolyle étant substitués par un substituant phényle,
le groupe phényle pouvant être substitué par un substituant choisi dans le groupe constitué par fluoro, chloro, cyano, méthyle, méthoxy, hydroxycarbonyle et méthoxycarbonyle,
et
les groupes oxazolyle et thiazolyle pouvant être substitués par un substituant choisi dans le groupe constitué par fluoro, méthyle, éthyle, méthoxy, hydroxycarbonyle et méthoxycarbonyle,
R² représente un atome d'hydrogène,
R³ représente un groupe de formule 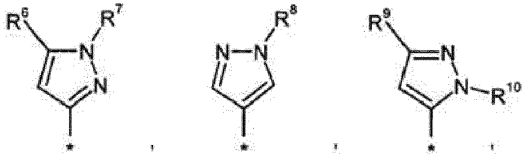 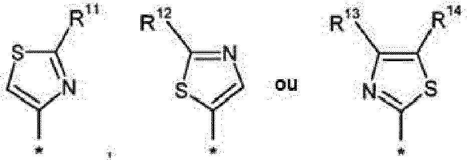 * représentant la position d'attachement à la pyridine,
et
R⁶ représentant un atome d'hydrogène, le groupe méthyle ou éthyle,
R⁷ représentant un atome d'hydrogène ou un groupe alkyle en C₁-C₆,
le groupe alkyle en C₁-C₆ pouvant être substitué par 1 ou 2 substituants choisis indépendamment l'un de l'autre dans le groupe constitué par hydroxy et méthoxy,
R⁸ représentant un atome d'hydrogène ou un groupe alkyle en C₁-C₆,
le groupe alkyle en C₁-C₆ pouvant être substitué par 1 ou 2 substituants choisis indépendamment l'un de l'autre dans le groupe constitué par hydroxy et méthoxy,
R⁹ représentant un atome d'hydrogène, le groupe méthyle ou éthyle,
R¹⁰ représentant un atome d'hydrogène,
R¹¹ représentant un atome d'hydrogène ou un groupe alkyle en C₁-C₆,
le groupe alkyle en C₁-C₆ pouvant être substitué par 1 ou 2 substituants choisis indépendamment l'un de l'autre dans le groupe constitué par hydroxy et méthoxy,
R¹² représentant un atome d'hydrogène ou un groupe alkyle en C₁-C₆, le groupe alkyle en C₁-C₆ pouvant être substitué par 1 ou 2 substituants choisis indépendamment l'un de l'autre dans le groupe constitué par hydroxy et méthoxy,
R¹³ représente un atome d'hydrogène, le groupe méthyle ou éthyle,
et R¹⁴ représentant un atome d'hydrogène ou un groupe alkyle en C₁-C₆, le groupe alkyle en C₁-C₆ pouvant être substitué par 1 ou 2 substituants choisis indépendamment l'un de l'autre dans le groupe constitué par hydroxy et méthoxy,
R⁴ représente un atome d'hydrogène,
R⁵ représente un atome d'hydrogène, le groupe éthyle, n-propyle ou sec-butyle, les groupes éthyle, n-propyle et sec-butyle pouvant être substitués par 1 ou 2 substituants hydroxy,
ou
R⁴ et R⁵ forment ensemble avec l'atome d'azote, auquel ils sont liés, un cycle azétidinyle ou pyrrolidinyle, qui peut être substitué par un substituant hydroxy,
ainsi que ses sels, produits de solvatation et produits de solvatation des sels.

4. Composé de formule (I) selon la revendication 1, dans lequel
X représente O ou S,
R¹ représente un groupe oxazolyle, thiazolyle ou pyridyle,
les groupes oxazolyle, thiazolyle et pyridyle, étant substitués par 1 ou 2 substituants choisis indépendamment l'un de l'autre dans le groupe constitué par fluoro, chloro, cyano, alkyle en C₁-C₄, trifluorométhyle, alcoxy en C₁-C₄, hydroxycarbonyle, alcoxy(C₁-C₄)carbonyle, aminocarbonyle, monoalkyl(C₁-C₄)aminocarbonyle, dialkyl(C₁-C₄)aminocarbonyle, pyrrolidino, pipéridino, morpholino, pipérazino, N'-alkyl(C₁-C₄) pipérazino, phényle et hétéroaryle à 5 ou 6 chaînons
les groupes phényle et hétéroaryle à 5 ou 6 chaînons pouvant être substitués par 1 ou 2 substituants choisis indépendamment l'un de l'autre dans le groupe constitué par fluoro, chloro, cyano, alkyle en C₁-C₄, difluorométhyle, trifluorométhyle, alcoxy en C₁-C₄, difluorométhoxy, trifluorométhoxy, hydroxycarbonyle et alcoxy(C₁-C₄)carbonyle,
R² représente un atome d'hydrogène ou un groupe alkyle en C₁-C₄,
R³ représente un groupe thién-2-yle ou thién-3-yle, les groupes thién-2-yle et thién-3-yle pouvant être substitués par 1 ou 2 substituants choisis indépendamment l'un de l'autre dans le groupe constitué par fluoro, alkyle en C₁-C₆, alcoxy en C₁-C₄ et -NR^{A}R^{B},
les groupes alkyle en C₁-C₆ et alcoxy en C₂-C₄ pouvant être substitués par 1 ou 2 substituants choisis indépendamment l'un de l'autre dans le groupe constitué par fluoro, trifluorométhyle, hydroxy, méthoxy, éthoxy, hydroxycarbonyle, amino, méthylamino, éthylamino, *N,N*-diméthylamino et *N,N*-diéthyl-amino,
et
R^{A} représentant un atome d'hydrogène ou un groupe alkyle en C₁-C₄,
le groupe alkyle en C₁-C₄ pouvant pour sa part être substitué par un substituant choisi dans le groupe constitué par hydroxy et alcoxy en C₁-C₄,
R^{B} représentant un atome d'hydrogène ou un groupe alkyle en C₁-C₄,
le groupe alkyle en C₁-C₄ pouvant pour sa part être substitué par 1 ou 2 substituants choisis indépendamment l'un de l'autre dans le groupe constitué par hydroxy, alcoxy en C₁-C₄ et hydroxycarbonyle,
R⁴ représente un atome d'hydrogène ou le groupe méthyle,
R⁵ représente un atome d'hydrogène ou un groupe alkyle en C₁-C₄,
le groupe alkyle en C₁-C₄ pouvant être substitué par 1 ou 2 substituants choisis indépendamment l'un de l'autre dans le groupe constitué par hydroxy, méthoxy et éthoxy,
ou
R⁴ et R⁵ forment ensemble avec l'atome d'azote, auquel ils sont liés, un hétérocycle à 4 à 6 chaînons, qui peut contenir un autre hétéroatome dans le cycle, choisi dans la série consistant en N, O ou S, et être substitué par 1 ou 2 substituants choisis indépendamment l'un de l'autre dans le groupe constitué par alkyle en C₁-C₄, hydroxy, méthoxy et éthoxy,
ainsi que ses sels, produits de solvatation et produits de solvatation des sels.

5. Composé de formule (I) selon la revendication 4, dans lequel
X représente S,
R¹ représente un groupe oxazolyle, thiazolyle ou pyridyle,
le groupe pyridyle étant substitué par un substituant choisi dans le groupe constitué par cyano, méthoxy, éthoxy, hydroxycarbonyle, méthoxycarbonyle, éthoxycarbonyle, aminocarbonyle, méthylaminocarbonyle et diméthylaminocarbonyle, et
le groupe pyridyle pouvant être substitué par un substituant choisi dans le groupe constitué par fluoro, chloro, méthyle, éthyle et trifluorométhyle,
et
les groupes oxazolyle et thiazolyle étant substitués par un substituant phényle,
le groupe phényle pouvant être substitué par un substituant choisi dans le groupe constitué par fluoro, chloro, cyano, méthyle, méthoxy, hydroxycarbonyle et méthoxycarbonyle,
et
les groupes oxazolyle et thiazolyle pouvant être substitués par un substituant choisi dans le groupe constitué par fluoro, méthyle, éthyle, méthoxy, hydroxycarbonyle et méthoxycarbonyle,
R² représente un atome d'hydrogène,
R³ représente un groupe de formule 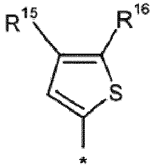 * représentant la position d'attachement à la pyridine,
et
R¹⁵ représentant un atome d'hydrogène, le groupe méthyle ou éthyle,
R¹⁶ représentant un atome d'hydrogène ou un groupe alkyle en C₁-C₄,
le groupe alkyle en C₁-C₄ pouvant être substitué par 1 ou 2 substituants choisis indépendamment l'un de l'autre dans le groupe constitué par hydroxy et méthoxy,
R⁴ représente un atome d'hydrogène,
R⁵ représente un atome d'hydrogène, le groupe éthyle, n-propyle ou sec-butyle,
les groupes éthyle, n-propyle et sec-butyle pouvant être substitués par 1 ou 2 substituants hydroxy,
ou
R⁴ et R⁵ forment ensemble avec l'atome d'azote, auquel ils sont liés, un cycle azétidinyle ou pyrrolidinyle, qui peut être substitué par un substituant hydroxy,
ainsi que ses sels, produits de solvatation et produits de solvatation des sels.

6. Procédé pour la préparation de composés de formule (I), tels que définis dans les revendications 1 à 5, **caractérisé en ce que**
[A] on fait réagir un composé de formule (II) 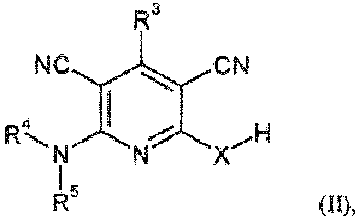 dans laquelle X, R³, R⁴ et R⁵ ont chacun les significations indiquées dans les revendications 1 à 5,
dans un solvant inerte, en présence d'une base, avec un composé de formule (III) 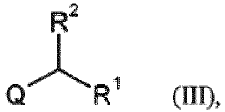 dans laquelle R¹ et R² ont chacun les significations indiquées dans les revendications 1 à 5 et
Q représente un groupe partant approprié, de préférence un atome d'halogène, en particulier de chlore, de brome ou d'iode, ou le groupe mésylate, tosylate ou triflate,
ou
[B] dans le cas où X représente O, on fait réagir un composé de formule (IV) 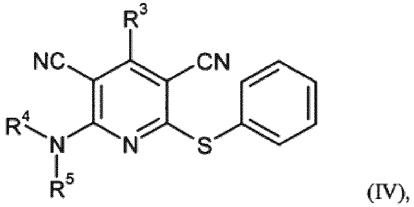 dans laquelle R³, R⁴ et R⁵ ont chacun les significations indiquées dans les revendications 1 à 5,
dans un solvant inerte, en présence d'une base, avec un composé de formule (V) 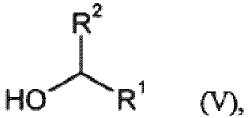 dans laquelle R¹ et R² ont les significations indiquées dans les revendications 1 à 5,
ou
[C] d'abord on convertit un composé de formule (I-A) 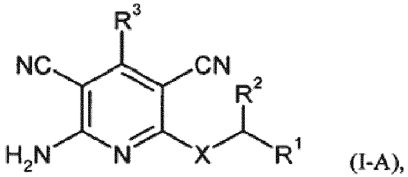 dans laquelle R¹, R² et R³ ont chacun les significations indiquées dans les revendications 1 à 5
à l'aide de chlorure de cuivre(II) et de nitrite d'isoamyle, dans un solvant approprié, en un composé de formule (XV) 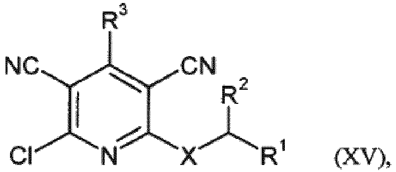 dans laquelle R¹, R² et R³ ont chacun les significations indiquées dans les revendications 1 à 5,
et ensuite on fait réagir ce dernier avec un composé de formule (VIII) 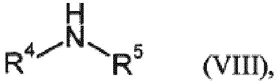 dans laquelle R⁴ et R⁵ ont chacun les significations indiquées dans les revendications 1 à 5, et
au moins l'un des deux radicaux R⁴ et R⁵ étant différent d'un atome d'hydrogène,
pour aboutir à un composé de formule (I-B) 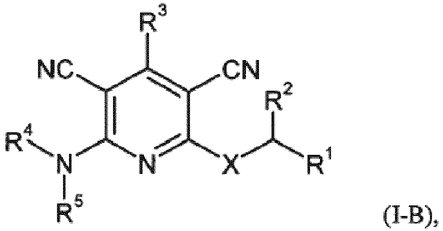 dans laquelle R¹, R², R³, R⁴ et R⁵ ont chacun les significations indiquées dans les revendications 1 à 5,
au moins l'un des deux radicaux R⁴ et R⁵ étant différent d'un atome d'hydrogène,
ensuite on élimine les groupes protecteurs éventuellement présents et éventuellement on convertit les composés de formule (I) résultants, à l'aide (i) des solvants correspondants et/ou (ii) des bases correspondantes ou des acides correspondants, en leurs produits de solvatation, sels et/ou produits de solvatation des sels.

7. Composé de formule (I), tel que défini dans l'une quelconque des revendications 1 à 5, destiné au traitement et/ou à la prévention de maladies.

8. Composé de formule (I), tel que défini dans l'une quelconque des revendications 1 à 5, pour utilisation dans un procédé pour le traitement et/ou la prophylaxie de la cardiopathie coronarienne, du syndrome coronarien aigu, de l'angine de poitrine, de l'insuffisance cardiaque, de l'infarctus du myocarde et de la fibrillation auriculaire.

9. Composé de formule (I), tel que défini dans l'une quelconque des revendications 1 à 5, pour utilisation dans un procédé pour le traitement et/ou la prophylaxie du diabète, du syndrome métabolique et de dyslipidémies.

10. Utilisation d'un composé de formule (I), tel que défini dans l'une quelconque des revendications 1 à 5, pour la fabrication d'un médicament destiné au traitement et/ou à la prévention de la cardiopathie coronarienne, du syndrome coronarien aigu, de l'angine de poitrine, de l'insuffisance cardiaque, de l'infarctus du myocarde et de la fibrillation auriculaire.

11. Médicament contenant un composé de formule (I), tel que défini dans l'une quelconque des revendications 1 à 5, en association avec un adjuvant inerte, non toxique, pharmaceutiquement approprié.

12. Médicament contenant un composé de formule (I), tel que défini dans l'une quelconque des revendications 1 à 5, en association avec une ou plusieurs autres substances actives choisies dans le groupe constitué par les substances actives modifiant le métabolisme des lipides, les antidiabétiques, les substances actives abaissant la pression artérielle et les agents à action antithrombotique.

13. Médicament selon la revendication 11 ou 12, destiné au traitement et/ou à la prévention de la cardiopathie coronarienne, du syndrome coronarien aigu, de l'angine de poitrine, de l'insuffisance cardiaque, de l'infarctus du myocarde et de la fibrillation auriculaire.

14. Médicament selon la revendication 11 ou 12, destiné au traitement et/ou à la prévention du diabète, du syndrome métabolique et des dyslipidémies.
